(19) Europäisches Patentamt
European Patent Office
Office européen des brevets

(11) **EP 1 828 197 B1**

(12) **EUROPEAN PATENT SPECIFICATION**

(45) Date of publication and mention of the grant of the patent:
**13.05.2009 Bulletin 2009/20**

(21) Application number: **04804787.2**

(22) Date of filing: **13.12.2004**

(51) Int Cl.:
***C07D 495/04*** $^{(2006.01)}$ ***A61K 31/519*** $^{(2006.01)}$

(86) International application number:
**PCT/EP2004/053424**

(87) International publication number:
**WO 2006/063615 (22.06.2006 Gazette 2006/25)**

(54) **NOVEL SUBSTITUTED THIOPHENEPYRIMIDINONE DERIVATIVES AS INHIBITORS OF 17ß-HYDROXYSTEROID DEHYDROGENASE**

NEUE SUBSTITUIERTE THIOPHENPYRIMIDINONDERIVATE ALS INHIBITOREN VON 17ß-HYDROXYSTEROIDDEHYDROGENASE

NOUVEAUX DÉRIVÉS SUBSTITUÉS DE THIOPHÈNEPYRIMIDINONE EN TANT QU'INHIBITEURS DE LA 17ß-HYDROXYSTÉROÏDE DÉSHYDROGÉNASE

(84) Designated Contracting States:
**AT BE BG CH CY CZ DE DK EE ES FI FR GB GR HU IE IS IT LI LT LU MC NL PL PT RO SE SI SK TR**
Designated Extension States:
**HR LV**

(43) Date of publication of application:
**05.09.2007 Bulletin 2007/36**

(73) Proprietor: **Solvay Pharmaceuticals GmbH**
**30173 Hannover (DE)**

(72) Inventors:
- **HIRVELÄ, Leena**
  **FIN-90520 Oulu (FI)**
- **HUSEN, Bettina**
  **30173 Hannover (DE)**
- **KIVINIEMI, Johanna**
  **FIN-20500 Turku (FI)**
- **KOSKIMIES, Pasi**
  **FIN-20740 Turku (FI)**
- **LEHTOVUORI, Pekka**
  **FIN-02110 Espoo (FI)**
- **MESSINGER, Josef**
  **31319 Sehnde (DE)**
- **PENTIKÄINEN, Olli Taneli**
  **40800 Vaajakoski (FI)**
- **Kallio, Lila**
  **20740 Turku (FI)**
- **SAARENKETO, Pauli**
  **FIN-21500 Piikkiö (FI)**
- **THOLE, Heinrich-Hubert**
  **30655 Hannover (DE)**
- **UNKILA, Mikko**
  **FIN-20780 Kaarina (FI)**
- **VAN STEEN, Bartholomeus Johannes**
  **NL-3572 HD Utrecht (NL)**

(74) Representative: **Gosmann, Martin et al**
**IP & Scientific Information**
**Solvay Pharmaceuticals GmbH**
**Hans-Böckler-Allee 20**
**30173 Hannover (DE)**

(56) References cited:
**WO-A-96/22992**
**WO-A-20/04080271**
**WO-A-20/04110459**
**WO-A-20/05032527**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 828 197 B1

## Description

FIELD OF THE INVENTION

The present invention relates to novel substituted thiophenepyrimidinone derivatives which represent inhibitory compounds of the 17β-hydroxysteroid dehydrogenase enzymes, preferably of the 17β-hydroxysteroid dehydrogenase type 1 (17β-HSD1), type 2 (17β-HSD2) or type 3 (17β-HSD3) enzyme, to their salts, to pharmaceutical preparations containing these compounds and to processes for the preparation of these compounds. Furthermore, the invention concerns the therapeutic use of said benzothiophenepyrimidinone derivatives, particularly their use in the treatment or prevention of steroid hormone dependent diseases or disorders, such as steroid hormone dependent diseases or disorders requiring the inhibition of 17β-hydroxysteroid dehydrogenase enzymes, in particular 17β-HSD type I enzymes, and/or requiring the modulation of the endogenous 17β-estradiol and/or testosterone concentration.

BACKGROUND OF THE INVENTION

The publications and other materials used herein to illuminate the background of the invention, and in particular, cases to provide additional details respecting the practice, are incorporated by reference.

Mammalian 17β-Hydroxystemid dehydrogenases (17β-HSDs) are NAD(H) or NADP(H) dependent enzymes which catalyze - besides other reactions - the final steps in male and female sex hormone biosynthesis. These enzymes convert inactive 17-keto-steroids into their active 17β-hydroxy-forms or catalyze the oxidation of the 17β-hydroxy-forms into the 17-keto-steroids. Because both estrogens and androgens have the highest affinity for their receptors in the 17β-hydroxy form, 17β-HSD enzymes play an essential role in the tissue-selective regulation of the activity of sex steroid hormones.

At present, 10 human members of the 17β-HSD enzyme family have been described (types 1-5, 7, 8, 10, 11 and 12). The human 17β-HSD family members share less than 30% similarity in their primary structure. The 17β-HSDs are expressed in distinct, though in some cases, overlapping patterns. The different types of 17βHSDs also differ in their substrate and cofactor specificities. In intact cells in culture, the 17β-HSDs catalyze the reaction in a unidirectional way: types 1, 3, 5 and 7 use NADP(H) as a cofactor and catalyze the reductive reaction (activation), while types 2, 4, 8 and 10 catalyze the oxidative reaction (inactivation) using NAD(H) as a cofactor. [see e.g. Labrie et al. (2000) Trends Endocrinol Metab., 11:421-7].

Due to their essential role in the tissue-selective regulation of the activity of sex steroid hormones 17β-HSDs can be involved in the occurrence and development of estrogen-sensitive pathologies (f. ex. breast, ovarian, uterine and endometrium cancers etc.) and androgen-sensitive pathologies (f. ex. prostate cancer, benign prostatic hyperplasia, acne, hirsutism, etc). Furthermore, many types of 17β-HSD have been shown to be involved in the pathogenesis of particular human disorders. For example, 17β-HSD3 is known to be involved in the development of pseudohermaphroditism, the 17β-HSD8 plays a role in polycystic kidney disease and the 17β-HSD4 is related to the occurrence of bifunctional enzyme deficiency. Therefore treatment of sex steroid-sensitive diseases by administration of specific inhibitors of the 17β-HSDs enzymes have been suggested, optionally in combination with potent and specific antiestrogens and antiandrogens [Labrie F et al. (1997) Steroids, 62:148-58].

Due to the fact that each type of 17β-HSD has a selective substrate affinity, directional (reductive or oxidative) activity in intact cells, and a particular tissue distribution, the selectivity of drug action could be achieved by targeting a particular 17β-HSD isozyme. By individual modulation of the particular 17β-HSDs it is possible to influence or even control the local and paracrine concentration of estrogens and androgens in different target tissues.

The best characterized member of the 17β-HSD family is the type 1 17β-HSD [EC 1.1.1.62]. This enzyme could be crystallized in different states of functionality (e.g. with and without ligand and/or co-factor). The 17β-HSD1 catalyzes in vitro the reduction as well as the oxidation between estrone (E1) and estradiol (E2). However, under physiological in vivo conditions the enzyme only catalyzes the reductive reaction from the estrone (E1) to the estradiol (E2). The 17β-HSD1 was found to be expressed in a variety of hormone-dependent tissues, e.g. placenta, mammary gland tissue or uterus and endometrium tissue, respectively. Estradiol itself is, especially in comparison to the significantly less active estrone, a very potent hormone, which regulates the expression of a variety of genes by binding to the nuclear estrogen receptor and plays an essential role in the proliferation and differentiation of the target cell. Physiological as well as pathological cell proliferations can be estradiol dependent. Especially many breast cancer cells are stimulated by a locally raised estradiol concentration. Furthermore, the occurrence or course of benign pathologies such as endometriosis, uterine leiomyomas (fibroids or myomas), adenomyosis, menorrhagia, metrorrhagia and dysmenorrhea is dependent from the existence of significantly high estradiol levels.

Endometriosis is a well-known gynaecological disorder that affects 10 to 15% of women in the reproductive age. It is a benign disease defined as the presence of viable endometrial gland and stroma cells outside the uterine cavity. It is most frequently found in the pelvis area. In women developing endometriosis, the endometrial cells entering

the peritoneal cavity by retrograde menstruation (the most likely mechanism) have the capacity to adhere to and invade the peritoneal lining, and are then able to implant and grow. The implants respond to steroid hormones of the menstrual cycle in a similar way as the endometrium in the uterus. The infiltrating lesions and the blood from these lesions which are unable to leave the body cause inflammation of the surrounding tissue. The most common symptoms of endometriosis are dysmenorrhoea, dyspareunia and (chronic) abdominal pain. The occurrence of these symptoms is not related to the extent of the lesions. Some women with severe endometriosis are asymptomatic, while women with mild endometriosis may have severe pain. Endometriosis is found in up to 50% of the women with infertility. However, currently no causal relation has been proven between mild endometriosis and infertility. Moderate to severe endometriosis can cause tubal damage and adhesions leading to infertility. The aims of treatment of endometriosis are pain relief, resolution of the endometriotic tissue and restoration of fertility (if desired). The two common treatments are surgery or anti-inflammatory and/or hormonal therapy or a combination thereof.

Uterine leiomyomas (fibroids or myomas), benign clonal tumours, arise from smooth muscle cells of the human uterus. They are clinically apparent in up to 25% of women and are the single, most common indication for hysterectomy. They cause significant morbidity, including prolonged and heavy menstrual bleeding, pelvic pressure and pain, urinary problems, and, in rare cases, reproductive dysfunction. The pathophysiology of myomas is not well understood. Myomas are found submucosally (beneath the endometrium), intramurally (within the myometrium) and subserosally (projecting out of the serosal compartment of the uterus), but mostly are mixed forms of these 3 different types. The presence of estrogen receptors in leiomyoma cells has been studied by Tamaya et al. [Tamaya et al. (1985) Acta Obstet Gynecol Scand., 64:307-9]. They have shown that the ratios of estrogen receptor compared to progesterone and androgen receptor levels were higher in leiomyomas than in the corresponding normal myometrium. Surgery has long been the main treatment for myomas. Furthermore, medical therapies that have been proposed to treat myomas include administration of a variety of steroids such as the androgenic steroids danazol or gestrinone, GnRH agonists and progestogens, whereby the administration is often associated a variety of serious side-effects.

Everything that has been said above in relation to the treatment of uterine leiomyomas and endometriosis equally applies to other benign gynaecological disorders, notably adenomyosis, functional menorrhagia and metrorrhagia. These benign gynaecological disorders are all estrogen sensitive and are treated in a comparable way as described herein before in relation to uterine leiomyomas and endometriosis. The available pharmaceutical treatments, however, suffer from the same major drawbacks, i.e. they have to be discontinued once the slde-effeds become more serious than the symptoms to be treated and symptoms reappear after discontinuation of the therapy.

Since the aforementioned malign and benign pathologies are all 17β-estradiol dependent, a reduction of the endogenous 17β-estmdiol concentration in the respective tissue will result in an impaired or reduced proliferation of 17β-estradiol cells in said tissue. Therefore, it may be concluded that selective inhibitors of the 17β-HSD1 enzyme are well suited for their use to impair endogenous productions of estrogens, in particular of 17β-estradiol, in myomas, endometriotic, adenomyotic and endometrial tissue. The application of a compound acting as selective inhibitor on the 17β-HSD1 which preferentially catalyzes the reductive reaction will result in a lowered intracellular estradiol-concentration since the reductive conversion of the estrone into the active estradiol is reduced or suppressed. Therefore, reversible or even irreversible inhibitors of the 17β-HSD1 may play a significant role in the prophylaxis and/or treatment of steroid-hormone, in particular 17β-estradiol, dependent disorders or diseases. Furthermore, the reversible or even irreversible inhibitors of the 17β-HSD1 should have no or only pure antagonistic binding activities to the estradiol receptor, in particular to the estrogen receptor a subtype, since agonistic binding of the estrogen receptor would lead to activation and therefore - by regulation of a variety of genes - to the proliferation and differentiation of the target cell. In contrast, antagonists of the estrogen receptor, so called anti-estrogens, bind competitively to the specific receptor protein thus preventing access of endogenous estrogens to their specific, binding site. At present it is described in the literature that several malignant disease as breast cancer, prostate carcinoma, ovarian cancer, uterine cancer, endometrial cancer and endometrial hyperplasia may be treated by the administration of a selective 17β-HSD1 inhibitor. Furthermore, a selective 17β-HSD1 inhibitor may be useful for the prevention of the aforementioned hormone-dependent cancers, especially breast cancer.

Several reversible or irreversible inhibitors of the 17β-HSD1 enzyme of steroidal and even non-steroidal origin are already known from the literature. The characteristics of these inhibitory molecules, which mainly have a substrate or cofactor-like core structure, have been reported in the literature [reviewed in: Poirier D. (2003) Curr Med Chem. 10: 453-77]. The following recently published international application WO 2004/085457 disclose s a variety of estron derivatives with different subsituents in C3, C6, C16 and/or C17 position as potent 17β-HSD1 inhibitors].

A further well characterized member of the 17β-HSD family is the 17β-HSD type 3 enzyme (17β-HSD3). The 17β-HSD3 has a distinct feature compared to other 17HSDs: it is found to be expressed almost exclusively in the testis, whereas the other iso-enzymes are expressed more widely in several tissues. 17β-HSD3 has a crucial role in androgen biosynthesis. It converts 4-androstene-3,17-one (A) to testosterone (T). The biological significance of the 17β-HSD3 is of undeniable physiological importance. Mutations in the gene for 17β-HSD3 have found to lead to decreased T formation in the fetal testis and consequently to a human intersex disorder termed male pseudohermaphroditism [Geissler WM et al. (1994) Nat Genet., 7:34-9].

With regard to the indication prostate cancer, the primary cancer cells mostly retain their responsiveness to androgens in their regulation of proliferation, differentiation, and programmed cell death for some period. At present, androgen deprivation is the only effective systemic hormonal therapy available for prostate cancer. The development of selective inhibitors against 17β-HSD3 is a new therapeutic approach for the treatment of androgen dependent disease [Labrie et al. (2000) Trends Endocrinol Metab., 11:421-7]. Furthermore, Oefelein et al. reported that the depot GnRH analogue fails, in nearly 20% of cases, to achieve castrate levels of T in men [Oefelein MG & Cornum R (2000) J Urol.; 164:726-9]. In order to improve the response rate to endocrine therapy for men with prostate cancer it may be important to selectively inhibit testicular 17β-HSD3 activity. Besides prostate cancer, many other androgen-sensitive diseases, i.e. diseases whose onset or progress is aided by androgenic activity, may be treated by selectively inhibiting 17β-HSD3 activity. These diseases include but are not limited to benign prostatic hyperplasia, prostatitis, acne, seborrhea, hirsutism, androgenic alopecia, precocious puberty, adrenal hyperplasia, and polycystic ovarian syndrome. Furthermore, considering the fact that 17β-HSD3 is found mainly in the testis, the development of potent inhibitors could be of interest for blocking spermatogenesis and as an anti-fertility agent for males.

Several reversible or irreversible inhibitors of the 17β-HSD3 enzymes of steroidal and even non-steroidal origin are already known from the literature. The characteristics of these inhibitory molecules have been reported in the literature [reviewed in: Poirier D. (2003) Curr Med Chem. 10:453-77]. For example, US patent No. 6,541,463 discloses androsterone derived inhibitors for 17β-HSD3. These derivatives have been synthesised by parallel solid- and liquid-phase chemistry and some of these compounds showed 2 to 18-fold higher inhibition activity than that of the natural substrate of the enzyme, A-dione, used itself as a inhibitor. Furthermore, the international patent application WO 01/42181 discloses benzyl-tetralins, the chemical structure of which is related to that of the phytoestrogen biochanin, as 17β-HSD3 inhibitors. Furthermore, international patent applications WO 98/32724, WO 98/30556 and WO 99/12540 disclose tetralone, benzopyrane and benzofuranone derivatives, which have a 17β-HSD inhibitory activity, for the treatment of hormone sensitive diseases.

Microsomal 17β-hydroxysteroid dehydrogenase of human endometrium and placenta (designated 17β-HSD type 2 or 17β-HSD2) was cloned by expression cloning and found to be equally active using androgens and estrogens as substrates for oxidation [Andersson S. (1995) J. Steroid Biochem. Molec. Biol., 55:533-534]. The recombinant 17β-HSD2 converts the highly active 17β-hydroxysteroids such as estradiol (E2), testosterone (T), and dehydrotestosterone (DHT) to their inactive keto forms. In addition, the 17β-HSD2 can, to a lesser extent, also convert 20β-hydroxyprogesterone (20βP) to progesterone (P). The broad tissue distribution together with the predominant oxidative activity of 17β-HSD2 suggest that the enzyme may play an essential rote in the inactivation of highly active 17β-hydroxysteroids, resulting in diminished sex hormone action in target tissues. Dong and colleagues showed significant 17β- HSD2 activity in cultured human osteoblasts and osteoblast-like osteosarcoma cells MG63 and TE85, but not in SaOS-2 [Dong Y et al. (1998) J. Bone Min. Res., 13:1539-1546]. The potential for interconversion of E1 to E2, T to A, and DHT to A by bone cells could therefore represent important mechanism for the local regulation of intracellular ligand supply for the estrogen and androgen receptors in the osteoblasts and other steroid sensitive cells. This modulation of steroid levels may be employed for a wide variety of indications, including the following: for the prevention and treatment of osteoporosis, for the treatment of ovarian cancer, for the treatment of breast cancer, for the treatment of endometrial cancer, for the treatment of endometriosis, for the treatment of prostate cancer and/or for the treatment of androgen-dependent hair-loss.

Several reversible or irreversible inhibitors of the 17β-HSD2 enzymes of steroidal and even non-steroidal origin are already known from the literature. The characteristics of these inhibitory molecules have been reported in the literature [reviewed in: Poirier D. (2003) Curr Med Chem. 10:453-77]. In addition, the international patent application WO 02/26706 discloses 17β-HSD2 inhibitors of non-steroidal origin.

Some thienopyrimidinones derivatives that are described as being useful in therapy have already been disclosed in the literature: The Japanese patent publication JP48042271 B (Yoshi-tomi Pharmaceutical industries Ltd.) disclose compounds useful as central nervous depressants and anti-inflammatory agents. The US patent No. 5,597,823 (Abbott Laboratories, Inc.) describes adrenergic antagonist useful in the treatment of benign prostate hyperplasia. The Japanese patent application JP62132884 (Mitsubishi Chem. Ind. Ltd.) discloses Benzylthieno-pyrimidinones useful as cardiovascular agents. Manhas et al describe the synthesis and antiinflammatory activity of some substituted thienopyrimidinones [Manhas MS et al. (1972) J Med Chem. 15(1):106-7]. Gadad et al. describe the synthesis and antihyperlipaemic activity of some 2-aminomethyl-3-aryl-5,6,7,8-tetrahydrobenzo(b) / 5,6-dimethylthieno (2,3-d)-pyrimidin-4-ones [Gadad AK et al. (1996) Arzeimittelforschung. 46(10):981-5]. Manjunath et al. describe the synthesis and evaluation of 2-chloromethyl-3-N-substituted arylthieno(2,3-d)pyrimidin-4-ones and derivatives for central nervous system depressant activity [Manjunath KS et al. (1997) Arzneimittelforschung. 47(9):1005-8].

Furthermore, several other thienopyrimidinone derivatives have been described but were not related to any medical use so far. For example, the compounds (3-Benryl-7-tert-butyl-4-oxo-3,4,5,6,7,8-hexahydro-benzo[4,5]thieno [2,3-d]pyrimidin-2-yl)-acetic acid methyl ester (CAS reg. no 423749-31-9) and 2,3-Dibezyl-7-tert-butyl-5,6,7,8-tetrahydin-3H-benzo[4,5]thieno[2,3-d]pyrimidin-4-one (CAS reg. no 372974-51-1) are commercially available.

In addition, closely related thienopyrimidinone derivatives are disclosed in unpublished intemational application

s PCTIEP 2004/006230 and PCT/EP 2004/006231 being useful in the treatment and/or prevention of a steroid hormone dependent disease or disorder, particularly a steroid hormone dependent disease or disorder requiring the inhibition of the 17β-hydroxysteroid dehydrogenase (17HSD) type 1, type 2 or type 3 enzyme. However, at least some of these compounds are quite insoluble and have difficulties in membrane permeability.

As a consequence, there is still a need for the development of improved compounds that are selectively inhibiting the 17β-HSD1, 17β-HSD3 and/or 17β-HSD2 enzyme, while desirably failing to inhibit substantially other members of the 17β-HSD protein family, or other catalysts of sex steroid degradation or activation. In particular, it is an aim of the present invention to develop elective inhibitors of the 17β-HSD1 enzyme, whereby in addition the compounds have no or only pure antagonistic binding affinities to the estrogen receptor (both subtypes α and β).

SUMMARY OF THE INVENTION

Therefore, it is an object of the present invention to develop novel inhibitors of the 17β-HSD1 and 17β-HSD2 enzyme, which have valuable pharmacological properties and which are suited for the treatment of estrogen dependent diseases and disorders. It is a further object of the present invention to develop novel inhibitors of the 17β-HSD3 enzyme, which have valuable pharmacological properties and which are suited for the treatment of androgen dependent diseases and disorders.

It has now been found that the substituted benzothiophenepyrimidinone derivatives of the present invention would be valuable in therapy, especially in the treatment or prevention of steroid hormone dependent diseases or disorders, such as steroid hormone dependent diseases or disorders requiring the inhibition of 17β-hydroxysteroid dehydrogenase (HSD) enzymes. In particular, compounds of formula (I) represent potent inhibitors of the 17β-HSD1,17β-HSD3 and/or 17β-HSD2 enzyme and possess valuable pharmacological properties for the treatment and/or prophylaxis of malignant steroid dependent diseases or disorders such as breast cancer, prostate carcinoma, ovarian cancer, uterine cancer, endometrial cancer and endometrial hyperplasia, but also for the treatment and/or prophylaxis of benign steroid dependent diseases or disorders such as endometriosis, uterine fibroids, uterine leiomyoma, adenomyosis, dysmenorrhea, menorrhagia, metrorrhagia, prostadynia, benign prostatic hyperplasia, prostatitis, acne, seborrhea, hirsutism, androgenic alopecia, precocious puberty, adrenal hyperplasia, polycystic ovarian syndrome, or urinary dysfunction. Further estrogen-dependent diseases which may be treated and/or prevented with an effective amount of a compound of the invention are multiple sclerosis, rheumatoid arthritis, Alzheimer's disease, colon cancer, tissue wounds, skin wrinkles and cataracts. Furthermore, compounds of formula (I) may be useful for the prevention and treatment of osteoporosis, and for blocking spermatogenesis and as an anti-fertility agent for males.

Accordingly, the present invention relates to a compound having the structural formula (I)

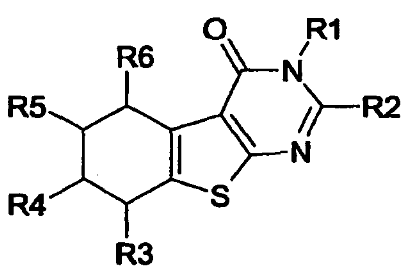

(I)

wherein

R1 and R2 are individually selected from the group consisting of

(i) -$C_1$-$C_{12}$-alkyl, which alkyl is linear, cyclic, branched or partially unsaturated, which is optionally substituted with one, two or three substituents individually selected from the group consisting of hydroxyl, $C_1$-$C_{12}$-alkoxy, thiol, $C_1$-$C_{12}$-alkylthio, aryloxy, arylacyl, -CO-OR, -O-CO-R, heteroaryl-acyloxy, and -N(R)$_2$;
wherein the aryl moiety is phenyl or naphthyl, and is optionally substituted with one, two or three halogen;
wherein the heteroaryl moiety is thienyl, furyl or pyridinyl
(ii) aryl and aryl-$C_1$-$C_{12}$-alkyl,
whereby the alkyl moiety is optionally substituted with one or two hydroxyl groups, and
whereby the aryl moiety is optionally substituted with one to five substituents individually selected from the

group consisting of halogen, hydroxyl, $C_1$-$C_{12}$-alkoxy, nitro, nitrile, $C_1$-$C_{12}$-alkyl, halogenated $C_1$-$C_{12}$-alkyl, -$SO_2$-N(R)$_2$, $C_1$-$C_{12}$-alkylsulphonyl;

or which aryl may be optionally substituted by two groups which are attached to adjacent carbon atoms and are combined into a saturated cyclic 5, 6 or 7-membered ring system, optionally containing one, two or three heteroatoms, such as N or O, the number of N atoms being 0-3 and the number of O atoms each being 0-2, whereby the cyclic ring system may optionally be further substituted by an oxo group;

(iii) heteroaryl and heteroaryl-$C_1$-$C_{12}$-alkyl, whereby the heteroaryl group is optionally substituted with one, two or three substituents

individually selected from the group consisting of halogen, $C_1$-$C_{12}$-alkyl, halogenated $C_1$-$C_8$-alkyl, -CO-OR, aryl or aryloxy,

whereby the aryl group is selected from phenyl or naphthyl and is optionally substituted with one, two or three halogen atoms;

(iv) cycloheteroalkyl and cycloheteroalkyl-$C_1$-$C_8$-alkyl,

whereby the cycloheteroalkyl moiety is selected from the group consisting of pyrrolidinyl, tetrahydrofuryl, tetrahydrothiophenyl, tetrahydropyridinyl, dioxolyl, azetidinyl, thiazolidinyl, oxazolidinyl, piperidinyl, morpholinyl, thiomorpholinyl, piperazinyl, azepanyl, diazepanyl, oxazepanyl, thiazepanyl, dihydro-1H-pyrrolyl, and 1,3-dihydro-benzoimidazolyl;

whereby the cycloheteroalkyl moiety is optionally substituted with up to two substituents individually selected from the group consisting of oxo, $C_1$-$C_{12}$-alkyl, hydroxyl, $C_1$-$C_{12}$-alkoxy and aryl-$C_1$-$C_{12}$-alkyl;

or R2 itself is independently selected from -CO-R, -CO-O-R, or -CO-N(R)$_2$;
whereby R1 and R2 cannot be simultaneously unsubstituted alkyl;

R3 and R4 are individually selected from the group consisting of hydrogen, oxo, thio, halogen or dihalogen, -CO-R, preferably CHO, -CO-O-R, nitrile, -CO-N(R)$_2$, -O-CO-R, -O-R, -S-R, -N(R)$_2$, -$C_1$-$C_{12}$-alkyl, which alkyl is linear, cyclic, branched or partially unsaturated, and which alkyl is optionally substituted with one, two or three substituents individually selected from the group consisting of hydroxyl, $C_1$-$C_{12}$-alkoxy, thiol, and -N(R)$_2$;

R5 and R6 are individually selected from the group consisting of hydrogen, halogen, -O-R, -CO-O-R, -CO-R, $C_1$-$C_{12}$-alkyl, which alkyl is linear, cyclic, branched or partially unsaturated, and which alkyl is optionally substituted with one, two or three substituents individually selected from the group consisting of hydroxyl, $C_1$-$C_{12}$-alkoxy, thiol, $C_1$-$C_{12}$-alkylthio, and - CO-OR; and

aryl and aryl-$C_1$-$C_{12}$-alkyl, whereby the aryl moiety is optionally substituted with one to five substituents individually selected from the group consisting of halogen, hydroxyl, $C_1$-$C_{12}$alkoxy, nitro, nitrile, $C_1$-$C_{12}$-alkyl, halogenated $C_1$-$C_{12}$-alkyl,

whereby R5 and R6 cannot be simultaneously hydrogen, and
whereby R6 is different from halogen, if R5 represents hydrogen;

wherein R represents hydrogen, $C_1$-$C_{12}$-alkyl or phenyl, the phenyl group being optionally substituted with one, two or three substituents selected from the group consisting of halogen, hydroxyl, and $C_1$-$C_4$-alkoxy; and
the hydrocarbon chain -C(R3)-C(R4)-C(R5)-C(R6)- of the six-membered ring is saturated or contains one or two double bonds between the carbon atoms,
or a pharmaceutically acceptable salt thereof.

According to a second aspect, the invention concerns at least one compound of formula (I) as defined herein for use as a medicament or for use in therapy.

According to an third aspect, the invention concerns a pharmaceutical composition comprising as active agent a compound of formula (I) as defined herein and at least a pharmaceutically acceptable carrier.

According to a fourth aspect, the invention concerns the use of a compound of formula (I) as defined herein for the manufacture of a medicament for the treatment or prevention of a steroid hormone dependent disease or disorder. Preferably, the steroid hormone dependent disease or disorder is a diseases or disorder requiring the inhibition of a 17β-hydroxysteroid dehydrogenase enzyme, preferably of the 17β-HSD type 1, 17β-HSD type 2 or 17β-HSD type 3.

DETAILED DESCRIPTION OF THE INVENTION

**Definitions:**

The following terms are used to describe various constituents of the chemical composition useful in this invention. The terms are defined as follows:

As used herein, the terms "comprising" and "including" are used herein in their open, nonlimiting sense.

The word "compound" shall here be understood to cover any and all isomers (e. g., enantiomers, stereoisomers, diastereomers, rotomers, and tautomers), racemates or any mixture of isomers, prodrugs, and any pharmaceutically acceptable salt of said compound.

Where the plural form is used for compounds, salts, and the like, this is taken to mean also a single compound, salt, or the like.

The term "substituted" means that the specified group or moiety bears one or more substituents. Where any group may carry multiple substituents and a variety of possible substituents is provided, the substituents are independently selected and need not be the same. The term "unsubstituted" means that the specified group bears no substituents. The term "optionally substituted" means that the specified group is unsubstituted or substituted by one or more substituents.

Any asymmetric carbon atoms may be present in the (R)-, (S)- or (R,S)-configuration, preferably in the (R)- or (S)-configuration, whichever is most active. Substituents at a double bond or a ring may be present in cis- (.=Z-) or trans (=E-) form.

The compounds of the present invention may contain asymmetric centers on the molecule, depending upon the nature of the various substituents. In certain instances, asymmetry may also be present due to restricted rotation about the central bond adjoining the two aromatic rings of the specified compounds. It is intended that all isomers (including enantiomers and diastereomers), either by nature of asymmetric centers or by restricted rotation as described above, as separated, pure or partially purified isomers or racemic mixtures thereof, be included within the ambit of the instant invention.

The term "halogen" refers to fluorine (F, Fluoro-), bromine (Br, Bromo-), chlorine (Cl, Chloro), and iodine (J, Iodo-) atoms. Preferred in the context of the present invention are Br, Cl and F. The terms "dihalogen", "trihalogen" and "perhalogen" refer to two, three and four substituents, respectively, each individually selected from the group consisting of fluorine, bromine, chlorine, and iodine atoms.

The term "hydroxyl" refers to the group -OH

The term "oxo" refers to the group =O

The term "thio" refers to the group =S

The term "thiol" refers to the group -SH

The term "sulfoxyl" refers to the group $-S(O)_{1-2}-$

The term "sulfamoyl" refers to the group $-SO_2-NH_2$

The term "nitro" refers to the group $-NO_2$

The term "nitrile" or "cyano" refers to the group-CN

For the purpose of the present invention, the carbon content of various hydrocarbon containing moieties is indicated by a prefix designating the minimum and maximum number of carbon atoms in the moiety, i.e., the prefix $C_i$-$C_j$ defines the number of carbon atoms present from the integer "i" to the integer "j" inclusive. Thus $C_1$-$C_4$-alkyl refers to alkyl of 1-4 carbon atoms, inclusive, or methyl, ethyl, propyl, butyl and isomeric forms thereof.

The term "alkyl" stands for a hydrocarbon radical which may be linear, cyclic or branched, with single or multiple branching, whereby the alkyl group comprises 1 to 12 carbon atoms. In one embodiment, the term "alkyl" stands for a linear or branched (with single or multiple branching) alkyl chain of 1 to 8 carbon atoms, exemplified by the term ($C_1$-$C_8$) alkyl, more preferably of 1 to 4 carbon atoms exemplified by the term ($C_1$-$C_4$)alkyl. The term ($C_1$-$C_8$)alkyl is further exemplified by such groups as methyl; ethyl; n-propyl; isopropyl; n-butyl; sec-butyl; isobutyl; tert-butyl; n-pentyl; isopentyl; neopentyl; tert-pentyl; 2- or 3-methylpentyl; n-hexyl; isohexyl, and the like. The alkyl group may be partially unsaturated, forming such groups as, for example, propenyl (allyl), methyl-propenyl, butenyl, pentenyl, pentinyl, hexenyl, octadienyl, and the like. The term "alkyl" further comprises cycloalkyl groups, preferably cyclo($C_3$-$C_8$)alkyl which refers to cyclopropyl, cyclobutyl, cyclopentyl, cyclohexyl, cycloheptyl, cyclooctyl and isomeric forms thereof such as methylcyclopropyl; 2- or 3-methylcyclobutyl; 2-, or 3-methylcyclopentyl, and the like. The cycloalkyl group may also be partly unsaturated, forming such groups as, for example, cyclohexenyl, cyclopentenyl, cyclooctadienyl, and the like. Furthermore, the term "alkyl" comprises a cycloalkyl-alkyl group comprising 4 to 12 carbon atoms, preferably "cyclo($C_3$-$C_8$)alkyl-($C_1$-$C_4$)alkyl" which refers to a alkyl group of 1 to 4 carbon atoms as described above substituted with a cyclo($C_3$-$C_8$)alkyl group as described above, forming such groups as for example cyclopropylmethyl, cyclohexylmethyl, cyclopentylethyl or cyclohexenylethyl.

The term "substituted alkyl" refers to alkyl as just described and substituted by up to five, more preferably by up to three, most preferably by one or two substituents independently selected from the group consisting of halogen, hydroxyl, thiol, nitro, nitrile, alkoxy, aryloxy, acyloxy, amino, amido, acylamino, alkylthio, arylthio, acyl, carboxyl, sulfamoyl, sulfonamide, and alkylsulfonyl, as defined herein. These groups may be attached to any carbon atom of the alkyl moiety. Substituted alkyl is preferably substituted with hydroxyl, halogen, $C_1$-$C_4$-alkoxy, arylacyl, preferably phenylacyl, phenoxy, benzyloxy, $C_1$-$C_4$-alkylthio, an alkylamino group -NR"$_2$, a carboxyl group -(C=O)-OR", an alkylacyloxy group -O-CO-R, or a heteroaryl acyloxy group - O-CO-HetAr, wherein R" represents hydrogen or $C_1$-$C_4$-alkyl. Preferably substituted alkyl, refers to substituted $C_1$-$C_4$-alkyl.

Halogenated alkyl, halogenated alkoxy and halogenated alkylithio are substituents in which the alkyl moieties (preferably ($C_1$-$C_6$)alkyl, more preferably ($C_1$-$C_4$)alkyl, and most preferably methyl) are substituted either partially or in full with halogens, generally with chlorine and/or fluorine. Preferred examples of such substituents are trifluoromethyl, trifluoromethoxy, trifluoromethylthio, dichloromethyl, pentafluoroethyl, dichloropropyl, fluoromethyl and difluoromethyl.

The term "alkoxy" refers to a group -OR, where R may be alkyl, arylalkyl, substituted arylalkyl, heteroarylalkyl or substituted heteroarylalkyl as defined herein, wherein the alkyl chain may be optionally further substituted as defined herein. Preferably, the term "alkoxy" refers to - O-($C_1$-$C_4$)alkyl (or ($C_1$-$C_4$)alkoxy), with the ($C_1$-$C_4$)alkyl group as defined above, or to -O-($C_1$-$C_4$)alkyl-phenyl, preferably benzoxy or phenethyloxy, optionally substituted in the aryl group with up to five independently selected substituents, in particular hydroxyl, halogen, ($C_1$-$C_4$)-alkyl, ($C_1$-$C_4$)-alkoxy, halogenated ($C_1$-$C_4$)-alkyl, or halogenated ($C_1$-$C_4$)-alkoxy, the number of said substituents being up to five for halogen, and up to three for any combination of said other substituents.

The term "aryloxy" refers to a group -OAr, where Ar may be aryl, substituted aryl, heteroaryl or substituted heteroaryl as defined herein. Preferably, Ar represents aryl as defined herein, which is optionally substituted in the aryl group with up to five independently selected substituents, in particular hydroxyl, halogen, ($C_1$-$C_4$)-alkyl, ($C_1$-$C_4$)-alkoxy, halogenated ($C_1$-$C_4$)-alkyl, or halogenated ($C_1$-$C_4$)-alkoxy; the number of said substituents being up to five for halogen, and up to three for any combination of said other substituents. Preferably, aryloxy refers to phenoxy, optionally substituted as defined above.

The term "acyloxy" refers to a group -O-CO-R, where R may be alkyl, arylalkyl, substituted arylalkyl, heteroarylalkyl, substituted heteroarylalkyl, aryl, substituted aryl, heteroaryl or substituted heteroaryl as defined herein, wherein the alkyl chain may be optionally further substituted as defined herein.

The term "alkylacyloxy" represents a preferred selection of the term "acyloxy" and refers to the group -O-CO-$C_1$-$C_{12}$-alkyl, preferably to -O-CO-$C_1$-$C_8$-alkyl, and most preferably to -O-CO-$C_1$-$C_4$-alkyl.

The term "arylacyloxy" represents a preferred selection of the term "acyloxy" and refers to the group -O-CO-Ar, wherein Ar represents aryl as defined herein, preferably phenyl, which is optionally substituted in the aryl group with up to five independently selected substituents, in particular hydroxyl, halogen, ($C_1$-$C_4$)-alkyl, ($C_1$-$C_4$)-alkooy, halogenated ($C_1$-$C_4$)-alkyl, or halogenated ($C_1$-$C_4$)-alkoxy; the number of said substituents being up to five for halogen, and up to three for any combination of said other substituents

The term "heteroaryl-acyloxy" represents a preferred selection of the term "acyloxy" and refers to the group -O-CO-HetAr, wherein HetAr represents heteroaryl as defined herein, preferably thienyl, furyl or pyridinyl.

The term "acyl" refers to a group -(C=O)-R, where R may be hydrogen, alkyl, aryl or aryl-($_1$-$C_4$)-alkyl (both optionally substituted in the aryl group with independently selected substituents as defined herein), heteroaryl or heteroaryl-($C_1$-$C_4$)alkyl (both optionally substituted in the heteroaryl group with up to three independently selected substituents as defined herein), as defined herein. Preferably, the term "acyl" refers to a group -(C=O)-R', where R' represents hydrogen, ($C_1$-$C_4$)alkyl, phenyl, or phenyl-($C_1$-$C_4$)alkyl, preferably benzyl.

The term "carbonyl" represents a preferred selection of the term "acyl" and refers to the group -CHO.

The term "alkylacyl" represents a preferred selection of the term "acyl" and refers to a group -(C=O)-alkyl, preferably -(C=O)-($C_1$-$C_4$)alkyl.

The term "arylacyl" represents a preferred selection of the term "acyl" and refers to the group -CO-Ar, wherein Ar represents aryl as defined herein, preferably phenyl, which is optionally substituted in the aryl group as defined herein.

The term "heteroarylacyl" represents a referred selection of the term "acyl" and refers to the group -CO-HetAr, wherein HetAr represents heteroaryl as defined herein, preferably thienyl, furyl or pyridinyl.

The term "carboxyl" refers to a group -(C=O)-OR, where R may be hydrogen, alkyl, substituted alkyl, aryl or aryl-($C_1$-$C_4$)-alkyl (both optionally substituted in the aryl group with independently selected substituents as defined herein), heteroaryl or heteroaryl-($C_1$-$C_4$)-alkyl (both optionally substituted in the heteroaryl group with independently selected substituents as defined herein), as defined herein. Preferably, the term "carboxyl" refers to a group -(C=O)-OR', where R' represents hydrogen, ($C_1$-$C_4$)alkyl, phenyl, or ($C_1$-$C_4$)alkyl-phenyl, preferably benzyl; whereby the phenyl moiety may be optionally substituted with substituents independently selected from the group consisting of hydroxyl, halogen, ($C_1$-$C_4$)alkoxy, ($C_1$-$C_4$)-alkyl, halogenated ($C_1$-$C_4$)alkyl and halogenated ($C_1$-$C_4$)alkoxy, the number of said substituents being up to five for halogen, and up to three for any combination of said other substituents.

The term "alkylcarboxyl" represents a preferred selection of the term "carboxyl" and refers to a group -(C=O)-OR, where R is hydrogen or $C_1$-$C_4$ alkyl.

The terms "alkylthio" (or "alkylsulfanyl") and "alkysulfonyl" ("alkylsuffoxyl") refers to a group -SR and -S(O)$_{n=1-2}$-R, respectively, where R may be alkyl, substituted alkyl, or arylalkyl, substituted arylalkyl, heteroarylalkyl or substituted heteroarylalkyl, as defined herein. Preferably, the term "alkylthio" ("alkylsulfanyl") refers to a group -SR'and the term "alkylsulfonyl" refers to a group -S(O)$_{n=1-2}$-R', respectively, where R' represents ($C_1$-$C_4$)alkyl, or ($C_1$-$C_4$)alkyl-phenyl, preferably benzyl; optionally substituted in the alkyl chain with up to threee substituents as defined herein, preferably hydroxyl, ($C_1$-$C_4$)-alkoxy or halogen.

The term "arylthio" ("arylsulfanyl") and "arylsulfonyl" refers to a group -S-Ar and -S(O)$_{n=1-2}$-Ar, respectively,

where Ar represents aryl, substituted aryl, heteroaryl or substituted heteroaryl, as defined herein. Preferably Ar represents aryl, which is optionally substituted in the aryl group with independently selected substituents as defined herein, in particular hydroxyl, halogen, ($C_1$-$C_4$)-alkyl, ($C_1$-$C_4$)alkoxy, halogenated ($C_1$-$C_4$)-alkyl, or halogenated ($C_1$-$C_4$)-alkoxy, the number of said substituents being up to five for halogen, and up to three for any combination of said other substituents. Preferably, arytthio refers to phenylsulfanyl, optionally substituted as defined above.

The term "amino" refers to the group -NRR', where R and R' may independently be hydrogen, alkyl (optionally substituted in the alkyl chain with up to five independently selected substituents as defined herein, in particular hydroxyl, halogen or ($C_1$-$C_4$)-alkoxy), aryl or aryl-($C_1$-$C_4$)-alkyl (both optionally substituted in the aryl group with up to five independently selected substituents as defined herein, in particular hydroxyl, halogen, ($C_1$-$C_4$)-alkyl, ($C_1$-$C_4$)-alkoxy, halogenated ($C_1$-$C_4$)-alkyl, or halogenated ($C_1$-$C_4$)-alkoxy, the number of said substituents being up to five for halogen, and up to three for any combination of said other substituents), heteroaryl or heteroaryl-($C_1$-$C_4$)-alkyl (both optionally substituted in the heteroaryl group with up to three independently selected substituents as defined herein), as defined herein.

The term "alkylamino" represents a preferred selection of the term "amino" and refers to the group -NRR', where R and R' may independently be hydrogen or ($C_1$-$C_4$)alkyl.

The term "amido" refers to the group -(C=O)-NRR', where R and R' may independently be hydrogen, alkyl (optionally substituted in the alkyl chain with up to five independently selected substituents as defined herein, in particular hydroxyl, halogen or ($C_1$-$C_4$)-alkoxy), aryl or aryl-($C_1$-$C_4$)-alkyl (both optionally substituted in the aryl group with independently selected substituents as defined herein, in particular hydroxyl, halogen, ($C_1$-$C_4$)-alkyl, ($C_1$-$C_4$)-alkoxy, halogenated ($C_1$-$C_4$)-alkyl, or halogenated ($C_1$-$C_4$)-alkoxy, the number of said substituents being up to five for halogen, and up to three for any combination of said other substituents), heteroaryl or heteroaryl-($C_1$-$C_4$)-alkyl (both optionally substituted in the heteroaryl group with up to three independently selected substituents as defined herein), as defined herein.

The term "alkylamido" represents a preferred selection of the term "amido" and refers to the group -(C=O)-NRR', where R and R' may be independently selected from hydrogen or ($C_1$-$C_4$)alkyl.

The term "acylamino" refers to the group -NR-CO-R', where R and R' may independently be hydrogen, alkyl (optionally substituted in the alkyl chain with up to five independently selected substituents as defined herein, in particular hydroxyl, halogen or ($C_1$-$C_4$)-alkoxy), aryl or aryl-($C_1$-$C_4$)-alkyl (both optionally substituted in the aryl group with independently selected substituents as defined herein, in particular hydroxyl, halogen, ($C_1$-$C_4$)-alkyl, ($C_1$-$C_4$)-alkoxy, halogenated ($C_1$-$C_4$)-alkyl, or halogenated ($C_1$-$C_4$)-alkoxy, the number of said substituents being up to five for halogen, and up to three for any combination of said other substituents), heteroaryl or heteroaryl-($C_1$-$C_4$)-alkyl (both optionally substituted in the heteroaryl group with up to three independently selected substituents as defined herein), as defined herein.

The term "carbonylamino" represents a preferred selection of the term "acylamino" and refers to the group -NR-CO-$CH_2$-R', where R and R' may be independently selected from hydrogen or ($C_1$-$C_4$)alkyl.

The term "sulfonamide" refers to the group -$SO_2$-NRR', wherein R and R' may independently be selected from hydrogen or ($C_1$-$C_4$)alkyl.

The term "aryl" refers to an aromatic carbocyclic group comprising 6 to 14, more preferably 6 to 10, carbon atoms and having at least one aromatic ring or multiple condensed rings in which at least one ring is aromatic. Preferably, aryl is phenyl, naphthyl, indanyl, indenyl, fluorenyl, 1,2,3,4-tetrahydro-naphthalen-1-yl or even biphenyl.

The term "heteroaryl" refers to an aromatic carbocyclic group of having a single 4 to 8 membered ring or multiple condensed rings comprising 6 to 14, more preferably 6 to 10, ring atoms and containing at least one heteroatom, such as N, O or S, within at least one ring, the number of N atoms being 0-3 and the number of O and S atoms each being 0-1; in which group at least one heterocyclic ring is aromatic. Examples of such groups include pyrrolyl, thienyl, furyl, imidazolyl, thiazolyl, isothiazolyl, oxazolyl, isoxazolyl, pyrazolyl, pyridinyl, pyrimidinyl, pyrazinyl, indolyl, quinolinyl, isoquinolinyl, benzoimidazolyl, 1,3-dihydro-benzoimidazolyl, benzofuryl, benzo[b]thiophene and the like. Preferably, heteroaryl is quinolinyl, furyl, benzoimidazolyl, pyridinyl, thienyl, indolyl, benzo[b]thiophene, pyridinyl, imidazolyl, pyrazolyl or thiazolyl.

The aryl and the heteroaryl group may optionally be substituted by substituents independently selected from the group consisting of halogen, hydroxyl, ($C_1$-$C_6$)alkoxy, ($C_1$-$C_6$)alkyl, halogenated ($C_1$-$C_6$)alkyl, halogenated ($C_1$-$C_6$) alkoxy, oxo, thiol, nitro, nitrile, sulfamoyl, sulfonamide, carboxyl, aryloxy or arylalkyloxy (both optionally substituted in the aryl moiety with indepndently selected substituents as defined herein), ($C_1$-$C_6$)alkylthio, arylthio or arylalkylthio (both optionally substituted in the aryl moiety with independently selected substituents as defined herein), amino, amido, acyl, and acylamino, as defined herein, the number of said substituents being up to five for halogen, and up to three for any combination of said other substituents. The heteroaryl group may further be optionally substituted with an aryl or an aryloxy group, which aryl group may be optionally substituted in the aryl moiety with substituents, especially hydroxyl, halogen, ($C_1$-$C_6$)alkoxy, ($C_1$-$C_6$)alkyl, halogenated ($C_1$-$C_6$)alkyl or halogenated ($C_1$-$C_6$)alkoxy, the number of said substituents being up to five for halogen, and up to three for any combination of said other substituents. The aryl group may further be optionally substituted with a heteroaryl group.

Substituted aryl is preferably substituted by substituents selected from the group consisting of ($C_1$-$C_6$)alkoxy, preferably methoxy, hydroxyl, ($C_1$-$C_4$)alkyl, halogen, halogenated ($C_1$-$C_4$)alkyl, preferably halogenated methyl, most

preferably trifluoromethyl, halogenated ($C_1$-$C_6$)alkoxy, and sulfonamide, the number of said substituents being up to five for halogen, and up to four, preferably up to three, for any combination of said other substituents. Preferably substituted aryl is substituted phenyl.

The aryl may be further substituted by two groups which are attached to adjacent carbon atoms and are combined into a saturated or partly unsaturated cyclic 5, 6, 7, or 8 membered ring system, optionally containing up to three heteroatoms, such as N, O or S, the number of N atoms being 0-3 and the number of O and S atoms each being 0-2. Preferably, the two groups which are attached to adjacent carbon atoms, are combined into a saturated cyclic 5 or 6 membered ring system, optionally containing up to three heteroatoms, such as N or O, the number of N atoms being 0-3 and the number of O atoms each being 0-2. This cyclic ring system may optionally be further substituted by an oxo group. Preferred examples of such a substituted aryl groups are benzo[1,3]dioxol, 2,3-dihydrobenzofuran, 3H-isobenzofuran-1-one and 1,3-dihydro-benzoimidazol-2-one.

Substituted heteroaryl is preferably substituted by up to three, preferably up to two substituents selected from the group consisting of halogen, ($C_1$-$C_4$)-alkoxy, ($C_1$-$C_4$)-alkyl, preferably methyl, ($C_1$-$C_4$)-alkyl-carboxyl, preferably carboxylmethylester, halogenated ($C_1$-$C_4$)-alkyl, preferably halogenated methyl, halogenated ($C_1$-$C_4$)-alkoxy, phenoxy (optionally substituted with up to three, preferably one halogen), benzyloxy, benzyl or phenyl.

The term "cycloheteroalkyl" refers to a four- to eight-membered heterocyclic ring containing at least one heteroatom, such as N, O or S, the number of N atoms being 0-3 and the number of O and S atoms each being 0-1, which system may be saturated, partly unsaturated or hydroaromatic, and which ring can be part of a multiple condensed ring-system in which some rings may be aromatic. Examples of such cycloheteroalkyls include pyrrolidinyl, tetrahydrofuryl, tetrahydrothiophenyl, tetrahydropyridinyl, dioxolyl, azetidinyl, thiazolidinyl, oxazolidinyl, piperidinyl, morpholinyl, thiomorpholinyl, piperazinyl, azepanyl, diazepanyl, oxazepanyl, thiazepanyl, dihydro-1H-pyrrolyl, 1,3-dihydro-benzoimidazolyl and the like. Preferred examples of such cycloheteroalkyl groups are pyrrolidinyl, morpholinyl, tetrahydrofuryl, piperidinyl or dioxolyl.

The cycloheteroalkyl group may optionally be substituted by up to three substituents, independently selected from the group consisting of oxo, alkyl, aryl or aryl-($C_1$-$C_4$)-alkyl (both optionally substituted in the aryl group with independently selected substituents as defined herein), hydroxyl, ($C_1$-$C_6$)alkoxy, halogenated ($C_1$-$C_6$)alkyl, halogenated ($C_1$-$C_6$)alkoxy, carboxyl-($C_1$-$C_6$)alkyl, thiol, nitrile, sulfamoyl, sulfonamide, carboxyl, aryloxy or arylalkyloxy (both optionally substituted in the aryl moiety with independently selected substituents as defined herein), ($C_1$-$C_6$)alkylthio, arylthio or arylalkylthio (both optionally substituted in the aryl moiety with independently selected substituents as defined herein), amino, amido, acyl, and acylamino, as defined herein. These groups may be attached to any carbon atom of the cycloheteroalkyl moiety. Substituted cycloheteroalkyl is preferably substituted with oxo, ($C_1$-$C_4$)alkyl, preferably methyl, phenyl and/or ($C_1$-$C_4$)alkylphenyl, in particular benzyl.

The term "arylalkyl" refers to an alkyl group substituted with up to three independently selected aryl groups; preferably the term "arylalkyl" refers to "aryl-($C_1$-$C_4$)-alkyl" or diaryl-($C_1$-$C_4$)-alkyl, whereby the aryl is an aryl group as defined above. Arylalkyl is preferably benzyl (-$CH_2$-Phenyl) or phenethyl (-$CH_2$-$CH_2$-Phenyl).

The term "substituted arylalkyl" refers to an arylalkyl group as defined above, wherein the aryl group is substituted as defined above.

The term "heteroarylalkyl" refers to an alkyl group substituted with up to three independently selected heteroaryl groups; preferably the term "heteroarylalkyl" refers to "heteroaryl-($C_1$-$C_4$)-alkyl", whereby the heteroaryl is a heteroaryl group as defined above.

The term "substituted heteroarylalkyl" refers to a heteroarylalkyl group as defined above, wherein the heteroaryl group is substituted as defined above.

The term "cycloheteroalkyl-alkyl" refers to an alkyl group substituted with up to three independently selected cycloheteroalkyl groups; preferably the term "cycloheteroalkyl-alkyl" refers to "cycloheteroalkyl-($C_1$-$C_4$)-alkyl", whereby the cycloheteroalkyl is a cycloheteroalkyl group as defined above. Preferably, "cycloheteroalkyl-alkyl" is morpholinylethyl, morpholinylpropyl, piperidinylethyl, tetrahydrofurylmethyl or pyrrolidinylpropyl.

The term "substituted cycloheteroalkyl-alkyl" refers to a cyclohoteroalkyl-alkyl group as defined above, wherein the cycloheteroalkyl-alkyl group is substituted as defined above. Preferably, "substituted cycloheteroalkyl-alkyl" is dimethyl-[1,3]-dioxolylmethyl or 2-oxo-pyrrolidinylpropyl.

The term "pro-drug" as used herein, represents derivatives of the compounds of the invention that are drug precursors which, following administration to a patient, release the drug in vivo via a chemical or physiological process. In particular, pro -drugs are derivatives of the compounds of the invention in which functional groups carry additional substituents which may be cleaved under physiological conditions in vivo and thereby releasing the active principle of the compound (e. g., a pro-drug on being brought to a physiological pH or through an enzyme action is converted to the desired drug form).

The term "pharmaceutically acceptable salts" refers to salt forms that are pharmacologically acceptable and substantially non-toxic to the subject being administered the compounds of the invention. Pharmaceutically acceptable salts of compounds of formula I include conventional and stoichiometrical acid-addition salts or base-addition salts

formed from suitable non-toxic organic or inorganic acids or inorganic bases. Add addition salts, for example, from compounds of formula I with a basic nitrogen atom are formed preferably with organic or inorganic adds. Suitable inorganic acids are, for example, halogenic acids such as hydrochloric acid, sulfuric acid, or phosphoric acid. Suitable organic acids are, for example, carboxylic, phosphonic, or sulfonic acids, for example acetic acid, propionic acid, glycolic acid, lactic acid, hydroxybutyric acid, malic acid, malenic acid, malonic acid, salicylic acid, fumaric acid, succinic acid, adipic acid, tartaric add, citric acid, glutaric acid, 2- or 3-glycerophosphoric acid and other mineral and carboxylic adds well known to those skilled in the art. The salts are prepared by contacting the free base forms with a sufficient amount of the desired acid to produce a salt in the conventional manner. Compounds containing acidic substituents may also form salts with inorganic or organic bases. Examples of suitable bases for salt formation include, but are not limited to, inorganic bases such as alkali or alkaline earth-metal (e.g., sodium, potassium, lithium, calcium, or magnesium) hydroxides, and those derived from ammonium hydroxides (e.g., a quaternary ammonium hydroxide such as tetramethylammonium hydroxide). Also contemplated are salts formed with pharmaceutical acceptable amines such as ammonia, alkyl amines, hydroxyalkylamines, N-methylglucamine, benzylamines, piperidines, and pyrrolidines and the like. Certain compounds will be acidic in nature, e. g. those compounds which possess a carboxyl or phenolic hydroxyl group. Salts of phenols can be made by heating acidic compounds with any of the above mentioned bases according to procedures well known to those skilled in the art.

As used herein, the term "composition" is intended to encompass a product comprising the specified ingredients in the specified amounts, as well as any product which results, directly or indirectly, from combination of the specified ingredients in the specified amounts.

The phrase "effective amount" as used herein, means an amount of a compound or composition which is sufficient enough to significantly and positively modify the symptoms and/or conditions to be treated (e. g., provide a positive clinical response). The effective amount of an active ingredient for use in a pharmaceutical composition will vary with the particular condition being treated, the severity of the condition, the duration of the treatment, the nature of concurrent therapy, the particular active ingredient(s) being employed, the particular pharmaceutically acceptable excipient(s)/carrier(s) utilized, and like factors within the knowledge and expertise of the attending physician.

**Preferred Embodiments**

Especially preferable compounds are those where the six-membered ring comprising the hydrocarbon chain -C(R3)-C(R4)-C(R5)-C(R6)- is an aromatic ring.

A further preferable embodiment related to compounds, wherein the six-membered ring comprising the hydrocarbon chain -C(R3)-C(R4)-C(R5)-C(R6)- is other than an aromatic ring.

Particularly preferable compounds of formula (I) are those where R2 is selected from the group consisting of

(i) -$C_1$-$C_8$-alkyl, which alkyl is linear, cyclic, branched or partially unsaturated, which is optionally substituted with one, two or three substituents individually selected from the group consisting of hydroxyl, $C_1$-$C_8$-alkoxy, thiol, $C_1$-$C_8$-alkylthio, aryloxy, - CO-O-$C_1$-$C_8$-alkyl, and -O-CO-R';
whereby said aryl group is phenyl or naphthyl, and is optionally substituted with one, two or three halogen atoms;
(ii) aryl and aryl-$C_1$-$C_8$-alkyl,
whereby the aryl moiety is optionally substituted with one to five substituents individually selected from the group consisting of halogen, hydroxyl, $C_1$-$C_8$-alkoxy, nitro, nitrile, halogenated $C_1$-$C_8$-alkyl, -$SO_2$-N(R')$_2$,
(iii) heteroaryl and heteroaryl-$C_1$-$C_8$-alkyl,
whereby the heteroaryl group is optionally substituted with one, two or three substituents individually selected from the group consisting of halogen, $C_1$-$C_8$-alkyl, halogenated $C_1$-$C_8$-alkyl, aryl or aryloxy,
whereby the aryl group is selected from phenyl or naphthyl and is optionally substituted with one, two or three halogen atoms;
(iv) -CO-R',
(v) -CO-N(R')$_2$, and
(vi) -CO-O-R';

wherein R' represents hydrogen or $C_1$-$C_8$-alkyl.

Most preferable compounds of formula (I) are those where R2 is

(a) a residue of formula (II)

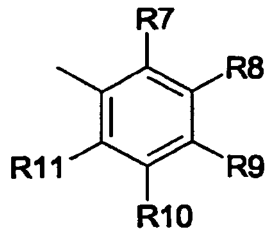

(II)

wherein

R7 is hydrogen, halogen, hydroxyl or $C_1$-$C_4$-alkoxy,
R8 is hydrogen, $C_1$-$C_4$-alkoxy, hydroxyl, nitrile, halogen, or halogenated $C_1$-$C_4$-alkyl,
R9 is hydrogen, $C_1$-$C_4$-alkoxy, hydroxyl, nitrile, halogen, or N,N-di-$C_1$-$C_4$-alkyl-sulphonamide
R10 is hydrogen, $C_1$-$C_4$-alkoxy, hydroxyl, nitrile, halogen, or halogenated $C_1$-$C_4$-alkyl, and
R11 is hydrogen, halogen, hydroxyl or $C_1$-$C_4$-alkoxy;

or (b)

(i) -$C_1$-$C_8$-alkyl, which alkyl is linear, cyclic, branched or partially unsaturated;
(ii) -$C_1$-$C_4$-alkyl, substituted with one or two substituents selected from the group consisting of
-CO-O-R";
-O-R";
-O-Ar, whereby Ar is phenyl optionally substituted with halogen;
-O-CO-R",
phenyl or biphenyl, optionally substituted in the phenyl moiety with one, two or three $C_1$-$C_4$-alkoxy groups;
(iii) -CO-O-R",
(iv) -CO-R",
(v) naphthyl,
(vi) heteroaryl,
whereby the heteroaryl group is optionally substituted by one or two substituents individually selected from the group consisting of halogen, $C_1$-$C_4$-alkyl, halogenated $C_1$-$C_4$-alkyl, phenyl and phenoxy,
whereby the phenyl group is optionally substituted with one, two or three halogen; wherein R" represents hydrogen or $C_1$-$C_4$-alkyl.

Preferred R2 substituents are those where'R2 is selected from the group consisting of

(i) -$C_3$-$C_8$-alkyl, which alkyl is linear, cyclic or branched,
(ii) -$C_1$-$C_4$-alkyl, optionally substituted with one or two substituents independently selected from the group consisting of $C_1$-$C_4$-alkoxy and hydroxyl,
(iii) phenyl-$C_1$-$C_4$-alkyl,
wherein the phenyl group is optionally substituted with one or two $C_1$-$C_4$-alkoxy groups,
(iv) heteroaryl,
which heteroaryl moiety is selected from the group consisting of furyl, pyridinyl, thienyl, thiazolyl and pyrimidinyl,
(v) a residue of formula (II)

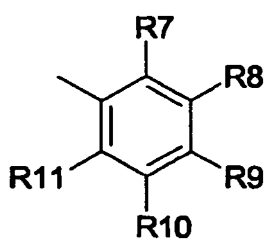

(II)

wherein

R7 is hydrogen, $C_1$-$C_4$-alkoxy or halogen,
R8 is hydrogen, halogen, $C_1$-$C_4$-alkoxy, or hydroxyl,
R9 is hydrogen, hydroxyl or $C_1$-$C_4$-alkoxy,
R10 is hydrogen, halogen, $C_1$-$C_4$-alkoxy, or hydroxyl, and
R11 is hydrogen, $C_1$-$C_4$-alkoxy or halogen.

Particularly preferable compounds of formula (I) are those where R1 is selected from the group consisting of

(i) -$C_1$-$C_8$-alkyl, which alkyl is linear, cyclic, branched or partially unsaturated,
which is optionally substituted with one, two or three substituents individually selected from the group consisting of hydroxyl, $C_1$-$C_8$-alkoxy, thiol, -$NH_2$, $C_1$-$C_8$-alkylthio, aryloxy, arylacyl, -CO-O-$C_1$-$C_8$-alkyl, $C_1$-$C_8$-alkylacyloxy, heteroaryl-acyloxy, and $C_1$-$C_8$-alkylamino;
whereby said aryl group is phenyl or naphthyl, and is optionally substituted with one, two or three halogen;
whereby said heteroaryl group is thienyl, furyl or pyridinyl,
(ii) aryl and aryl-$C_1$-$C_8$-alkyl,
wherein the alkyl moiety is optionally substituted with one or two hydroxyl groups, and
wherein the aryl moiety is optionally substituted with one to five substituents individually selected from the group consisting of halogen, hydroxyl, $C_1$-$C_8$-alkoxy, $C_1$-$C_8$-alkylsulphonyl, -$SO_2$-N($C_1$-$C_8$-alkyl)$_2$, $C_1$-$C_8$-alkyl, halogenated $C_1$-$C_8$-alkyl;
or which aryl may be optionally substituted by two groups which are attached to adjacent carbon atoms and are combined into a saturated cyclic 5 or 6-membered ring system, optionally containing one, two or three heteroatoms, such as N or O, the number of N atoms being 0-3 and the number of O atoms each being 0-2, whereby the cyclic ring system may optionally be further substituted by an oxo group;
(iii) heteroaryl and heteroaryl-$C_1$-$C_8$-alkyl,
whereby the heteroaryl group is optionally substituted with one, two or three substituents individually selected from the group consisting of halogen, $C_1$-$C_8$-alkyl, and -CO-O-$C_1$-$C_8$-alkyl;
(iv) cycloheteroalkyl and cycloheteroalkyl-$C_1$-$C_8$-alkyl,
whereby the cycloheteroalkyl moiety is optionally substituted with up to two substituents individually selected from the group consisting of oxo, $C_1$-$C_8$-alkyl, hydroxyl, $C_1$-$C_8$-alkoxy and aryl-$C_1$-$C_8$-alkyl.

Most preferable compounds of formula (I) are those where R1 is selected from the group consisting of

(i) -$C_1$-$C_8$-alkyl, which alkyl is linear, cyclic or branched,
(ii) -$C_1$-$C_4$-alkyl, substituted with one or two substituents independently selected from the group consisting of -O-R"; -O-Ar, -O-CO-HetAr, -CO-Ar, -CO-O-R", and -N(R")$_2$,
(iii) aryl and aryl-$C_1$-$C_4$-alkyl,
wherein the alkyl moiety is optionally substituted with a hydroxyl group; and
wherein the aryl moiety is optionally substituted with one, two or three substituents individually selected from the group consisting of halogen, -O-R"; -$SO_2$-R", -$SO_2$-N(R")$_2$,
or which aryl may be optionally substituted by two groups which are attached to adjacent carbon atoms and are combined into a saturated cyclic 5 or 6 membered ring system, optionally containing up to two O atoms,
whereby the cyclic ring system may optionally be further substituted by an oxo group;
(iv) heteroaryl and heteroaryl-$C_1$-$C_4$-alkyl,
whereby the heteroaryl group is optionally substituted with one or two substituents individually selected from the group consisting of halogen, -$C_1$-$C_4$-alkyl, and -CO-O-R";
(v) cycloheteroalkyl and cycloheteroalkyl-$C_1$-$C_4$-alkyl,
whereby the cycloheteroalkyl moiety is optionally substituted with up to two substituents individually selected from the group consisting of oxo, $C_1$-$C_4$-alkyl, preferably methyl, and -$C_1$-$C_4$-alkyl-Ar,

wherein

Ar represent phenyl, optionally substituted with halogen or $C_1$-$C_4$-alkoxy,
HetAr represents thienyl, furyl, or pyridinyl, and
R" represents hydrogen or $C_1$-$C_4$-alkyl.

Preferred R1 substituents are those where R1 is selected from the group consisting of

(i) -$C_1$-$C_4$-alkyl, optionally substituted with one or two hydroxy groups,
(ii) phenyl-$C_1$-$C_4$-alkyl,
wherein the phenyl group is optionally substituted with one or two $C_1$-$C_4$-alkoxy groups, or wherein the phenyl group is substituted by two groups which are attached to adjacent carbon atoms and are combined into a saturated cyclic 5 or 6-membered ring system, optionally containing one or two O-atoms;
(iii) heteroaryl or heteroaryl-$C_1$-$C_4$-alkyl,
which heteroaryl moiety is selected from the group consisting of furyl, pyridinyl, thienyl, thiazolyl and pyrimidinyl, wherein the heteroaryl group is optionally substituted with $C_1$-$C_4$-alkyl, and
(iv) cycloheteroalkyl-$C_1$-$C_4$-alkyl;
which cycloheteroalkyl moiety is selected from the group consisting of tetrahydrofuryl, piperidinyl, morpholinyl, and pyrrolidinyl.

Mostly preferred are compounds, wherein R1 and R2 are independently selected from the group consisting of

(i) $C_3$-$C_8$-alkyl, which alkyl is linear, cyclic or branched,
(ii) -$C_1$-$C_4$-alkyl, optionally substituted with one or two substituents independently selected from the group consisting of $C_1$-$C_4$-alkoxy and hydroxyl,
(iii) phenyl or phenyl-$C_1$-$C_4$-alkyl,
wherein the phenyl group is optionally substituted with one to five substituents individually selected from the group consisting of halogen, $C_1$-$C_4$-alkoxy, and hydroxyl,
or wherein the phenyl group is substituted by two groups which are attached to adjacent carbon atoms and are combined into a saturated cyclic 5 or 6-membered ring system, optionally containing one or two heteroatoms, such as N or O;
(iv) heteroaryl or heteroaryl-$C_1$-$C_4$-alkyl,
wherein the heteroaryl group is optionally substituted with $C_1$-$C_4$-alkyl, and
(v) cycloheteroalkyl-$C_1$-$C_4$-alkyl;

Particularly preferable compounds of formula (I) are those, wherein R3 is selected from the group consisting of hydrogen, oxo, -O-R', -O-Ar, -O-CO-R', halogen, thio, -S-R', and -S-Ar,
wherein
R' represents hydrogen or $C_1$-$C_4$-alkyl, and
Ar represents phenyl, optionally substituted with one or more substituents selected from the group consisting of halogen, hydroxyl or $C_1$-$C_4$-alkoxy. Mostly preferred R3 substituents are those where R3 is selected from the group consisting of hydrogen, oxo, and hydroxyl.

More preferable compounds of formula (I) are those, where R4 is selected from the group consisting of hydrogen, $C_1$-$C_4$-alkyl, optionally substituted with hydroxyl; -CO-R', -CO-O-R', halogen and dihalogen,
wherein R' represents hydrogen or $C_1$-$C_4$-alkyl.

Preferred R4 substituents are those where R4 is selected from the group consisting of hydrogen and halogen.

Particularly preferable compounds of formula (I) are those where R5 is selected from the group consisting of hydrogen, -COOR', phenyl-$C_1$-$C_4$-alkyl, $C_1$-$C_4$-alkyl, and $C_1$-$C_4$-alkyl substituted with -COOR', wherein R' represents H or $C_1$-$C_4$-alkyl.

Preferred compounds of formula (I) are further those wherein R5 is selected from the group consisting of hydrogen, benzyl and $C_1$-$C_4$-alkyl.

Preferred R6 substituents are those where R6 is selected from the group consisting of hydrogen, halogen, -O-R', phenyl-$C_1$-$C_4$-alkyl, $C_1$-$C_4$-alkyl, and $C_1$-$C_4$-alkyl substituted with - COOR', wherein R' represents H or $C_1$-$C_4$-alkyl.

Mostly preferred R6 substituents are those where R6 is selected from the group consisting of hydrogen, halogen, benzyl, and $C_1$-$C_4$-alkyl.

Especially preferable compounds of formula (I) are the compounds of formula (I)

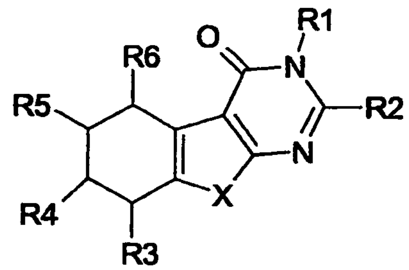

(I)

wherein

R1 and R2 are independently selected from the group consisting of:

(i) $C_3$-$C_8$-alkyl, which alkyl is linear, cyclic or branched,
(ii) -$C_1$-$C_4$-alkyl, optionally substituted with one or two substituents independently selected from the group consisting of $C_1$-$C_4$-alkoxy and hydroxyl,
(iii) phenyl or phenyl-$C_1$-$C_4$-alkyl,
wherein the phenyl group is optionally substituted with one to five substituents individually selected from the group consisting of halogen, $C_1$-$C_4$-alkoxy, and hydroxyl,
or wherein the phenyl group is substituted by two groups which are attached to adjacent carbon atoms and are combined into a saturated cyclic 5 or 6-membered ring system, optionally containing one or two heteroatoms, such as N or O;
(iv) heteroaryl or heteroaryl-$C_1$-$C_4$-alkyl,
wherein the heteroaryl group is optionally substituted with $C_1$-$C_4$-alkyl, and
(v) cycloheteroalkyl-$C_1$-$C_4$-alkyl;

R3 is selected from the group consisting of hydrogen, oxo, and hydroxyl;
R4 is selected from the group consisting of hydrogen and halogen,
R5 is selected from the group consisting of hydrogen, phenyl-$C_1$-$C_2$-alkyl, $C_1$-$C_4$-alkyl and -$C_1$-$C_4$-alkyl-CO-O-$C_1$-$C_4$-alkyl; and
R6 is selected from the group consisting of hydrogen, halogen, phenyl-$C_1$-$C_2$-alkyl, $C_1$-$C_4$-alkyl and -$C_1$-$C_4$-alkyl-CO-O-$C_1$-$C_4$-alkyl.

Even more preferred are compounds, wherein R1 is selected from the group consisting of

(i) -$C_1$-$C_4$-alkyl, optionally substituted with one or two hydroxy-groups,
(ii) phenyl-$C_1$-$C_4$-alkyl,
wherein the phenyl group is optionally substituted with one or two $C_1$-$C_4$-alkoxy groups, or wherein the phenyl group is substituted by two groups which are attached to adjacent carbon atoms and are combined into a saturated cyclic 5 or 6-membered ring system, optionally containing one or two O-atoms;
(iii) heteroaryl or heteroaryl-$C_1$-$C_4$-alkyl,
which heteroaryl moiety is selected from the group consisting of furyl, pyridinyl, thienyl, thiazolyl and pyrimidinyl, wherein the heteroaryl group is optionally substituted with $C_1$-$C_4$-alkyl, and
(iv) cycloheteroalkyl-$C_1$-$C_4$-alkyl;
which cycloheteroalkyl moiety is selected from the group consisting of tetrahydrofuryl, piperidinyl, morpholinyl, and pyrrolidinyl;

and wherein R2 is selected from the group consisting of

(i) $C_3$-$C_8$-alkyl, which alkyl is linear, cyclic or branched,
(ii) -$C_1$-$C_4$-alkyl, optionally substituted with one or two substituents independently selected from the group consisting of $C_1$-$C_4$-alkoxy and hydroxyl,
(iii) phenyl-$C_1$-$C_4$-alkyl,
wherein the phenyl group is optionally substituted with one or two $C_1$-$C_4$-alkoxy groups,
(iv) heteroaryl,
which heteroaryl moiety is selected from the group consisting of furyl, pyridinyl, thienyl, thiazolyl and pyrimidinyl,

(v) a residue of formula (II)

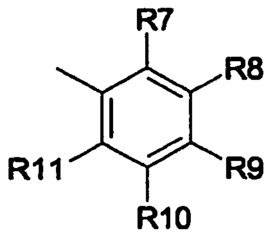

(II)

wherein

R7 is hydrogen, halogen or $C_1$-$C_4$-alkoxy,
R8 is hydrogen, halogen, $C_1$-$C_4$-alkoxy, or hydroxyl,
R9 is hydrogen, hydroxyl or $C_1$-$C_4$-alkoxy,
R10 is hydrogen, halogen, -$C_1$-$C_4$-alkoxy, or hydroxyl, and
R11 is hydrogen, halogen or $C_1$-$C_4$-alkoxy.

The following compounds and their use, respectively, are especially preferred in the context of the present invention:

**No. 1:** 3-Benzyl-5-methyl-2-(3,4,5-trimethoxyphenyl)-5,6,7,8-tetrahydro-3H-benzo[4,5]thieno[2,3-d]pyrimidin-4-one,
**No. 2:** 3-Benzyl-5-methyl-(2,3,4,5-trimethoxyphenyl)-6,7-dihydro-3H,5H-benzo[4,5]thieno[2,3-d]pyrimidin-4,8-dione,
**No. 3:** 3-Benzyl-7-bromo-2-(2-bromo-3,4,5-trimethoxyphenyl)-5-methyl-6,7-dihydro-3H,5H-benzo[4,5]thieno[2,3-d]pyrimidine-4,8-dione,
**No. 4:** 3-Benzyl-2-(2-bromo-3,4,5-trimethoxyphenyl)-8-hydroxy-5-methyl-3H-benzo[4,5]thieno[2,3-d]pyrimidin-4-one,
**No. 5:** 3-Benzyl-6-methyl-2-(3,4,5-trimethoxyphenyl)-5,6,7,8-tetrahydro-3H-benzo[4,5]thieno[2,3-d]pyrimidin-4-one,
**No. 6:** 3-Benzyl-6-methyl-2-(3,4,5-trimethoxyphenyl)-6,7-dihydro-3H,5H-benzo[4,5]thieno[2,3-d]pyrimidin-4,8-dione,
**No. 7:** 3-Benzyl-7-bromo-6-methyl-2-(2-bromo-3,4,5-trimethoxyphenyl)-6,7-dihydro-3H-benzo[4,5]thieno[2,3-d]pyrimidin-4,8-dione,
**No. 8:** 3-Benzyl-2-(2-bromo-3,4,5-trimethooyphenyl)-8-hydroxy-6-methyl-3H-benzo[4,5]thieno[2,3-d]pyrimidin-4-one,
**No. 9:** 3-Butyl-6-methyl-2-(3,4,5-trimethoxyphenyl)-5,6,7,8-tetrahydro-3H-benzo[4,5]thieno[2,3-d]pyrimidin-4-one,
**No. 10:** 3-Butyl-6-methyl-2-(3,4,5-trimethoxyphenyl)-6,7-dihydro-3H,5H-benzo[4,5]thieno[2,3-d]pyrimidin-4,8-dione,
**No. 11:** 3-Butyl-7-bromo-6-methyl-2-(2-bromo-3,4,5-trimethoxyphenyl)-6,7-dihydro-3H-benzo[4,5]thieno[2,3-d]pyrimidin-4,8-dione,
**No. 12:** 3-Butyl-2-(2-bromo-3,4,5-timethoxyphenyl)-8-hydroxy-6-methyl-3H-benzo[4,5]thieno[2,3-d]pyrimidin-4-one,
**No. 13:** 2-(2-Bromo-3-hydroxy-4,5-dimethoxyphenyl)-3-butyl-8-hydroxymethyl-3H-benzo[4,5]thieno[2,3-d]pyrimidin-4-one,
**No. 14:** 2-(2-Bromo-3,4-dihydroxy-5-methoxyphenyl)-3-butyl-8-hydroxy-6-methyl-3H-benzo[4,5]thieno[2,3-d]pyrimidin-4-one,
**No. 16:** 7-bromo-3-butyl-2-(2-bromo-3,4,5-trimethoxyphenyl)-8-hydroxy-6-methyl-3H-benzo[4,5]thieno[2,3-d]pyrimidin-4-one, and
**No. 17:** 7-Bromo-2-(2-bromo-3-hydroxy-4,5-dimethoxyphenyl)-3-butyl-8-hydroxy-8-methyl-3H-benzo[4,5]thieno[2,3-d]pyrimidin-4-one,

or a physiologically acceptable salt thereof.

The invention also relates to pharmaceutical compositions comprising one or more of the compounds of the invention for which no use in therapy has been disclosed earlier, or their salts , as active agent and at lease one

pharmaceutically acceptable carrier.

Furthermore, the invention relates to the use of an effective amount of a novel compound as defined herein for the manufacture of a medicament for the treatment or prevention of a steroid hormone dependent disease or disorder in a mammal, in particular a human. Preferably the steroid hormone dependent disease or disorder is an estradiol or testosterone dependent disease or disorder.

In a preferred embodiment, the invention relates to the use of an effective amount of a novel compound as defined within the present invention for the the manufacture of a medicament for the treatment or prevention of a steroid hormone dependent disease or disorder in a mammal, whereby the steroid hormone dependent disease or disorder requires the inhibition of a 17β-hydroxysteroid dehydrogenase (HSD) enzyme, preferably the human 17β-hydroxysteroid dehydrogenase (HSD) enzyme type 1, type 2 or type 3.

The steroid hormone dependent disease or disorder to be treated and/or prevented requires the lowering of the endogenous 17β-estradiol or testosterone concentration in a generalized and/or tissue specific manner.

The invention also relates to the manufacture of a medicament for treating a mammal such as a human having a condition related to 17β-hydroxysteroid dehydrogenase (HSD) type 1, type 2 or type 3 activity, comprising administering to the mammal an amount of a compound of this invention, or a salt thereof, which amount is effective to treat the condition. Administration of compounds of this invention in combination with other pharmaceuticals used in treatment of the listed conditions is contemplated.

The conditions to be treated and/or prevented in the context of the present invention include but are not limited to breast cancer, prostate carcinoma, ovarian cancer, uterine cancer, endometrial cancer, endometrial hyperplasia, endometriosis, uterine fibroids, uterine leiomyoma, adenomyosis, dysmenorrhea, menorrhagia, metrorrhagia, prostadynia, benign prostatic hyperplasia, prostatitis, acne, seborrhea, hirsutism, androgenic alopecia, precocious puberty, adrenal hyperplasia, polycystic ovarian syndrome, and urinary dysfunction. A further condition to be treated and/or prevented in the context of the present invention includes osteoporosis.

Further estrogen-dependent diseases which may be treated and/or prevented with an effective amount of a compound of the invention are multiple sclerosis, rheumatoid arthritis, Alzheimer's disease, colon cancer, tissue wounds, skin wrinkles and cataracts.

In a preferred embodiment the invention relates to use of an effective amount of a compound of the invention for the manufacture of a medicament for the treatment or prevention of one of the aforementioned disease or disorders in a mammal whereby the mammal is a human, preferably a female and most preferably a pre- or peri-menopausal female in the case of gynaecological disorders.

Furthermore, compounds of formula (I) may be useful for blocking spermatogenesis and as an anti-fertility agent for males.

The disclosed compounds are also useful as diagnostic agents (e.g. in diagnostic kits or for use in clinical laboratories) for screening for the presence or absence of 17β-hydroxysteroid dehydrogenase (HSD) type 1, type 2 and/or type 3 activity.

It will be appreciated that the methods of the present invention can be incorporated in the form of a variety of embodiments, only a few of which are disclosed herein. It will be apparent for the expert skilled in the field that other embodiments exist and do not depart from the spirit of the invention. Thus, the described embodiments are illustrative and should not be construed as restrictive.

**Administration forms**

The method of the invention is primarily intended for treatment in a mammal, preferably in humans and other primates, of steroid hormone dependent diseases or disorders, in particular estradiol dependent diseases or disorders, wherein the steroid hormone dependent disease or disorder preferably requires the inhibition of a 17β-hydroxysteroid dehydrogenase (HSD) enzyme, preferably the type 1 17β-hydroxysteroid dehydrogenase (HSD) enzyme [EC 1.1.1.62].

The compounds may be administered orally, dermally, parenterally, by injection, by pulmonal or nasal delivery, or sublingually, rectally or vaginally in dosage unit formulations. The term "administered by injection" includes intravenous, intraarticular, intramuscular (e.g. by depot injection where the active compounds are released slowly into the blood from the depot and carried from there to the target organs), intraperitoneal, intradermal, subcutaneous, and intrathecal injections, as well as use of infusion techniques. Dermal administration may include topical application or transdermal administration. One or more compounds may be present in association with one or more non-toxic pharmaceutically acceptable auxiliaries such as excipients, adjuvants (e.g. buffers), carriers, inert solid diluents, suspensing agents, preservatives, fillers, stabilizers, anti-oxidants, food additives, bioavailability enhancers, coating materials, granulating and disintegrating agents, binding agents etc., and, if desired, other active ingredients.

The pharmaceutical composition may be formulated for example as immediate release, sustained release, pulsatile release, two or more step release, depot or other kind of release formulations.

The manufacture of the pharmaceutical compositions according to the invention may be performed according

to methods known in the art and will be explained in further detail below. Commonly known and used pharmaceutically acceptable auxiliaries as well as further suitable diluents, flavorings, sweetening agents, coloring agents etc. may be used, depending on the intended mode of administration as well as particular characteristics of the active compound to be used, such as solubility, bioavailability etc. Suitable auxiliaries and further ingredients may be such as recommended for pharmacy, cosmetics and related fields and which preferably are listed in the European Pharmacopoeia, FDA approved or cited in the "GRAS" list (FDA List of food additives that are 'generally recognized as safe' (GRAS)).

One mode of application of the compounds of general formula (I) or of pharmaceutical compositions comprising one or more of said compounds is oral application, e. g., by tablets, pills, dragees, hard and soft gel capsules, granules, pellets, aqueous, lipid, oily or other solutions, emulsions such as oil-in-water emulsions, liposomes, aqueous or oily suspensions, syrups, elixiers, solid emulsions, solid dispersions or dispersible powders. For the preparation of pharmaceutical compositions for oral administration, the compounds suitable for the purposes of the present invention as defined above can be admixed with commonly known and used adjuvants and excipients such as for example, gum arabic, talcum, starch, sugars (such as, e. g., mannitose, methyl cellulose, lactose), gelatin, surface-active agents, magnesium stearate, aqueous or non-aqueous solvents, paraffin derivatives, cross-linking agents, dispersants, emulsifiers, lubricants, conserving agents, flavoring agents (e. g., ethereal oils), solubility enhancers (e. g., benzyl benzoate or benzyl alcohol) or bioavailability enhancers (e.g. Gelucire™). In the pharmaceutical composition, the active ingredients may also be dispersed in a microparticle, e. g. a nanoparticulate, composition.

For parenteral administration, the active agents can be dissolved or suspended in a physiologically acceptable diluent, such as, e. g., water, butter, oils with or without solubilizers, surface-active agents, dispersants or emulsifiers. As oils for example and without limitation, olive oil, peanut oil, cottonseed oil, soybean oil, castor oil and sesame oil may be used. More generally spoken, for parenteral administration the active agent can be in the form of an aqueous, lipid, oily or other kind of solution or suspension or even administered in the form of liposomes or nano-suspensions.

Transdermal application can be accomplished by suitable patches, as generally known in the art, specifically designed for the transdermal delivery of active agents, optionally in the presence of specific permeability enhancers. Furthermore, also emulsions, ointments, pastes, creams or gels may be used for transdermal delivery.

Another suitable mode of administration is via intravaginal devices (e. g. vaginal rings) or intrauterine systems (IUS) containing reservoirs for controlled release of active agents over extended periods of time. For rectal or vaginal administration of the drug the compounds may also be administered in the form of suppositories. These compositions can be prepared by mixing the drug with a suitable non-irritating excipient which is solid at ordinary temperatures but liquid at the rectal or vaginal temperature and will therefore melt in the rectum or vagina to release the drug.

Another mode of application is by implantation of a depot implant comprising an inert carrier material, such as biologically degradable polymers or synthetic silicones such as e. g. silicone rubber. Such implants are designed to release the active agent in a controlled manner over an extended period of time (e.g., 3 to 5 years).

It will be appreciated by those skilled in the art that the particular method of administration will depend on a variety of factors, all of which are considered routinely when administering therapeutics.

The actually required dosages of the agents of this invention for any given patient will depend upon a variety of factors, including, but not limited to the activity of the specific compound employed, the particular HSD type 1, type 2 or type 3 related condition being treated, the particular composition formulated, the mode of administration, time and duration of administration, route of administration and the particular site being treated, and furthermore the age of the patient, the body weight of the patient, the general health of the patient, the gender of the patient, the diet of the patient, rate of excretion, drug combinations, and the severity of the condition undergoing therapy.

It will be further appreciated by one skilled in the art that the optimal course of treatment, i.e., the mode of treatment and the daily number of doses of a compound of Formula I or a pharmaceutically acceptable salt thereof given for a defined number of days, can be ascertained by those skilled in the art using conventional treatment tests. Optimal dosages for a given set of conditions may be ascertained by those skilled in the art using conventional dosage-determination tests in view of the experimental data for a given compound. For oral administration, an exemplary daily dose generally employed will be from about 0.01 μg/kg to about 100 mg/kg of total body weight, whereby courses of treatment may be repeated at appropriate time intervals. Administration of pro-drugs may be dosed at weight levels that are chemically equivalent to the weight levels of the fully active compounds. The daily dosage for parenteral administration will generally be from about 0.01 μg/kg to about 100 mg/kg of total body weight. A daily rectal dosage regimen will generally be from about 0.01 μg/kg to about 200 mg/kg of total body weight A daily vaginal dosage regimen will generally be from about 0.01 μg/kg to about 100 mg/kg of total body weight. The daily topical dosage regimen will generally be from about 0.1 μg to about 100 mg administered between one to four times daily. The transdermal concentration will generally be that required to maintain a daily dose of from 0.01 μg/kg to 100 mg/kg of total body weight.

**Abbreviations and Acronyms**

As employed herein, the following terms have the indicated meanings.

| | |
|---|---|
| 20βP | 20β-hydroxyprogesterone |
| A | 4-androstene-3,17-one |
| Ac | Acetyl |
| AcOH | acetic acid |
| HSD | hydroxysteroid dehydrogenase |
| DHT | dehydrotestosterone |
| DMF | N,N-dimethylformamide |
| E1 | estron |
| E2 | estradiol |
| ER | estrogen receptor |
| EtOAc | ethyl acetate |
| GnRH | Gonadotropin Releasing Hormone |
| GRAS | generally recognized as safe |
| MS | mass spectrometry |
| NAD(P)[H] | nicotinamide-adenine-dinucleotide (phosphate) [reduced NAD(P)] |
| NMR | nuclear magnetic resonance |
| P | progesterone |
| PCC | pyridinium chlorochromate |
| T | testosterone |
| TBAB | Tetrabutylammonium Bromide |
| THF | tetrahydrofuran |
| TOF | 'Time-of-flight' |

**General Preparative Methods**

The compounds of the present invention may be prepared by use of known chemical reactions and procedures. Nevertheless, the following general preparative methods are presented to aid the reader in synthesizing the 17β-hydroxysteroid dehydrogenase inhibitors with specific details provided below in the experimental section to illustrate working examples.

All variable groups of these methods are as described in the generic description if they are not specifically defined below.

It is recognized that compounds of the invention with each claimed optional functional group may not be prepared by each of the below-listed methods. Within the scope of each method, optional substituents may appear on reagents or intermediates which may act as protecting or otherwise non-participating groups. Utilizing methods well known to those skilled in the art, these groups are introduced and/or removed during the course of the synthetic schemes which provide the compounds of the present invention.

**General Flow Diagrams**

The compounds according to this invention can be prepared as shown in Schemes 1 to 4.

*Scheme 1. General route to thienopyrimidinones.*

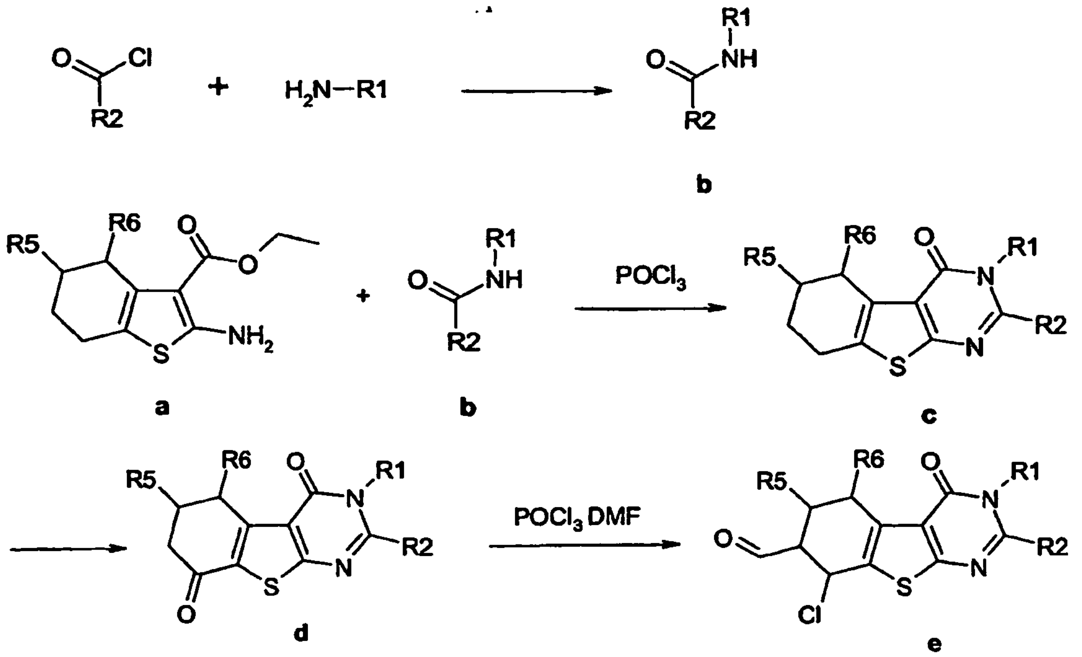


b

a b c

d e

*Scheme 1 [continued]*

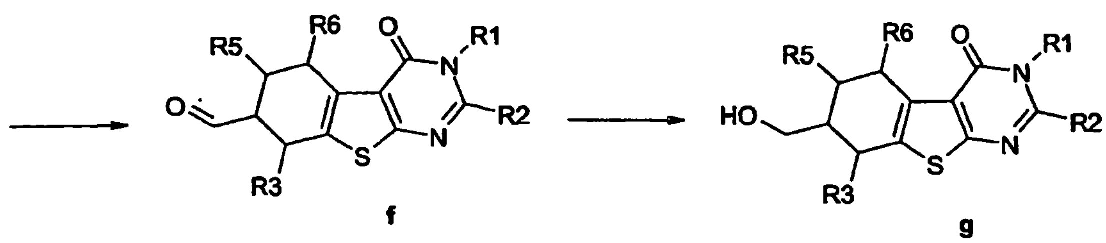

f g

Synthesis of thienopyrimidinones is presented in Scheme 1. Thienopyrimidinones of the formula **c** can be synthesized starting from derivated ethyl 2-amino-4,5,6,7-tetrahydrobenzo[b]thiophene-3-carboxylate **a** in a reaction with suitable amides **b** in the presence of phosphorous oxychloride to give compound of the formula **c.** (Ref. Kapustina, M.V., Kharizomenova, I.A, Shvedov, V.I., Chem. Heterocycl. Compd. (Engl. Trans.) 1991, 425.). The appropriate *N*-substituted amides **b** can be prepared by variety of synthetic methods. The treatment of acyl halides with primary amines is a general procedure for the preparation of amides. Oxidation of compound **c** was performed by using oxidant like PCC (pyridinium chlorochromate) affording 4,8-dione of the formula **d.** Carbonyl compound **d** was formylated in the Vilsmeier reaction using phosphorous oxychloride in DMF affording chloroaldehydes of the formula **e** (Ref. Koeller, S., Lellouche, J.-P., Tetrahedron Lett. 1999, 40, 7043. and Kapustina, M.V, Nikolaeva, I.S., Kharizomenova, I.A, Shvedov, V.I., Pushkina, T.V., Fomina, A. N., Pharm. Chem. J. 1992, 789.) Chloroaldehyde **e** was treated with alkyl and aryl alcohols or thiols in the presence of base to form compound of the formula **f.**

Aldehydes can be reduced to primary alcohols, *i.e.* compound of the formula **g,** by a number of reducing agents (*e.g.* $LiAlH_4$ and $NaBH_4$).

*Scheme 2. Aromatization*

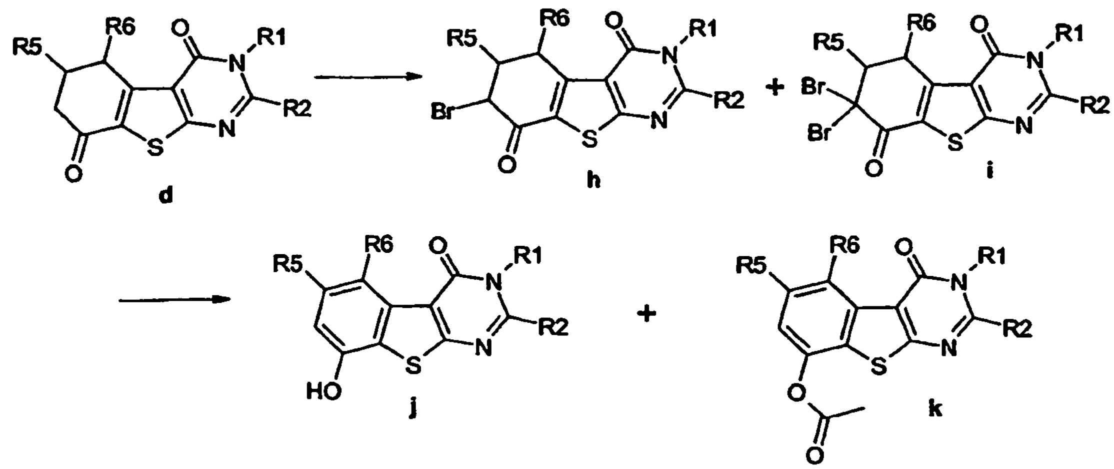

d h i j k

According to the reaction route of the *Scheme* 2 aromatic compounds of the formula **j** and **k** can be prepared by dehydrobromination of bromide derivatives of the formula **h** and **i**. The bromination of carbonyl compound of the formula **d** by using bromine and a catalyst *e.g.* benzoylperoxide afforded several different bromides which were isolated and identified. Generally 3,4,5-trimethoxyphenyl group in the R2-position was monobrominated as well as α-bromo- and α,α-dibromo carbonyl derivatives were produced.

The aromatization was achieved by the use of microwaves. In microwave dielectric heating the temperature increase is uniform throughout the sample (Ref. Lidstroem, P. et al. Tetrahedron 2001, 57, 9225). In addition, the temperature increase considerably above the conventional boiling point of the solvent used is rapidly achieved. Microwave chemistry is generally suitable for various chemical reactions having several benefits like decrease of the reaction time, increase of yield and purity. A bromide of the formula **h** or i in the presence of sodium acetate in acetic add was heated by the use of microwaves at 180°C. Both the phenol of the formula **j** as well as the acetylated compound of the formula **k** could be obtained.

*Scheme 3. Demethylation and acetylation*

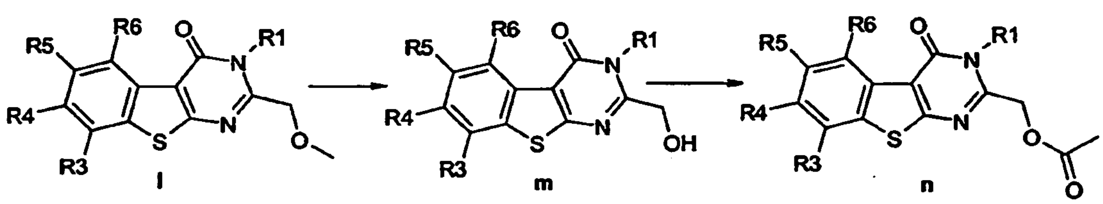

l m n

The cleavage of ethers can be achieved by numerous reagents *e.g.* different acidic re-agents or Lewis acids. Methoxymethyl derivative I was easily demethylated by using boron tribromide according to the Scheme 3. Alcohol m was acetylated to compound n by using a general procedure (AcOH, pyridine).

*Scheme 4.*

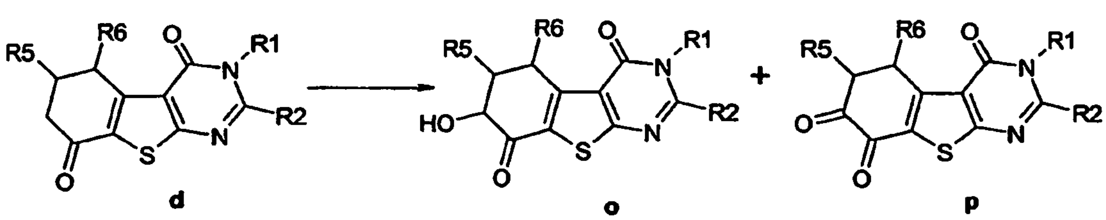

d o p

A number of methods are available to prepare α-hydroxy carbonyls and α-diketones. Carbonyl compounds of the formula d can be α-hydroxylated or α-carbonylated according to the reaction route of Scheme 4. Alternative route is, for example, the alkaline hydrolysis of α-bromo carbonyl compound affords α-hydroxy ketones of the formula **o** and α-diketones of the formula **p**. Further compounds of general formula **q** falling under the scope of general formula I can prepared by parallel chemistry using a reaction as shown in the following scheme 5 (according to the first step of general flow scheme 1), thereby using different separately synthesized starting materials of formula **I**:

***Scheme 5: General route to substituted Benzothienopyrimidinones.***

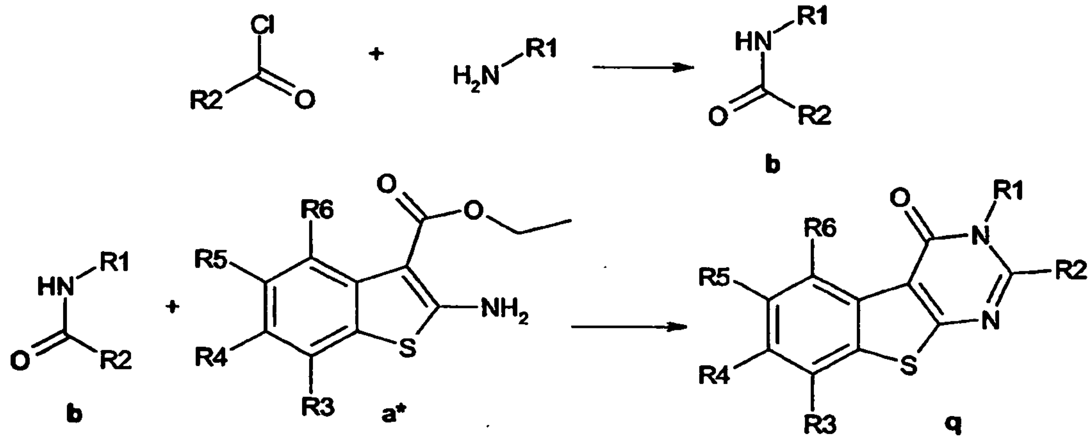

b

b a* q

Synthesis of substituted benzothienopyrimidinones is presented in Scheme 5. Substituted Benzothienopyrimidinones of the general formula **q** can be synthesized starting from a substituted 2-amino-benzo[b]thiophene-3-carboxylic add ethyl ester **a*** in a reaction with suitable amides **b** in the presence of phosphorous oxychloride to give compound of the formula **q.** (Ref. Kapustina, M.V., Kharizomenova, I.A., Shvedov, V.I., Chem. Heterocycl. Compd. (Engl. Trans.) 1991, 425.). The appropriate *N*-substituted amides **b** can be prepared by variety of synthetic methods. The treatment of acyl halides with primary amines is a general procedure for the preparation of amides.

The invention will be illuminated by the following non-restrictive Experimental Section.

EXPERIMENTAL SECTION

**Reference example: Preparation of *N*-benzyl-3,4,5-trimethoxybenzamide**

3,4,5-Trimethoxybenzoyl chloride (5.0 g, 21.7 mmol) was dissolved in dichloromethane (50 ml). The reaction mixture was cooled with an ice-bath and benzylamine (4.74 ml, 43.4 mmol) was added slowly. The solid material was removed by filteration. The filtrate was poured into 30 ml of water. The organic phase was washed several times with water. The crude product was recrystallized from *i*-propanol.

**Examples**

In order to more fully illustrate the nature of the invention and the manner of practicing the same, the following examples are presented.

The following compounds No. 1 to No. 17 falling under the scope of general formula (I) were prepared:

**Compound No.1:** 3-Benzyl-methyl-2-(3,4,5-trimethoxyphenyl)-5,6,7,8-tetrahydro-3H-benzo[4,5]thieno[2,3-d]pyrimidin-4-one

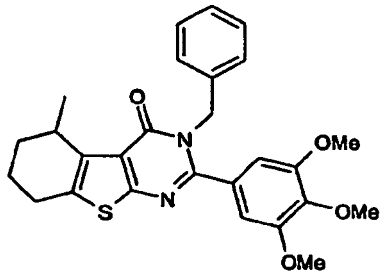

2-Amino-4-methyl-4,5,6,7-tetrahydro-benzo[b]-thiophene-3-carboxylic acid ethyl ester (26.6 mmol, 100 mol-%) and *N*-benzyl-3,4,5-trimethoxy-benzamide (34.6 mmol, 130 mol-%) were dissolved in dry 1,2-dichloroethane. The reaction mixture was cooled with an ice-salt-bath and $POCl_3$ (1.7 ml, 24.6 mmol, 130 mol-%) was added. The reaction mixture was refluxed for 24 hours. During refluxing $POCl_3$ (340 μl) was added twice. The reaction mixture was poured into ice-water and after neutralization with sodium acetate the product was extracted into dichloromethane. The organic phases combined were washed with sodium bicarbonate sat. (3 x 50 ml) and dried with $MgSO_4$.

The compound 2-amino-4-methyl-4,5,6,7-tetrahydro-benzo[b]thiophene-3-carboxylic acid ethyl ester was prepared starting from 2-methylcyclohexanone, cyanoacetic acid ethyl ester and sulphur using morpholino as a base (Gütschow M., et al. J. Med. Chem. 1999, 42, 5437).

Compound No.1:

NMR ($CDCl_3$): 1.35 (d, 3H), 1.78 (m, 1H), 1.91 (m, 4H), 2.79 (m, 2H), 3.59 (s, 6H), 3.92 (s, 3H), 5.24 (d, 2H), 6.48 (s, 2H), 7.01 (m, 2H), 7.30 (m, 3H).
MS (TOF, ES+) m/z 477 (M + 1)

**Compound No. 2:** 3-Benzyl-5-methyl-2-(3,4,5-trimethoxyphenyl)-6,7-dihydro-3H,5H-benzo[4,5]thieno[2,3-d]pyrimidin-4,8-dione

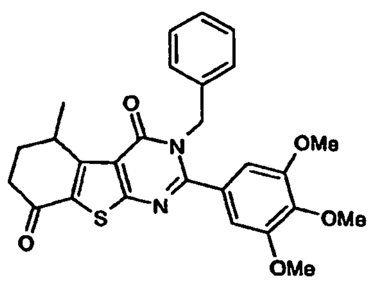

3-Benzyl-5-methyl-2-(3,4,5-trimethoxyphenyl)-5,6,7,8-tetrahydro-3H-benzo[4,5]thieno[2,3-d]pyrimidin-4-one (Compound No.1) (17.7 mmol) dissolved in dry dichloromethane (30 ml) was added quickly to a mixture of PCC (19.2 g, 89.0 mmol, 500 mol-%) in dichloromethane (200 ml). During refluxing PCC was added several times until the reaction was completed. The reaction mixture was filtered through Celite with dichloromethane. The crude product was purified by flash chromatography.

NMR ($CDCl_3$): 1.46 (d, 3H), 2.08 (m, 1H), 2.50 (m, 2H), 2.67 (m, 1H), 2.91 (m, 1H), 3.59 (s, 6H), 3.86 (s, 3H), 5.27 (d, 2H), 6.53 (s, 2H), 7.05 (m, 2H), 7.28 (m, 3H).
MS (TOF, ES+) m/z 491 (M + 1)

**Compound No. 3:** 3-Benzyl-7-bromo-2-(2-bromo-3,4,5-trimethoxyphenyl)-5-methyl-6,7-dihydro-3H,5H-benzo[4.5] thieno[2,3-d]pyrimidine-4,8-dione

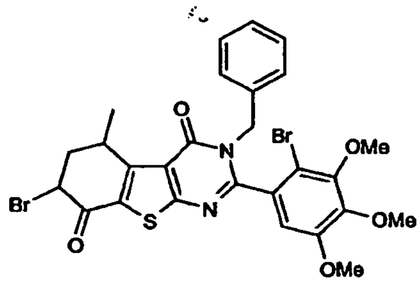

3-Benzyl-5-methyl-2-(3,4,5-trimethoxyphenyl)-6,7-dihydro-3H,5H-benzo[4,5]thieno[2,3-d]pyrimidin-4,8-dione (compound No. 2) (0.3 g, 100 mol-%) was diluted in methanol (20ml). Bromine (31 μl, 100 mol-%) was added dropwise. The reaction mixture was heated at 50°C for overnight. The reaction was cooled, and the solid material was filtered. The product was purified by chromatography using dichloromethane as an eluent.

NMR ($CDCl_3$): 1.60 (m, 1H), 1.84 (d, 3H), 2.62 (m, 1H), 3.01 (m, 1H), 3.46 (s, 3H), 3.92 (s, 3H), 3.99 (s, 3H), 4.46 (d, 1H), 4.78 (t, 1H), 5.91 (d, 1H), 6.15 (d, 1H), 6.87 (m, 2H), 7.21 (m, 3H).
MS (TOF, ES+) m/z 647/649/651

**Compound No. 4:** 3-Benzyl-2-(2-bromo-3,4,5-trimethoxyphenyl)-8-hydroxy-5-methyl-3H-benzo[4,5]thieno[2,3-d]pyrimidin-4-one

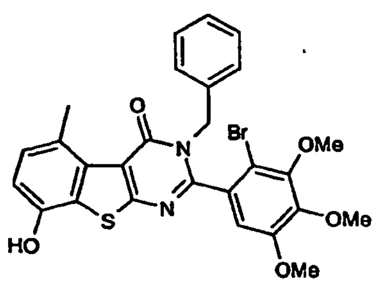

3-Benzyl-7-bromo-2-(2-bromo-3,4,5-trimethoxyphenyl)-5-methyl-6,7-dihydro-3H,5H-benzo[4,5]thieno[2,3-d] pyrimidine-4,8-dione (compound No. 3) (100 mg, 100 mol-%) and NaOH (20 mg, 400 mol-%) in 1.5 mi of ethanol was heated by using microwaves (100°C, 100 s). The solvent was evaporated. EtOAc was added and the reaction mixture was washed with 5% HCl-solution. The product was purified by chromatography using dichloromethane-EtOAc 9.5:0.5 as an eluent.

NMR ($CDCl_3$): 3.04 (s, 3H), 3.43 (s, 3H), 3.91 (s, 3H), 3.95 (s, 3H), 4.49 (d, 1H), 6.00 (dd, 2H), 6.19 (s, 1H), 6.76 (d, 1H), 6.90 (m, 2H), 7.13 (M, 3H).
MS (TOF, ES+) m/z 567/569

**Compound No. 5:** 3-Benzyl-6-methyl-2,3,4,5-trimethoxyphenyl)-5,6,7,8-tetrahydro-3H-benzo[4,5]thieno[2,3-d]pyrimidin-4-one

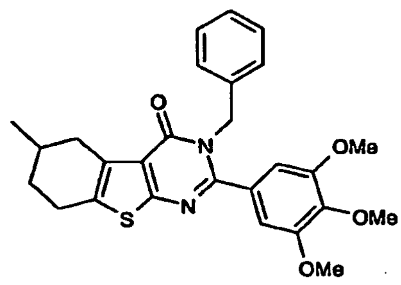

The compound No. 5 was prepared according to the method described for the compound No. 1 using *N*-benzyl-3,4,5-trimethoxy-benzamide and 2-amino-5-methyl-4,5,6,7-tetrahydro-benzo[b]-thiophene-3-carboxylic acid ethyl ester as starting materials. The starting material 2-amino-5-methyl-4,5,6,7-tetrahydro-benzo[b]thiophene-3-carboxylic acid ethyl ester was prepared starting from 3-methylcyclohexanone (Gütschow M., et al. J. Med. Chem. 1999, 42, 5437).

NMR ($CDCl_3$): 1.12 (d, 3H), 1.60 (m, 3H), 1.98 (m, 2H), 2.86 (m, 2H), 3.59 (s, 6H), 3.88 (s, 3H), 5.22 (d, 2H), 6.47 (s, 2H), 7.04 (m, 2H), 7.30 (m, 3H).
MS (TOF, ES+) m/z 477 (M + 1)

**Compound No. 6:** 3-Benzyl-6-methyl-2-(3,4,5-trimethoxyphenyl)-6,7-dihydro-3H,5H-benzo[4.5]thieno[2,3-d]pyrimidin-4,8-dione

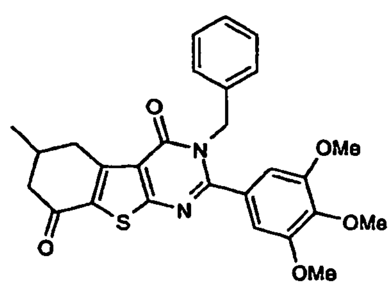

Compound No. 6 was prepared according to the method described for the compound No. 2 using the compound No. 5 as a starting material.
NMR ($COCl_3$): 1.21 (d, 3H), 1.57 (m, 1H), 2.50 (m, 2H), 2.70 (m, 2H), 3.59 (s, 6H), 3.85 (s, 3H), 5.25 (d, 2H), 6.52 (s, 2H), 7.05 (m, 2H), 7.30 (m, 3H).
MS (TOF, ES+) m/z 491 (M + 1)

**Compound No. 7:** 3-Benzyl-7-bromo-6-methyl-2-(2-bromo-3,4,5-trimethoxyphenyl)-6,7-dihydro-3H-benzo[4,5]thieno [2,3-d]pyrimidin-4,8-dione

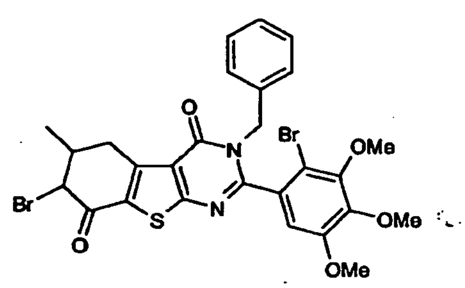

The compound No. 6 (4. mmol, 100 mol-%) and benzoylperoxide (99 mg, 0.4 mmol, 10 mmol%) were dissolved

in dichloromethane (40 ml). While the reaction mixture was refluxing bromine (440 µl, 8.4 mmol, 200 mol-%) in dichloromethane (16 ml) was added. The reaction was completed in 3.5 hours. The cooled reaction mixture was washed with water (40 ml). The organic phase was evaporated. The crude product was purified by chromatography using dichloromethane-EtOAc as an eluent.

NMR ($CDCl_3$): 1.31 (d, 3H), 2.44 (m, 1H), 2.95 (m, 1H), 3.42 (s, 3H), 3.64 (m, 1H), 3.92 (s, 3H), 3.96 (s, 3H), 4.43 (dd, 1H), 4.51 (t, 1H), 5.90 (dd, 1H), 6.16 (d, 1H), 6.89 (s, 2H), 7.27 (m, 3H).
MS (TOF, ES+) m/z 647/649/651

**Compound No. 8:** 3-Benzyl-2-(2-bromo-3,4,5-trimethoxyphenyl)-8-hydroxy-6-methyl-3H-benzo[4,5]thieno[2,3-d]pyrimidin-4-one

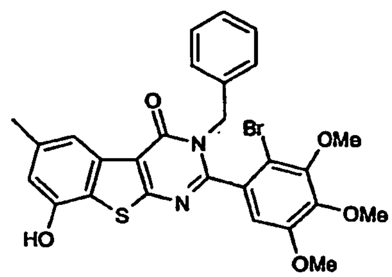

The compound No.7 (0.08 mmol) was dissolved in methanol (1.5 ml). NaOH (13 mg, 0.32 mmol) was added. The reaction mixture was heated using microwaves (120°C, 2 min.). The solvent was evaporated and the precipitate was dissolved into ethylacetate, washed with 1 N HCl and water. Purified by using chromatography (eluent: $CH_2Cl_2$-diethyl ether 9:1).

NMR ($CDCl_3$): 2.45 (s, 3H), 3.45 (s, 3H), 3.91 (s, 3H), 3.95 (s, 3H), 4.53 (d, 1H), 6.06 (d, 1H), 6.21 (s, 1H), 6.60 (s, 1H), 6.76 (s, 1H), 6.92 (m, 2H), 7.20 (m, 3H), 8.16 (s, 1H).
MS (TOF, ES+) m/z 567/569

**Compound No. 9:** 3-Butyl-6-methyl-2-(3,4,5-trimethoxyphenyl)-5,6,7,8-tetrahydro-3H-benzo[4,5]thieno[2,3-d]pyrimidin-4-one

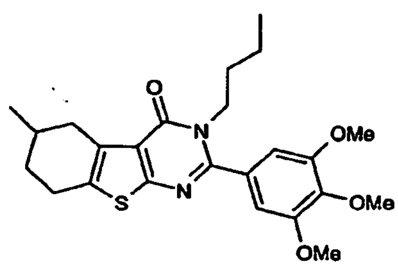

Compound No. 9 was prepared according to the method described for the compound No. 1 using *N*-butyl-3,4,5-trimethoxy-benzamide and 2-amino-5-methyl-4,5,6,7-tetrahydro-benzo[b]thiophene-3-carboxylic acid ethyl ester as starting materials.

NMR (CDCl3): 0.82 (t, 3H), 1.25 (m, 5H), 1.71 (m, 4H), 1.97 (m, 2H), 2.50 (m, 1H), 2.85 (m, 1H), 3.35 (m, 1H), 3.89 (s, 9H), 3.95 (m, 2H), 6.69 (s, 2H).
MS (TOF, ES+) m/z 443 (M + 1)

**Compound No. 10:** 3-Butyl-6-methyl-2-(3,4,5-trimethoxyphenyl)-6,7-dihydro-3H,5H-benzo[4,5]thieno[2,3-d]pyrimidin-4,8-dione

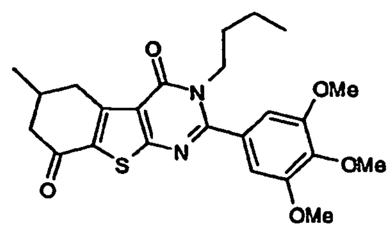

Compound No. 10 was prepared according to the method described for the compound No. 2 using the compound No. 9 as starting material.
NMR (CDCl3): 0.84 (t, 3H), 1.25 (m, 5H), 1.76 (m, 3H), 2.50 (m, 4H), 3.90 (s, 9H), 3.94 (m, 2H), 6.72 (s, 2H).
MS (TOF, ES+) m/z 457 (M + 1)

**Compound No. 11:** 3-Butyl-7-bromo-6-methyl-2-(2-bromo-3,4,5-trimethoxyphenyl)-6,7-dihydro-3H-benzo[4,5]thieno[2,3-d]pyrimidin-4,8-dione

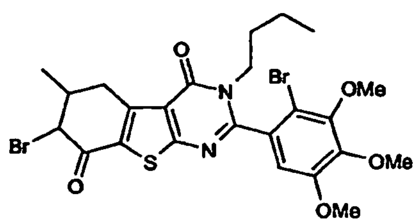

Compound No. 11 was prepared according to the method described for the compound No. 7 using the compound No. 10 as starting material. The compound 15 was isolated as a by-product.

$R_f$ (toluene-methanol 9:1) 0.55
NMR ($CDCl_3$): 0.77 (t, 3H), 1.40 (m, 7H), 2.90 (m, 1H), 3.53 (m, 2H), 3.90 (s, 9H), 3.94 (m, 2H), 4.48 (t, 1H), 6.76 (s, 1H).
MS (TOF, ES+) m/z 613/615/617

**Compound No.12** 3-Butyl-2-(2-bromo-3,4,5-trimethoxyphenyl)-8-hydroxy-6-methyl-3H-benzo[4,5]thieno[2,3-d]pyrimidin-4-one

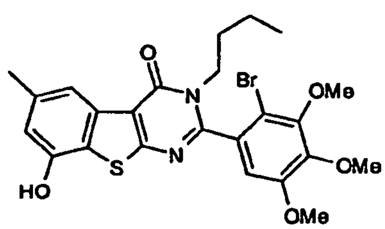

Compound No. 12 was prepared according to the method described for the compound No. 8 using the compound No. 11 as a starting material.

$R_f$ (toluene-methanol 9:1) 0.26
NMR ($CDCl_3$): 0.76 (t, 3H), 125 (m, 2H), 1.60 (m, 2H), 2.49 (s, 3H), 3.60 (m, 1H), 3.90 (s, 3H), 3.95 (s, 6H), 4.34 (m,

1H), 6.76 (s, 1H), 6.85 (s, 1H), 8.14 (s, 1H).
MS (TOF, ES+) m/z 533/535

**Compound No. 13:** 2-(2-Bromo-3-hydroxy-4,5-dimethoxyphenyl)-3-butyl-8-hydroxy-6-methyl-3H-benzo[4,5]thieno [2,3-d]pyrimidin-4-one

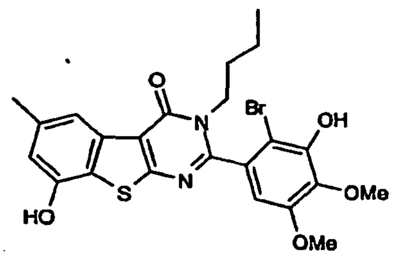

3-Butyl-2-(2-bromo-3,4,5-trimethoxyphenyl)-8-hydroxy-6-methyl-3H-benzo[4,5]thieno[2,3-d]pyrimidin-4-one (compound No. 12) (40 mg) was stirred with HBr in acetic acid (1 ml) for five days. Saturated sodium sulphate solution was added and the product was extracted into ethyl acetate. After washing with water and brine, the solvent was evaporated and the precipitate was purified by chromatography using dichloromethane-methanol 99:1 as an eluent. Compound No. 14 was isolated as a by product.

Compound No. 13:

NMR ($CDCl_3$): 0.85 (t, 3H), 1.25 (m, 2H), 1.62 (m, 2H), 2.58 (s, 3H), 3.63 (m, 1H), 3.92 (s, 3H), 3.97 (s, 3H), 4.28 (m, 1H), 6.01 (br s, 1H), 6.12 (s, 1H), 6.66 (s, 1H), 8.20 (s, 1 H).
MS (TOF, ES+) m/z 519/521

**Compound No. 14:** 2-(2-Bromo-3,4-dihydroxy-5-methoxyphenyl)-3-butyl-8-hydroxy-6-methyl-3H-benzo[4,5]thieno [2,3-d]pyrimidin-4-one.

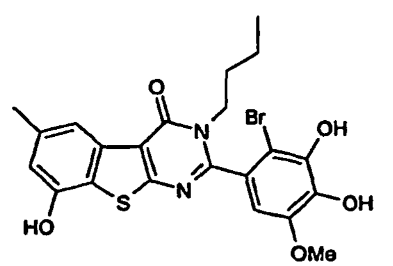

Compound No. 14 was isolated as a by-product in the demethylation of 3-Butyl-2-(2-bromo-3,4,5-trimemoxyphenyl)-8-hydroxy-6-methyl-3H-benzo[4,5]thieno[2,3-d]pyrimidin-4-one (Compound No. 12).
NMR (MeOH-d4): 0.62 (t, 3H), 1.10 (m, 2H), 1.50 (m, 2H), 2.29 (s, 3H), 3.71 (s, 3H), 3.90-4.35 (m, 2H), 6.41 (s, 1H), 6.66 (s, 1H), 7.8 (s, 1H).
MS (TOF, ES+) m/z 505/507

**Compound No. 15:** 7,7-Dibromo-3-butyl-6-methyl-2-(2-bromo-3,4,5-trimethoxyphenyl)-6,7-dihydro-3H-benzo[4,5] thieno[2,3-d]pyrimidine-4,8-dione

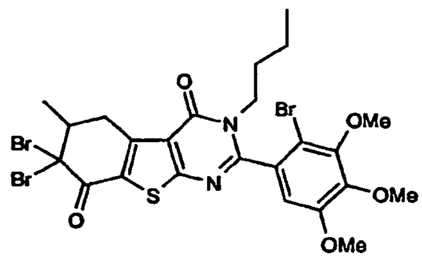

The compound 15 was isolated as a by-product in the bromination of the compound 10.

$R_f$ (toluene-methanol 9:1) 0.68
MS (TOF, ES+) m/z 691/693/695/697

**Compound No. 16:** 7-Bromo-3-butyl-2-(2-bromo-3,4,5-trimethoxyphenyl)-8-hydroxy-6-methyl-3H-benzo[4,5]thieno [2,3-d]pyrimidin-4-one

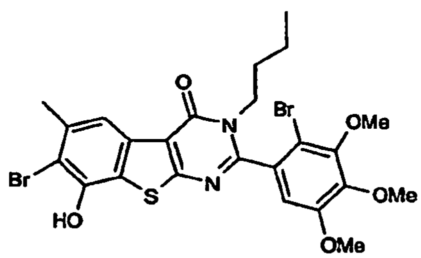

Compound No. 16 was prepared according to the method described for the compound No. 8, using the compound No. 15 as a starting material. The compound 16 was used directly in demethylation for the preparation of the compound 17.

$R_f$ (toluene-methanol 9:1) 0.39
MS (TOF, ES+) m/z 611/613/615

**Compound No. 17:** 7-Bromo-2-(2-bromo-3-hydroxy-4,5-dimethoxyphenyl)-3-butyl-8-hydroxy-6-methyl-3H-benzo[4,5] thieno[2.3-d]pyrimidin-4-one

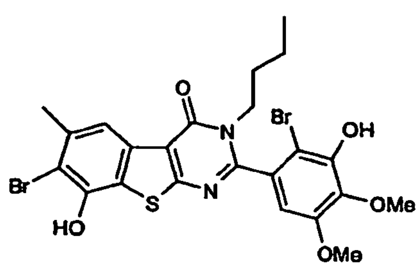

Compound No. 17 was prepared by demethylation of 7-bromo-3-butyl-2-(2-bromo-3,4,5-trimethoxyphenyl)-8-hydroxy-6-methyl-3H-benzo[4,5]thieno[2,3-d]pyrimidin-4-one (compound No. 16) according to the procedure described for the compound No. 13.

NMR ($CDCl_3$): 0.81 (t, 3H), 1.20 (m, 2H), 1.70 (m, 2H), 2.58 (s, 3H), 3.64 (m, 1H), 3.94 (d, 6H), 4.28 (m, 1H), 6.84 (s, 1H), 8.20 (s, 1H).
MS (TOF, ES+) m/z 597/599/601

Further compounds of general formula (I) can prepared by parallel chemistry using a reaction as shown in the following scheme 6:

***Scheme 6: General route to substituted Benzothienopyrimidinones***

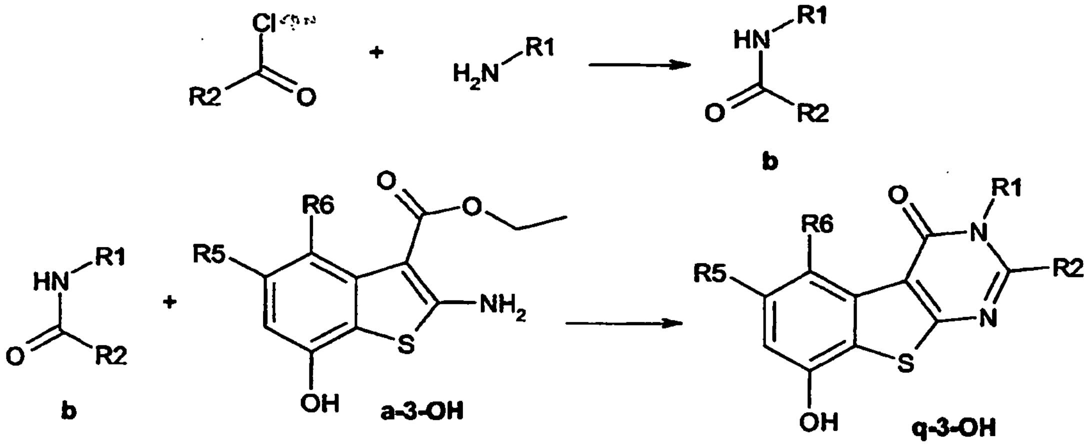

b

b a-3-OH q-3-OH

In a reaction vessel at room temperature are put together sequentially 0.25 M primary amine R1-$NH_2$, 1 M diisopropylethylamine and 0.25 M acid chloride R2-CO-Cl. To this mixture is added 0.25 M substituted and optionally protected 2-amino-benzo[b]thiophene-3-carboxylic acid ethyl ester **a-3-OH** followed by 0.25 M $POCl_3$. Of all reactants one equivalent is used as solution or suspension in chlorobenzene. After shaking for 80 hours at 100°C, the mixtures are cooled to room temperature, washed with 5% NaOAc and extracted with EtOAc. The organic layers are collected and concentrated to yield the desired compound. The obtained material of the formula **q-3-OH** was thereafter analyzed by LC-MS.

The LC-MS system consists of 2 Perkin Elmer series 200 micro-pumps. The pumps are connected to each other by a 50 μ tee mixer. The mixer is connected to the Gilson 215 autosampler. The LC methode consists of the following steps:

| Step | total time | flow (μl/min) | A(%) | B(%) |
|---|---|---|---|---|
| 0 | 0 | 2300 | 95 | 5 |
| 1 | 1.8 | 2300 | 0 | 100 |
| 2 | 2.5 | 2300 | 0 | 100 |
| 3 | 2.7 | 2300 | 95 | 5 |
| 4 | 3.0 | 2300 | 95 | 5 |

Solution A= 100% Water with 0.025% HCOOH and 10mmol $NH_4HCOO$ pH= +/- 3

Solution B= 100% MeOH with 0.025% HCOOH

The auto sampler has a 2 μl injection loop. The auto sampler is connected to a Varian Polaris C18 A 30*4.6 mm column with 3 μm particles. The column is thermo stated in a Perkin Elmer series 200 column oven at 40°C. The column is connected to an Applied Biosystems ABI 785 W meter with a 2.7 μl flowcel. The wavelength is set to 254 nm. The UVmeter is connected to a Sciex API 150EX mass spectrometer having the following parameters (Scanrange: 150-900 Amu, Polarity: positive, Scan mode: profile, Resolution Q1: UNIT, Step size: 0.10 amu, Time per scan: 0.500 sec, NEB: 10, CUR: 10, IS: 5200, TEM: 325, DF: 30, FP: 225, EP: 10). The light scattering detector is connected to the Sciex API 150. The light scattering detector is a Sedere Sedex 55 operating at 50°C and 3 bar $N_2$ pressure. The complete systems is controlled by a Dell optiplex GX400 computer operating under Windows NT.

<u>Substituted 2-amino-benzo[b]thiophene-3-carboxylic acid ethyl esters **a-3-OH**</u>

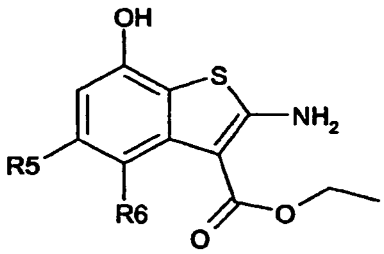

**a-3-OH**

Several substituted 2-amino-benzo[b]thiophene-3-carboxylic acid ethyl esters of the general formula **a-3-OH** which can be used in the synthesis scheme 6 are displayed in the following Scheme 7.

***SCHEME 7: Exemplary compounds of general formula a-3OH, wherein R represents e.g. hydrogen or a $C_1$-$C_4$-alkyl residue***

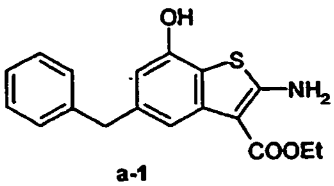

**a-1**

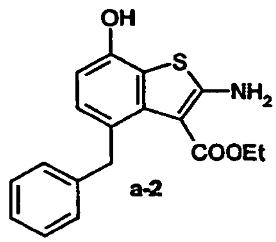

**a-2**

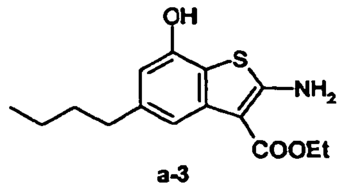

**a-3**

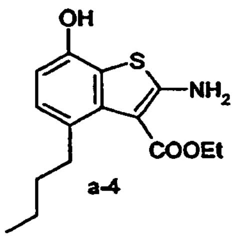

**a-4**

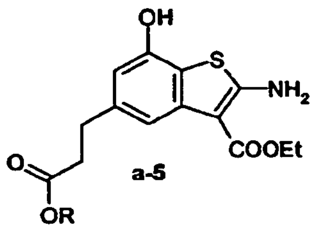

**a-5**

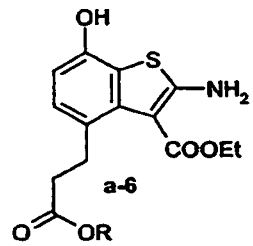

**a-6**

<u>Synthesis of 2-Amino-5-benzyl-7-hydroxy-benzo[b]thiophene-3-carboxylic acid ethyl ester **a-1**</u>

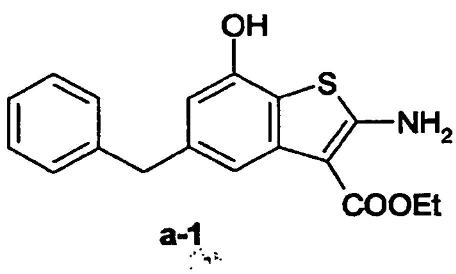

**a-1**

***SCHEME 8: Synthesis of compound a-1***

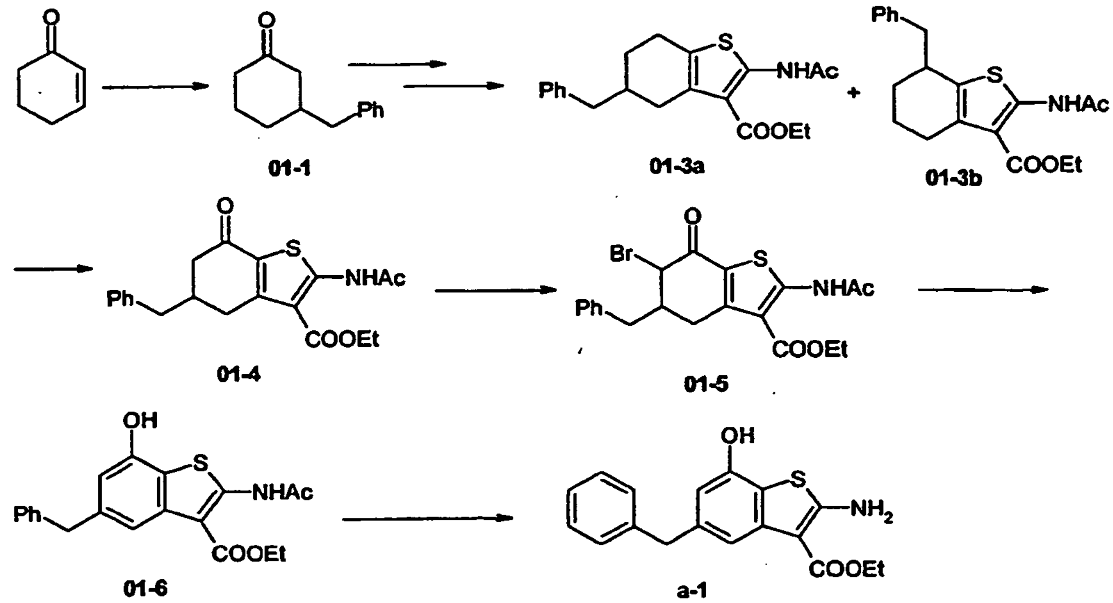

01-1 01-3a 01-3b 01-4 01-5 01-6 a-1

The synthesis of **a-1** (Scheme 1) started from 3-benzylcyclohexanone **(01-1)**, which was prepared according to literature [Mitra & Joshi (1988) Synth. Comm. 18:2259]. Compound **01-1** was converted to thiophene **01-3** over two steps using the literature procedure [Perrissin et al. (1980) Eur. J. Med. Chem. Chim. Ther. 15:413-418]. It was found that a mixture of two possible regio-isomers **01-3a** and **01-3b** in a ratio of 1:10 (according to NMR) was formed. Because it was not possible to separate the isomers by column chromatography, the crude mixture was treated with $Na_2Cr_2O_7$. It turned out that only the minor isomer, **01-3a**, was converted to ketone **01-4** in 12% yield from **01-1**. Monobromination of **01-4** with $CuBr_2$ in refluxing EtOAc gave **01-5** in 46% after crystallization [Tani et al. (1996) Chem. Pharm. Bull. 44: 55-61]. The compound was isolated as mixture of the *cis*- and *trans*-isomers in a ratio of 1:23. One major side-product (~10%) was identified to be the O-ethylated elimination product. Final elimination was achieved according to Tani et al. with UBr/$Li_2CO_3$ and gave **01-6** in 77% yield [Tani et al. (1996) Chem. Pharm. Bull. 44:55-61]. Conversion of **01-6** with $H_2SO_4$ in MeOH gave compound **a-1** in 91%.

<u>Synthesis of 2-Amino-5-butyl-7-hydroxy-benzo[b]thiophene-3-carboxylic acid ethyl ester **a-3**</u>

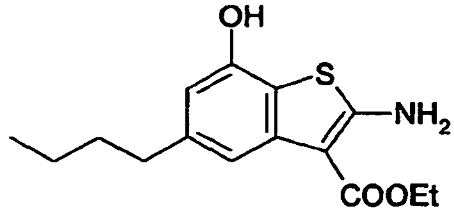

**a-3**

***SCHEME 9: Synthesis of compound a-3***

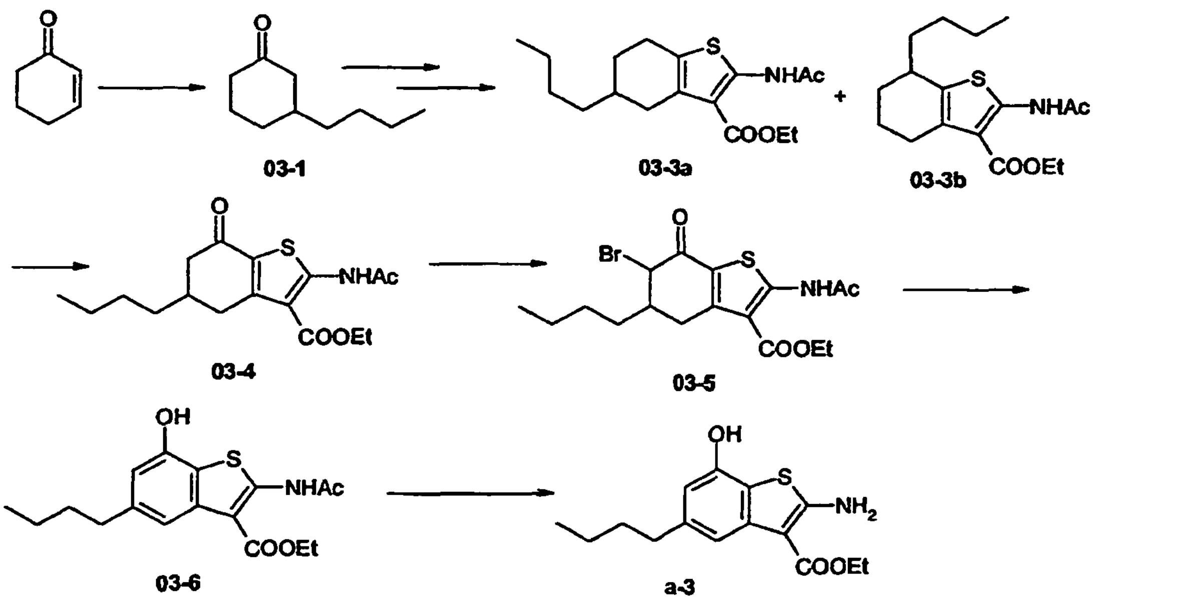

**03-1** **03-3a** **03-3b** **03-4** **03-5** **03-6** **a-3**

For the synthesis of **a-3** the same strategy as for **a-1** (Scheme 8) was used. 3-Butyl cyclohexanone **03-1** was prepared in 78% yield [Lipshutz et al. (1984) J. Org. Chem. 49:3938-3942]. The following thiophene formation and acylation gave a mixture of isomers **03-3a** and **03-3b** in a ratio 1:3. After oxidation with $Na_2Cr_2O_7$ ketone **03-4** was obtained in 18% yield from **03-1**. Bromination with $CuBr_2$ in refluxing EtOAc gave bromide 5 in 61% yield. Elimination with $LiBr/Li_2CO_3$ afforded **03-6** in 68%. Conversion of **03-6** with $H_2SO_4$ in MeOH/MeCN gave compound **a-3** in 82%.

**Detailed Synthesis:**

*3-Benzyl-cyclohexanone* **(01-1**)

To a solution of benzylmagnesiumchloride in THF (1.31 M, 393 mmol, 300 mL) CuCl (1.9 g) was added and the mixture was cooled to 0˚C. The mixture was stirred for 6 min., and a solution of cyclohex-2-enone (193 mmol, 18.9 g) in THF (75 mL) was added dropwise at 0˚C over a period of 1 h. The reaction mixture was warmed to RT overnight. After cooling again to 0˚C, saturated aqueous $NH_4Cl$ (450 mL) was added and the layers were separated. The aqueous layer was extracted with EtOAc (2 x 500 mL). The combined org. layers were washed with saturated aqueous $NH_4Cl$ (500 mL) and dried over $Na_2SO_4$. After evaporation, the crude product was subjected to column chromatography ($SiO_2$, EtOAc/Hept = 1/19 → 1/9) to give two fractions of product **01-1.** Fraction 1 (23.5 g, 125 mmol, 65%, yellow oil) was used in the next step.

*3-Butyl-cyclohexanone* **(03-1**)

A mixture of CuCN (49.3 mmol, 550 mmol, 1.1 equiv.) and $Et_2O$ (1 L) was cooled to -78˚C. A 2.6 M-solution of n-BuLi in hexane (440 mL, 1.1 mol, 2.2 equiv.) was added dropwise keeping the temperature below - 75 ˚C. After complete addition, the mixture was slowly warmed up. After reaching 0˚C, it was again cooled to -85˚C. Cyclohex-2-enone (48.1 g, 500 mmol) was added dropwise keeping the internal temperature around - 80˚C. After complete addition the mixture was stirred for 2 h at - 85˚C. Then the reaction was quenched by adding a mixture of saturated aqueous $NH_4Cl$ (750 mL) and concentrated $NH_4OH$ (750 mL). A grey precipitate was formed. The layers were separated, and the aqueous layer was extracted with TBME (2 x 250 mL). The combined org. layers were washed with saturated aqueous $NH_4Cl$ (3 x 500 mL) and dried over $Na_2SO_4$. After evaporation of the solvents *in vacuo* the crude product was filtered over $SiO_2$ using heptane as eluent. After evaporation of the solvent product **03-1** (602 g, 391 mmol, 78%) was obtained as a yellow oil.

*2-Amino-5-benzyl-4,5,6,7-tetrahydro-benzo[b]thiophene-3-carboxylic acid ethyl ester* **(01-2a)**

Compound **01-1** (26 g, 138 mmol) was mixed with EtOH (70 mL), $H_2O$ (1 mL), ethyl cyanoacetate (14.7 mL, 15.6 g, 138 mmol) and sulfur (4.42 g, 138 mmol). To the resulting mixture morpholine (14 mL, 13.9 g, 159 mmol) was added and the mixture was heated for 4 at 45°C. It was concentrated to half of the original volume and poured into $H_2O$ (500 mL). The aqueous layer was extracted with EtOAc (3 x 200 mL). The combined organic layers were washed with brine, dried over $Na_2SO_4$ and the solvent was removed *in vacuo* to give the crude product as a red brown oil, which was used in the next step without further purification.

*2-Amino-5-butyl-4,5,6,7-tetrahydro-benzo[b]thiophene-3-carboxylic acid ethyl ester* **(03-2a)**

Crude mixture **03-2** was prepared from **03-1** (26 g, 168 mmol), ethyl cyanoacetate (18 mL, 19.1 g, 168 mmol) and sulfur (5.4 g, 168 mmol) and morpholine (17 mL) using the procedure for **01-2** and was used in the next step without further purification.

*2-Acetylamino-5-benzyl-4,5,6,7-tetrahydro-benzo[b]thiophene-3-carboxylic acid ethyl ester* **(01-3a)**

The crude product mixture of isomers **01-2a** and **01-2b** was dissolved in AcOH (40 mL). Acetic anhydride (40 mL) was added and the mixture was heated at 35°C for 3 h. The mixture was cooled to RT and poured in $H_2O$ (250 mL). The aqueous layer was extracted with EtOAc (3 x 200 mL). The combined organic layers were washed with $H_2O$ (3 x 200 mL), brine (200 mL) and dried over $Na_2SO_4$. After removal of the solvent *in vacuo* the crude product was obtained as a red brown oil, which was used in the next step without further purification.

*2-Acetylamino-5-butyl-4,5,6,7-tetrahydro-benzo[b]thiphene-3-carboxylic acid ethyl ester* **(03-3a)**

Conversion of the crude mixture **03-2** according to the procedure for **01-3a** with $Ac_2O$ (55 mL) in AcOH (55 mL) gave crude mixture **03-3**, which was used in the next step without further purification.

*2-Acetylamino-5-benzyl-7-oxo-4,5,6,7-tetrahydro-benzo[b]thiophene--3-carboxylic acid ethyl ester* **(01-4)**

The crude product mixture of isomers **01-3a** and **01-3b** was dissolved in AcOH (450 mL). The mixture was heated to 50°C and a solution of $Na_2Cr_2O_7 \cdot 2H_2O$ (61.7 g, 207 mmol) in hot $H_2O$ (250 mL) was added dropwise. After complete addition the mixture was stirred for 1 h at 65° and then allowed to cool to RT within 5 h. The reaction mixture was quenched with saturated aqueous $Na_2SO_3$ (200 mL) and diluted with $H_2O$ (1.5 L). The resulting precipitate was filtered off and washed with $H_2O$. After drying, the solid was stirred in EtOAc (50 mL) and filtered to give product **01-4** (3.25 g, 8.7 mmol, 6% from **1**). The aqueous filtrate was extracted with EtOAc (3 x 300 mL). The combined organic layers were washed with $H_2O$ (3 x 300 mL), brine (200 mL) and dried over $Na_2SO_4$. After removal of the solvent *in vacuo* the crude product was subjected to column chromatography ($SiO_2$, EtOAc/Hept = 1/4) to give the product as a yellow solid. The solid was washed with EtOAc (20 mL), filtered off and washed with $Et_2O$ (2 x 25 mL) and dried to give **01-4** (3.25 g, 8.7 mmol) as an off-white solid. (Total yield of **01-4** from **01-1:** 6.5 g, 17.4 mmol, 12%).

*2-Acetylamino-5-butyl-7-oxo-4,5,6,7-tetrahydro-benzo[b]thiphene-3-carboxylic acid ethyl ester* **(03-4)**

Crude mixture **03-3** was converted with $Na_2Cr_2O_7 \cdot 2H_2O$ (75.1 g, 252 mmol) according to the procedure for **01-4.** The crude product was subjected to column chromatography ($SiO_2$, EtOAc/Hept = 1/4) giving product **03-4** as a yellow solid. The solid was washed with $Et_2O$ (25 mL), filtered and washed with a small amount of $Et_2O$ to give compound **03-4** (7.75 g, 23 mmol, 14% from **03-1)** as a white solid. From the mother liquor a second crop of compound **03-4** (2.2 g, 6.5 mmol, 4% from **03-1)** was obtained as a white solid. Total yield of **03-4:** 9.95 g, 29.5 mmol, 18% from **03-1.**

*2-Acetylamino-5-benzyl-6-bromo-7-oxo-4,5,6,7-tetrahydro-benzo[b]thiophene-3-carboxylic acid ethyl ester* **(01-5)**

A suspension of compound **01-4** (6.04 g, 16.3 mmol) in EtOAc (400 mL) was treated with $CuBr_2$ (7.3 g, 32.6 mmol, 2 equiv.). The resulting mixture was refluxed for 18 h under a $N_2$-atmosphere. After cooling to RT the mixture was filtered over Celite. The solvent was removed *in vacuo* to give a pink solid. After treatment with EtOAc the crude solid was filtered and washed with $Et_2O$ (3 x 20 mL) to give **01-5** as a pink solid (3.35 g, 7.4 mmol, 46%). The mother liquor was evaporated *in vacuo* and the residue was subjected to column chromatography ($SiO_2$, EtOAc/Hept = 1/4) to give the product (950 mg) as off-white solid with a purity of 66% (HPLC-MS).

*2-Acetylamino-5-butyl-6-bromo-7-oxo-4,5,6,7-tetrahydro-benzo[b]thiophene-3-carboxylic acid ethyl ester* **(03-5)**

Compound **03-4** (324 g, 9.6 mmol) was converted with $CuBr_2$ (4.3 g, 192 mmol) using the procedure for **01-5.** Two other batches (from a total of 4.44 g, 13.6 mmol of **03-4)** were prepared and the crude products were combined and purified by column chromatography ($SiO_2$, EtOAc/Hept = 1/4) to give two fractions of product. The first fraction (3.84 g, 92 mmol, 40%) was obtained as orange oil, the second fraction (2.02 g, 4.8 mmol, 21%) was obtained as a brown oily solid and contained some dibrominated ketone.

*2-Acetylamino--5-benzyl-7-hydroxy-benzo[b]thiophene-3-carboxylic acid ethyl ester* **(01-6)**

A flame dried three-necked flask was purged with $N_2$ and charged with bromide **01-5** (3.35 g, 7.4 mmol), LiBr (707 mg, 82 mmol, 1.1 equiv.), $Li_2CO_3$ (601 mg, 82 mmol, 1.1 equiv.) and DMF (85 mL). The resulting suspension was refluxed overnight under $N_2$-atmosphere. The reaction mixture was cooled to RT and poured into saturated aqueous $NH_4Cl$ (500 mL). The aqueous layer was extracted with EtOAc (3 x 100 mL). The combined organic layers were washed with $H_2O$ (3 x 200 mL), brine (200 mL) and dried over $Na_2SO_4$. The solvent was removed *in vacuo* to give the crude product as a brownish solid. The solid was washed with $Et_2O$ (25 mL) to give **01-6** (2.1 g, 5.7 mmol, 77 %) as a slightly brown solid.

*2-Acetylamino-5-butyl-7-hydroxy-benzo[b]thiophene-3-carboxylic acid ethyl ester* **(03-6)**

Compound **03-5** (4.54 g, 10.9 mmol) was converted with LiBr (1.04 g, 12 mmol, 1.1 equiv.), $Li_2CO_3$ (886 mg, 12 mmol, 1.1 equiv.) to **03-6** using the procedure for **01-6**. The crude product was washed with EtOAc (15 mL) and washed with $Et_2O$ (2 x 15 mL) to give **03-6** (1.8 g, 5.4 mmol 49%) as a brownish solid. The mother liquor was evaporated and treated with $Et_2O$ to give a second crop of **03-6** (0.67 g, 2.0 mmol, 18%) as a brownish solid. Total yield of **03-6:** 2.47 g, 7.4 mmol, 68%.

*2-Amino-5-benzyl-7-hydroxy-benzo[b]thiophene-3-carboxylic acid ethyl ester* **(a-1)**

Compound **01-6** (2.1 g, 5.7 mmol) was dissolved in MeOH (250 mL), $H_2SO_4$ (1 mL) was added and the mixture was stirred for 3 days. Another batch of **01-6** (450 mg, 1.2 mmol) was added and the mixture was stirred further for 14 days while monitoring the reaction each third day by HPLC. After completion the mixture was concentrated to half of the original volume. Water was added and the pH was cautiously adjusted to 8 with conc. $NH_4OH$. The aqueous layer was extracted with EtOAc (3 x 100 mL). The combined organic layers were washed with $H_2O$ (100 mL), brine (100 mL) and dried over $Na_2SO_4$. The solvent was removed *in vacuo* to give product **a-1** (2.05 g, 6.3 mmol, 91%) as a greenish brown foam.

*2-Amino-5-butyl-7-hydroxy-benzo[b]thiophene-3-carboxylic acid ethyl ester* **(a-3)**

Compound **03-6** (3.64 g, 10.9 mmol) was suspended in a mixture of MeOH (450 mL) and MeCN (100 mL). $H_2SO_4$ (1.5 mL) was added and the mixture was stirred under a $N_2$-atmosphere, while monitoring the reaction by HPLC. After 7 days the conversion was complete and the mixture was concentrated to half of the original volume. Water was added and the pH was cautiously adjusted to 8 with conc. $NH_4OH$. The aqueous layer was extracted with EtOAc (3 x 100 mL). The combined organic layers were washed with $H_2O$ (100 mL), brine (100 mL) and dried over $Na_2SO_4$. The solvent was removed *in vacuo* to give the crude product as a green sticky oil, which was purified by column chromatography ($SiO_2$, EtOAc/Hept =1/4 +1% $Et_3N$). The product fractions were pooled and the solvent was removed *in vacuo*. The residue was dissolved in $Et_2O$ (50 mL) and the solution was washed with conc. aq. $NH_4Cl$ (2 x 50 mL) and brine (50 mL) to remove residual $NEt_3$. After drying over $Na_2SO_4$, the solvent was removed *in vacuo* to give **a-3** (2.6 g, 8.9 mmol, 82%) as a greenish grey solid.

<u>Synthesis of 2-Amino-4-benzyl-7-hydroxy-benzo[b]thiophene-3-carboxylic acid ethyl ester **a-2** and of -Amino-5-butyl-7-hydroxy-benzo[b]thiophene-3-carboxylic acid ethyl ester **a-4**</u>

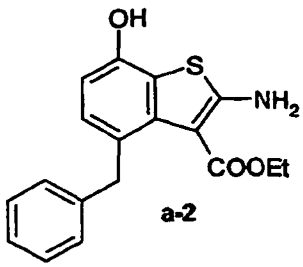

a-2

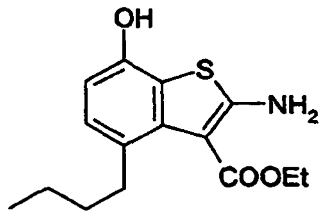

a-4

*SCHEME 10: Synthesis of compounds **a-2** (R = benzyl) and **a-4** (R = butyl)*

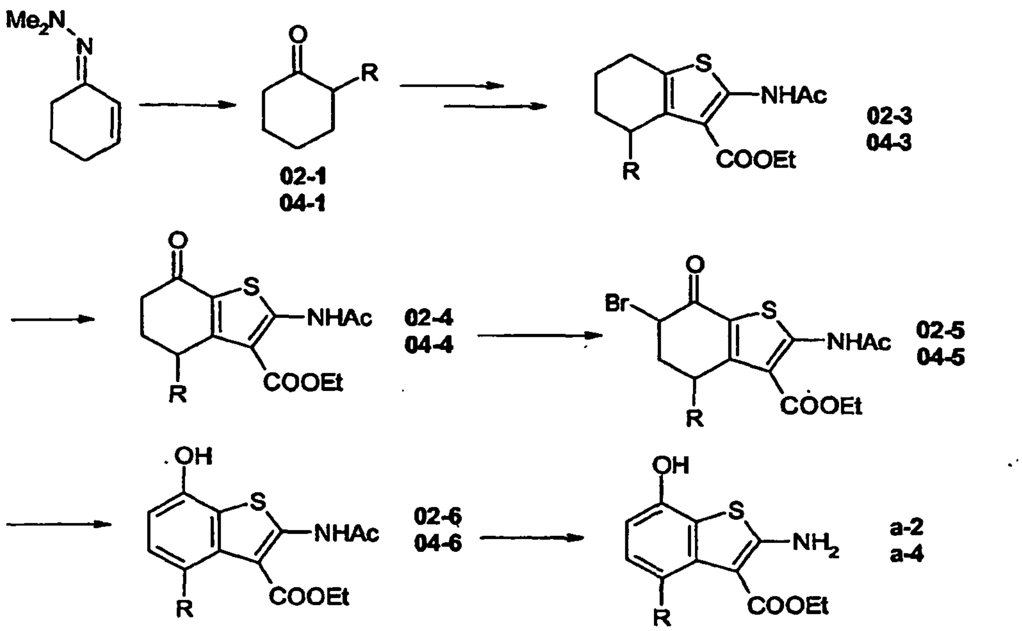

02-1 04-1
02-3 04-3
02-4 04-4
02-5 04-5
02-6 04-6
a-2 a-4

For the synthesis of compounds **a-2** and **a-4** a similar strategy as for compounds **a-1** and **a-3** was used (Scheme 10). Alkylation of cyclohexanone-N,N-dimethylhydrazone gave ketones **02-1** and **04-1.** The ketones were converted to the corresponding 2-amino thiophenes using the standard procedure, followed by immediate protection to give acetamides **02-3** and **04-3** in rather moderate yields (28, 30%). The thiophenes **02-3** and **04-3** were oxidised to ketones **02-4** and **04-4** with $Na_2Cr_2O_7$ in yields of 51% resp. 41%. Bromination with $CuBr_2$ in refluxing EtOAc afforded bromides **02-5** and **04-5** in 82% resp. 38% yield. Subsequent elimination with $LiBr/Li_2CO_3$ gave phenols **02-6** and **04-6** in 67% resp. 62% yield. Deprotection of **02-6** and **04-6** to the requested compounds **a-2** and **a-4** with $H_2SO_4$ in MeOH proceeded in 55% resp. 51% yield.

**Detailed Synthesis**

*2-Benzyl-cyclohexanone* **(02-1)**

A mixture of cyclohexanone-N,N-dimethylhydrazone (30 g, 214 mmol) and THF (400 mL) mixture was cooled to 5˚C. A 2.5 M-solution of *n*-BuLi (90 mL, 225 mmol, 1.05 equiv.) was added dropwise keeping the internal temperature below 0˚C. After complete addition the mixture was stirred for 1h at 5˚C. Then benzylbromide was added slowly. After complete addition the reaction mixture was warmed to RT and stirred overnight. A solution of 3 N HCl (400 mL) was added and the mixture stirred for 2 h at RT. After dilution with $H_2O$ (400 mL), the layers were separated and the aqueous layer was extracted with EtOAc (2 x 200 mL). The combined organic layers were washed with $H_2O$ (2 x 400 mL), brine (400 mL) and dried over $Na_2SO_4$. The solvent was removed *in vacuo* to give crude **1a** (41.5 g, >214 mmol) which was used in the next step without further purification.

*3-Butyl-cyclohexanone* **(04-1)**

Compound **04-1** was prepared analogeously to **02-1** from cyclohexanone-N,N-dimethyl-hydrazone (30 g, 214 mmol), *n*-BuLi (90 mL of 2.5 M, 225 mmol, 1.05 equiv.) and n-butylbromide (30.2 g, 220 mmol, 1.03 equiv) to give crude **04-1** (30.0 g, 194 mmol, 91%) as an orange oil, which was used in the next step without further purification.

*2-Amino-6-benzyl-4,5,6,7-tetrahydro-benzo[b]thiophene-3-carboxylic acid ethyl ester* **(02-2)**

Compound **02-1** (crude, max. 214 mmol) was mixed with EtOH (110 mL), ethyl cyanoacetate (23 mL, 24.4 g, 214 mmol) and sulfur (6.85 g, 214 mmol). To the resulting mixture morpholine (21 mL) was added and the mixture was heated to reflux for 24 h. It was concentrated to half of the original volume and poured into $H_2O$ (600 mL). The aqueous layer was extracted with EtOAc (3 x 200 mL). The combined organic layers were washed with $H_2O$ (2 x 200 mL), brine (200 mL), dried over $Na_2SO_4$ and the solvent was removed *in vacuo* to give the crude product as a red brown oil, which was used in the next step without further purification.

*2-Amino-6-butyl-4,5,6,7-tetrahydro-benzo[b]thiophene-3-carboxylic acid ethyl ester* **(04-2)**

Compound **04-2** was prepared from **04-1** (30 g, 194 mmol), ethyl cyanoacetate (20.7 mL, 21.9 g, 194 mmol), sulfur (6.2 g, 194 mmol) and morpholine (21 mL) using the procedure for **02-2** and was used in the next step without further purification.

*2-Acetylamino-6-benzyl-4,5,6,7-tetrahydro-benzo[b]thiophene-3-carboxylic acid ethyl ester* **(02-3)**

Crude **02-2** was dissolved in AcOH (65 mL). Acetic anhydride (65 mL) was added and the mixture was heated at 40˚C for 1.5 h. The mixture was cooled to RT and poured in $H_2O$ (600 mL), during which a solid precipitated. The solid was filtered off and washed with $H_2O$ and $Et_2O$ and dried in air to give **02-3** (4.9 g, 13.7 mmol, 6%) as an off-white solid. The filtrate was extracted with EtOAc (3 x 200 mL). The combined organic layers were washed with $H_2O$ (3 x 200 mL), brine (200 mL) and dried over $Na_2SO_4$. After removal of the solvent *in vacuo* the crude product was obtained as a semi-solid. After treatment with 100 mL of $Et_2O$, the solid was filtered off and washed with small portions of $Et_2O$ and dried in air to give **02-3** (172 g, 48 mmol, 22%) as a slightly yellow solid (total yield: 22.1 g, 62 mmol, 28%).

*2-Acetylamino-6-butyl-4,5,6,7-tetrahydro-benzo[b]thiophene-3-carboxylic acid ethyl ester* **(04-3)**

Conversion of crude **04-2** according to the procedure for **02-3** with $Ac_2O$ (60 mL) in AcOH (60 mL) gave crude product **04-3**. Column chromatography ($SiO_2$, EtOAc/hept = 1/9) afforded **04-3** (19.2 g, 59 mmol, 30%) as a yellow solid.

*2-Acetylamino-6-benzyl-7-oxo-4,5,6,7-tetrahydro-benzo[b]thiophene-3-carboxylic acid ethyl ester* **(02-4)**

Compound **02-3** (22.1 g, 62 mmol) was dissolved in AcOH (200 mL). The mixture was heated to 65˚C and a solution of $Na_2Cr_2O_7$-$2H_2O$ (27.2 g, 93 mmol) in hot $H_2O$ (200 mL) was added over a period of 2 min. After complete addition the mixture was stirred for 3 h at 65-67˚C and 2 h at 75˚C. The mixture was allowed to cool to RT and then quenched with saturated aq ueous $Na_2SO_3$ (100 mL) and diluted with $H_2O$ (1.5 L). The aqueous mixture was extracted with EtOAc (3 x 300 mL). The combined organic layers were washed with $H_2O$ (3 x 400 mL), brine (400 mL) and dried over $Na_2SO_4$. After removal of the solvent *in vacuo* the crude product was treated with $Et_2O$ (50 mL) and filtered off to give **02-4** (9.5 g, 25 mmol, 41%) as yellow powder containing about 8% of starting material **02-3** (HPLC).

*2-Acetylamino-6-butyl-7-oxo-4,5,6,7-tetrahydro-benzo[b]thiphene-3-carboxylic acid ethyl ester* **(04-4)**

Compound **04-b** (19.1 g, 59 mmol) was converted with $Na_2Cr_2O_7$-$2H_2O$ (26.4 g, 88.5 mmol) according to the procedure for **02-4**. The crude product was subjected to column chromatography ($SiO_2$, EtOAc/hept = 1/4) giving product **04-4** (10.1 g, 30 mmol, 51%) as a yellow oil, that solidified upon standing to a yellow wax-like solid.

*2-Acetylamino-6-benzyl-6-bromo-7-oxo-4,5,6,7-tetrahydro-benzo[b]thiophene-3-carboxylic acid ethyl ester* **(02-5)**

A suspension of compound **02-4** (4.5 g, 11 mmol) in EtOAc (80 mL) was treated with $CuBr_2$ (5.4 g, 24 mmol, 2.2 equiv.). The resulting mixture was refluxed for 6 h under a $N_2$-atmosphere. After cooling to RT the mixture was filtered over Celite. The solvent was removed *in vacuo* to give a pink solid, which was treated with EtOAc (25 mL). The solid was filtered off and washed with $Et_2O$ (3 x 20 mL) to give **02-5** as a slightly brown solid (4.1 g, 9.0 mmol, 82%).

*2-Acetylamino-6-butyl-6-bromo-7-oxo-4,5,6,7-tetrahydro-benzo[b]thiophene-3-carboxylic acid ethyl ester* **(04-5)**

Compound **04-4** (10.2 g, 39.1 mmol) was converted with $CuBr_2$ (13.4 g, 60.2 mmol) using the procedure for **02-5** giving **04-5** (4.7 g, 11.4 mmol, 38%) as a grey powder.

*2-Acetylamino-5-benzyl-7-hydroxy-benzo[b]thiophene-3-carboxylic acid ethyl ester* **(02-6)**

A suspension of the bromide **02-5** (8.5 g, 18.9 mmol), LiBr (1.8 g, 20.8 mmol, 1.1 equiv.), $Li_2CO_3$ (1.5 g, 20.8 mmol, 1.1 equiv.) and DMF (200 mL) was heated to 150° C for 4 h under a $N_2$-atmosphere. The reaction mixture was cooled to RT and poured into saturated aq eous $NH_4Cl$ (750 mL). The aqueous layer was extracted with EtOAc (3 x 200 mL). The combined organic layers were washed with $H_2O$ (3 x 200 mL), sat. aq. $NH_4Cl$ (200 mL) and dried over $Na_2SO_4$. The solvent was removed *in vacuo* to give the crude product. The solid was washed with $Et_2O$ (25 mL) to give **02-6** (1.52 g, 4.1 mmol, 22 %). The filtrate was evaporated and the residue was purified by column chromatography ($SiO_2$, EtOAc/hept = 1/1) to give another fraction of **02-6** (25 g, 6.8 mmol, 36%) as a yellow solid (total yield: 4.3 g, 11.7 mmol, 62%).

*2-Acetylamino-6-butyl-7-hydroxy-benzo[b]thiophene-3-carboxylic acid ethyl ester* **(04-6)**

Compound **04-5** (4.74 g, 10.9 mmol) was converted with LiBr (1.1 g, 12.5 mmol, 1.1 equiv.), $Li_2CO_3$ (0.9 g, 12.5 mmol, 1.1 equiv.) to **04-6** using the procedure for **02-6.** The crude product was filtered off after aqueous workup, washed with $H_2O$ and $Et_2O$ (25 mL) to give **04-6** (1.9 g, 5.7 mmol, 50%) as a grey solid. The mother liquor was evaporated and treated with $Et_2O$ to give a second crop of **04-6** (0.6 g, 1.9 mmol, 17%) as a brownish solid (total yield of **04-6:** 2.6 g, 7.6 mmol, 67%).

*2-Amino-6-benzyl-7-hydroxy-benzo[b]thiophene-3-carboxylic acid ethyl ester* **(a-2)**

Compound **02-6** (4.3 g, 11.7 mmol) was dissolved in MeOH (350 mL), conc. $H_2SO_4$ (2 mL) was added and the mixture was stirred for 8 days. The mixture was concentrated to half of the original volume. $H_2O$ was added and the pH was cautiously adjusted to 8 with conc. $NH_4OH$. The aqueous layer was extracted with EtOAc (3 x 100 mL). The combined organic layers were washed with $H_2O$ (100 mL), brine (100 mL) and dried over $Na_2SO_4$. The solvent was removed *in vacuo* to give the crude product, which was purified by column chromatography ($SiO_2$, EtOAc/hept = 1/4 + 1% $Et_3N$ to 1/1). The product was obtained in only 80% purity according to HPLC and therefore subjected to automated column chromatography to give **a-2** (2.1 g, 6.4 mmol, 55%) as a brown-yellow solid.

*2-Amino-6-butyl-7-hydroxy-benzo[b]thiophene-3-carboxylic acid ethyl ester* **(a-4)**

Compound **04-b** (2.6 g, 7.6 mmol) was mixed with MeOH (200 mL) and treated with conc. $H_2SO_4$ (1 mL) for 9 d using the procedure as described for **a-2** The crude product was purified by automated column chromatography to give **a-4** (1.1 g, 3.7 mmol, 50%) as orange oil.

**Compounds No. 21 to 123 of the general formula q-3-OH**

The following tables 1, 2, 3 and 4 list compounds No. 21 to 123 of the general formula **q**, which were prepared according to Scheme **6** starting with the primary amines R1-$NH_2$ and the acid chlorides R2-CO-Cl as given in the third and fourth column of each table. The compounds were synthesized using different starting compounds **a-3OH** (a-1, a-2, a-3 and a-4) giving rise to products of general formula **q-3-OH,** i.e. **q-1** (table 1), **q-2** (table 2), **q-3** (table 3) and **q-4** (table 4), respectively.

Synthesis Protocol:

In a reaction vessel at room temperature are put together sequentially 200 µl of 0.25 M primary amine R1-$NH_2$, 200 µl of 1 M diisopropylethylamine and 50 µl of 0.25 M add chloride R2-CO-Cl. To this mixture is added 200 µl of 0.25 M substituted and optionally protected 2-amino-benzo[b]thiophene-3-carboxylic acid ethyl ester **a-3-OH** followed by 200 µl of 0.25 M $POCl_3$. Of all reactants a solution or suspension in chlorobenzene is used. After shaking for 80 hours at 100°C, the mixtures are cooled to room temperature, washed with 5% NaOAc and extracted with EtOAc. The organic layers are collected and concentrated to yield the desired compound. The obtained material of the general formula **q-3-OH** was thereafter analyzed by LC-MS.

In addition, the Molecular Weight and the Retention Time of the synthesized compounds determined by the LC-MS analysis are shown in each table.

**Table 1: Compounds No. 21 to 41 of the general formula q-1:**

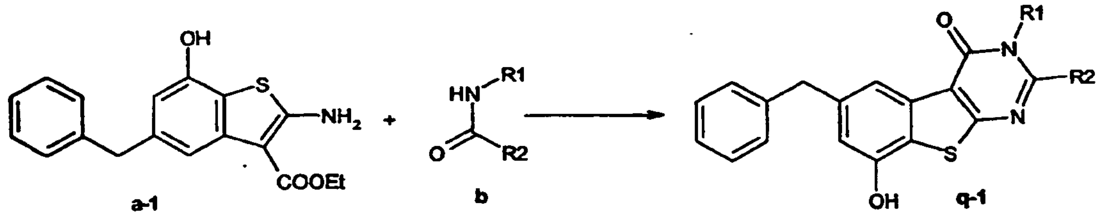


| No. | Formula | R1-$NH_2$ (name) | R2-CO-Cl (name) | MH+ | RT [min] |
|---|---|---|---|---|---|
| 21 | 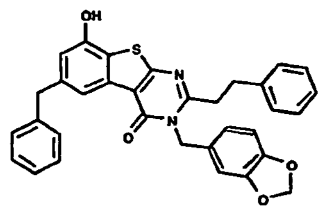 | 3,4-METHYLENE-DIOXYBENZYLAMINE | 3-PHENYL-PROPIONYL CHLORIDE | 546.16 | 2.21 |
| 22 | 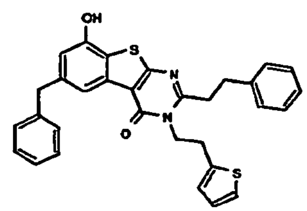 | THIOPHENE-2-ETHYLAMINE | 3-PHENYL-PROPIONYL CHLORIDE | 522.14 | 2.23 |
| 23 | 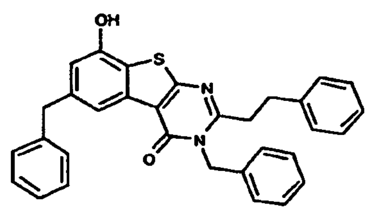 | BENZYLAMINE | 3-PHENYL-PROPIONYL CHLORIDE | 502.17 | 2.23 |

(continued)

| No. | Formula | R1-$NH_2$ (name) | R2-CO-Cl (name) | MH+ | RT [min] |
|---|---|---|---|---|---|
| 24 | 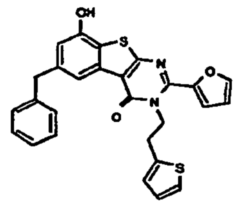 | THIOPHENE-2-ETHYLAMINE | 2-FUROYL CHLORIDE | 484.09 | 2.20 |
| 25 | 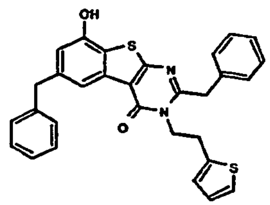 | THIOPHENE-2-ETHYLAMINE | PHENYLACETYL CHLORIDE | 508.13 | 2.21 |
| 26 | 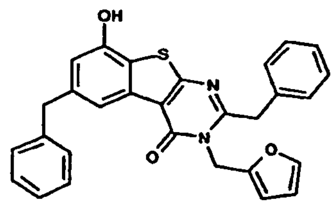 | FURFURYL-AMINE | PHENYLACETYL CHLORIDE | 478.14 | 2.15 |
| 27 | 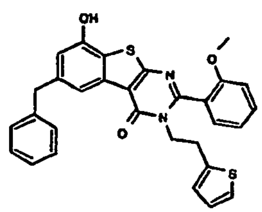 | THIOPHENE-2-ETHYLAMINE BENZOYL | 2-METHOXY-CHLORIDE | 524.12 | 2.12 |

(continued)

| No. | Formula | R1-NH$_2$ (name) | R2-CO-Cl (name) | MH+ | RT [min] |
|---|---|---|---|---|---|
| 28 | 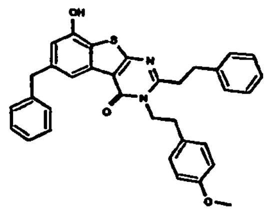 | 2-(4-METHOXY-PHENYL)-ETHYLAMINE | 3-PHENYL-PROPIONYL CHLORIDE | 546.20 | 2.25 |
| 29 | 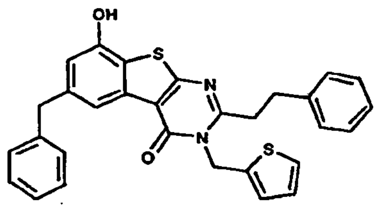 | THIOPHENE-2-METHYLAMINE | 3-PHENYL-PROPIONYL CHLORIDE | 508.13 | 2.20 |
| 30 | 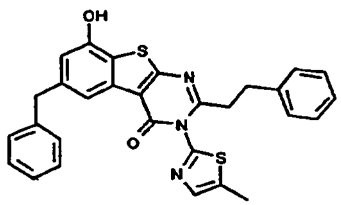 | 2-AMINO-5-METHYL-THIAZOLE | 3-PHENYL-PROPIONYL CHLORIDE | 509.12 | 2.19 |
| 31 | 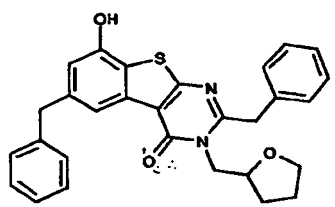 | TETRAHYDRO- FURFURYL-AMINE | PHENYLACETYL CHLORIDE | 482.17 | 2.14 |

(continued)

| No. | Formula | R1-$NH_2$ (name) | R2-CO-Cl (name) | MH+ | RT [min] |
|---|---|---|---|---|---|
| 32 | 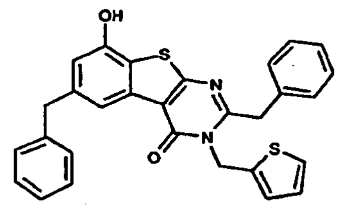 | THIOPHENE-2-METHYLAMINE | PHENYLACETYL CHLORIDE | 494.11 | 2.18 |
| 33 | 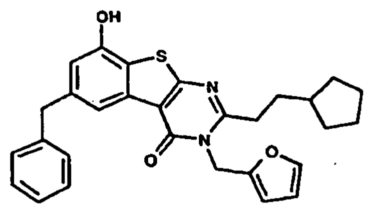 | FURFURYL-AMINE | 3-CYCLOPENTYL-PROPIONYL CHLORIDE | 484.18 | 2.29 |
| 34 | 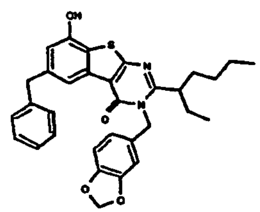 | 3,4-METHYLENE-DIOXY-BENZYLAMINE | 2-ETHYL-HEXANOYL CHLORIDE | 540.21 | 2.32 |
| 35 | 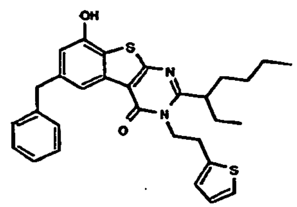 | THIOPHENE-2-ETHYLAMINE | 2-ETHYL-HEXANOYL CHLORIDE | 516.19 | 2.45 |

(continued)

| No. | Formula | R1-$NH_2$ (name) | R2-CO-Cl (name) | MH+ | RT [min] |
|---|---|---|---|---|---|
| 36 | 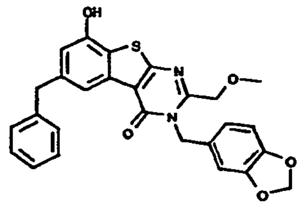 | 3,4-METHYLENE-DIOXY-BENZYLAMINE | METHOXY-ACETYL CHLORIDE | 486.12 | 2.08 |
| 37 | 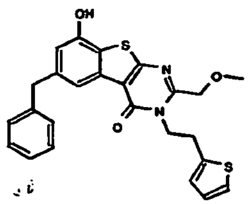 | THIOPHENE-2-ETHYLAMINE | METHOXY-ACETYL CHLORIDE | 462.11 | 2.10 |
| 38 | 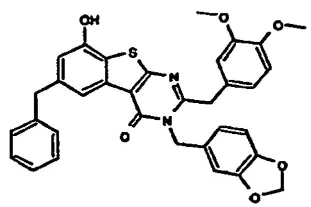 | 3,4-METHYLENE-DIOXY-BENZYLAMINE | (3,4-DIMETHOXY-PHENYL)-ACETYL CHLORIDE | 592.17 | 2.12 |
| 39 | 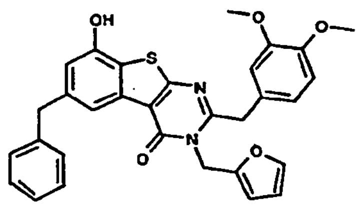 | FURFURYL-AMINE | (3,4-DIMETHOXY-PHENYL)-ACETYL CHLORIDE | 538.16 | 2.09 |

(continued)

| No. | Formula | $R1-NH_2$ (name) | R2-CO-Cl (name) | MH+ | RT [min] |
|---|---|---|---|---|---|
| 40 | 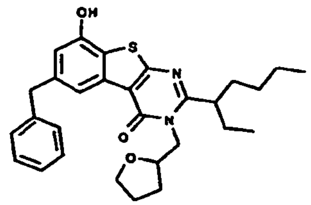 | TETRAHYDRO-FURFURYL-AMINE | 2-ETHYL-HEXANOYL CHLORIDE | 490.23 | 2.31 |
| 41 | 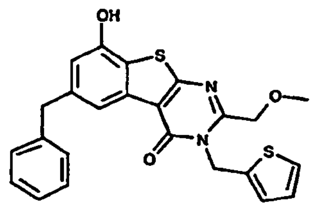 | THIOPHENE-2-METHYLAMINE | METHOXY- ACETYL CHLORIDE | 448.09 | 20.8 |

Table 2: Compounds No. 42 to 55 of the general formula q-2:

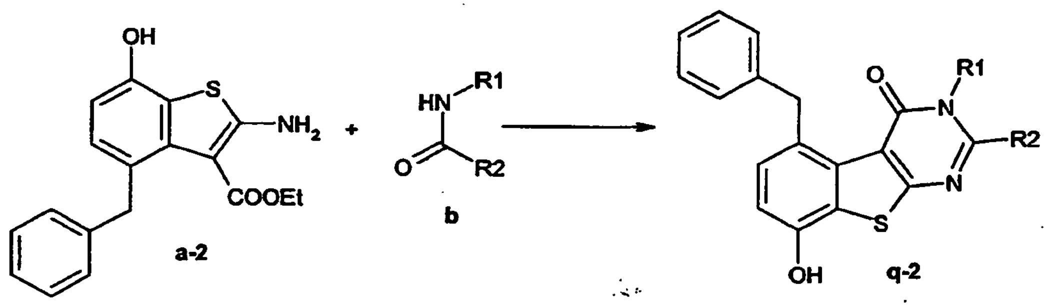

a-2 b q-2

| No | Formula | R1-NH$_2$ (name) | R2-CO-Cl (name) | MH+ | RT [min] |
|---|---|---|---|---|---|
| 42 | 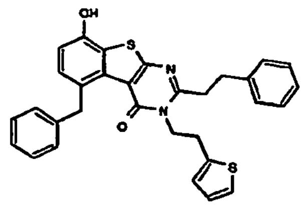 | THIOPHENE-ETHYLAMINE | 3-PHENYL-PROPIONYL CHLORIDE | 522.14 | 2.24 |
| 43 | 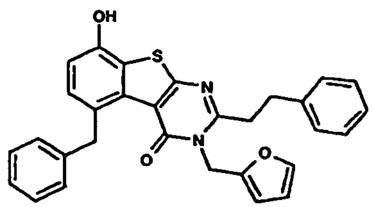 | FURFURYLAMINE | 3-PHENOL-PROPIONYL CHLORIDE | 492.15 | 2.18 |
| 44 | 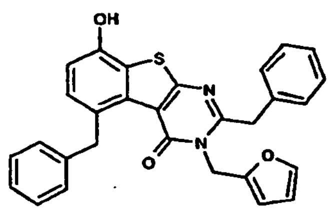 | FURFURYLAMINE | PHENYLACETYL CHLORIDE | 478.14 | 2.16 |

(continued)

| No | Formula | R1-$NH_2$ (name) | R2-CO-Cl (name) | MH+ | RT [min] |
|---|---|---|---|---|---|
| 45 | 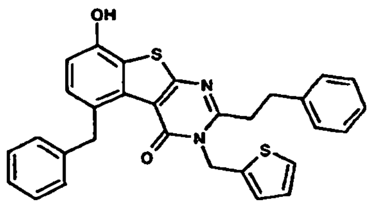 | THIOPHENE-2-METHYLAMINE | 3-PHENYL-PROPIONYL CHLORIDE | 508.13 | 2.20 |
| 46 | 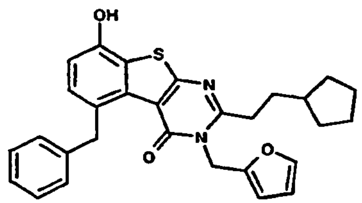 | FURFURYLAMINE | 3-CYCLOPENTYL-CHLORINE | 484.18 | 2.30 |
| 47 | 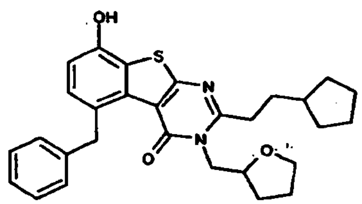 | TETRAHYDRO-FURFURYLAMINE | 3-CYCLOPENTYL-PROPIONYL CHLORIDE | 488.21 | 2.27 |
| 48 | 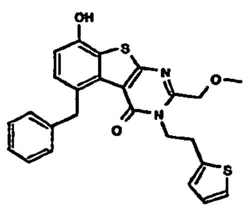 | THIOPHENE-2-ETHYLAMINE | METHOXY-ACETYL CHLO-RIDE | 462.11 | 2.10 |

(continued)

| No | Formula | R1-$NH_2$ (name) | R2-CO-Cl (name) | MH+ | RT [min] |
|---|---|---|---|---|---|
| 49 | 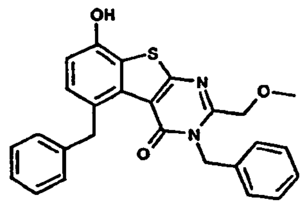 | BENZYLAMINE | METHOXY-ACETYL CHLORIDE | 442.14 | 2.10 |
| 50 | 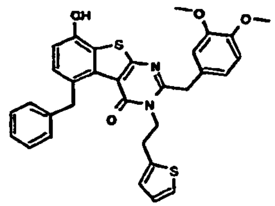 | THIOPHENE-2- ETHYLAMINE | (3,4-DIMETHOXY-PHENYL) ACETYL CHLORIDE | 568.15 | 2.14 |
| 51 | 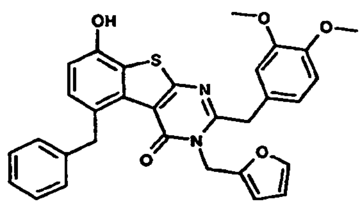 | FURFURYLAMINE | (3,4-DIMETHOXY-PHENYL) ACETYL CHLORIDE | 538.16 | 2.09 |
| 52 | 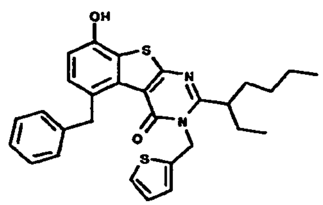 | THIOPHENE-2-METHYLAMINE | 2-ETHYL-HEXANOYL CHLORIDE | 502.17 | 2.31 |

(continued)

| No | Formula | R1-$NH_2$ (name) | R2-CO-Cl (name) | MH+ | RT [min] |
|---|---|---|---|---|---|
| 53 | 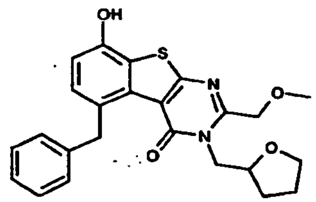 | TETRAHYDRO-FURFURYLAMINE | METHOXY-ACETYL CHLORIDE | 436.15 | 20.1 |
| 54 | 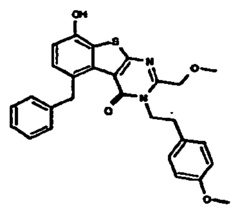 | 2-(4-METHOXY-PHENYL)-ETHYLAMINE | METHOXY-ACETYL CHLORIDE | 486.16 | 2.11 |
| 55 | 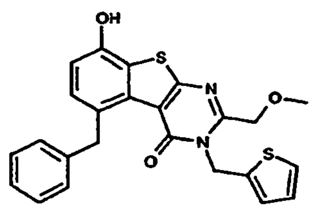 | THIOPHENE-2-METHYLAMINE | METHOXY-ACETYL CHLORIDE | 448.09 | 2.09 |

**Table 1: Compounds No. 56 to 96 of the general formula q-3:**

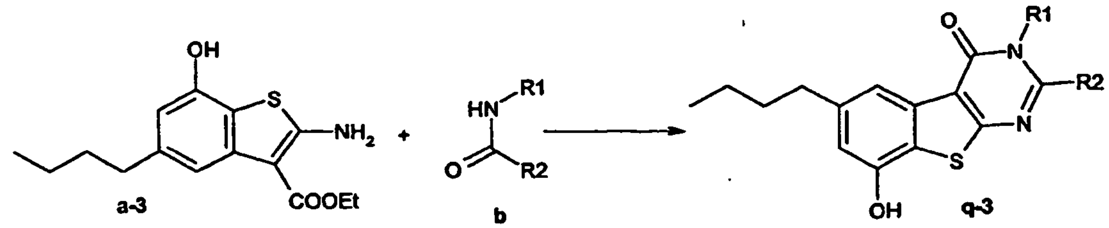

a-3 b q-3

| No | Formula | R1-$NH_2$ (name) | R2-CO-Cl (name) | MH+ | RT [min] |
|---|---|---|---|---|---|
| 56 | 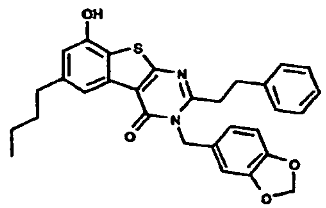 | 3,4-METHYLENE-DIOXYBENZYL-AMINE | 3-PHENYL-PROPIONYL CHLORIDE | 512.18 | 2.22 |
| 57 | 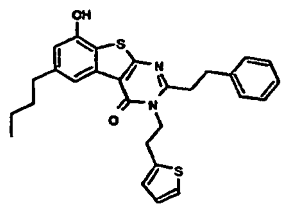 | THIOPHENE-2-ETHYLAMINE | 3-PHENYL-PROPIONYL CHLORIDE | 488.16 | 2.25 |
| 58 | 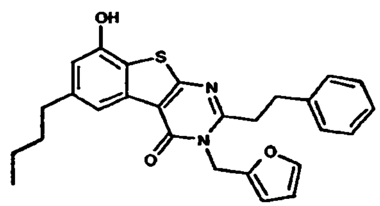 | FURFURYLAMINE | 3-PHENYL-PROPIONYL CHLORIDE | 458.17 | 2.18 |

(continued)

| No | Formula | R1-NH$_2$ (name) | R2-CO-Cl (name) | MH+ | RT [min] |
|---|---|---|---|---|---|
| 59 | 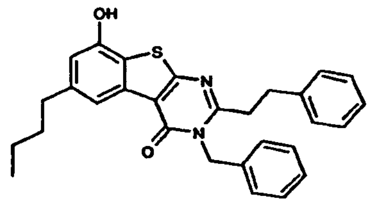 | BENZYLAMINE | 3-PHENYL-PROPIONYL CHLORIDE | 468.19 | 2.25 |
| 60 | 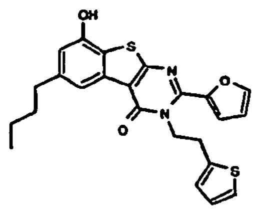 | THIOPHENE-2-ETHYLAMINE | 2-FUROYL CHLORIDE | 450.11 | 2.22 |
| 61 | 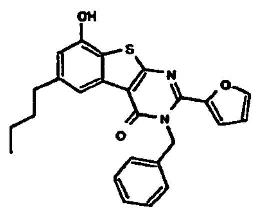 | BENZYLAMINE | 2-FUROYL CHLORIDE | 430.14 | 2.15 |
| 62 | 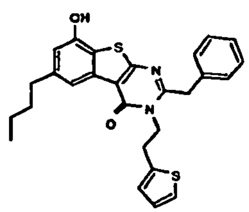 | THIOPHENE-2-ETHYLAMINE | PHENYLACETYL CHLORIDE | 474.14 | 2.24 |

(continued)

| No | Formula | R1-$NH_2$ (name) | R2-CO-Cl (name) | MH+ | RT [min] |
|---|---|---|---|---|---|
| 63 | 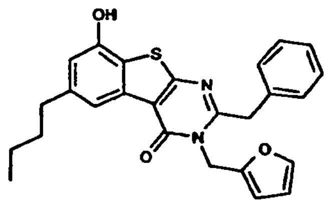 | FURFURYLAMINE | PHENYLACETYL CHLORIDE | 444.15 | 2.18 |
| 64 | 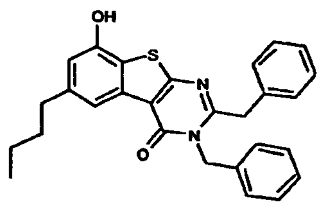 | BENZYLAMINE | PHENYLACETYL CHLORIDE | 454.17 | 2.22 |
| 65 | 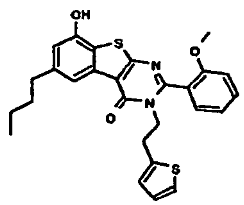 | THIOPHENE-2-ETHYLAMINE | 2-METHOXY-BENZOYL CHLORIDE | 490.14 | 2.15 |
| 66 | 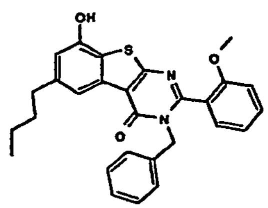 | BENZYLAMINE | 2-METHOXY-BENZOYL CHLORIDE | 470.17 | 2.15 |

(continued)

| No | Formula | R1-NH$_2$ (name) | R2-CO-Cl (name) | MH+ | RT [min] |
|---|---|---|---|---|---|
| 67 | 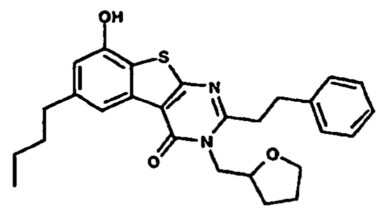 | TETRAHYDRO-FURFURYLAMINE | 3-PHENYL-PROPIONYL CHLORIDE | 462.20 | 2.18 |
| 68 | 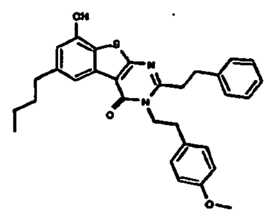 | 2-(4-METHOXY-PHENYL)-ETHYLAMINE | 3-PHENYL-PROPIONYL CHLORIDE | 512.21 | 2.28 |
| 69 | 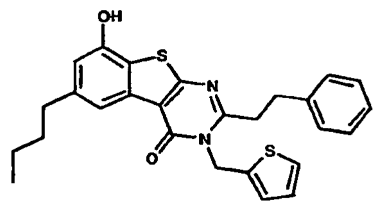 | THIOPHENE-2-METHYLAMINE | 3-PHENYL-PROPIONYL CHLORIDE | 474.14 | 2.22 |
| 70 | 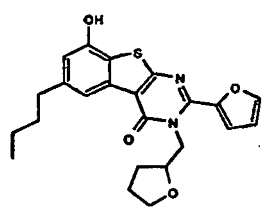 | TETRAHYDRO-FURFURYLAMINE | 2-FUROYL CHLORIDE | 424.15 | 2.08 |

(continued)

| No | Formula | R1-$NH_2$ (name) | R2-CO-Cl (name) | MH+ | RT [min] |
|---|---|---|---|---|---|
| 71 | 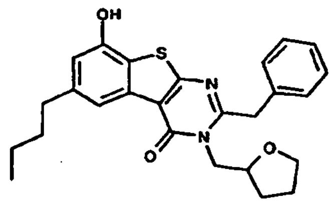 | TETRAHYDRO-FURFURYLAMINE | PHENYLACETYL CHLORIDE | 448.18 | 2.18 |
| 72 | 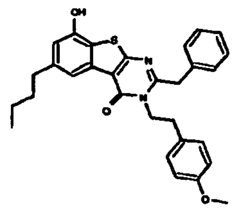 | 2-(4-METHOXY-PHENYL)-ETHYLAMINE | PHENYLACETYL CHLORIDE | 498.20 | 2.26 |
| 73 | 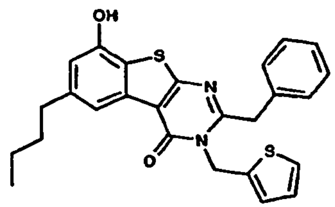 | THIOPHENE-2-METHYLAMINE | PHENYLACETYL CHLORIDE | 460.13 | 2.20 |
| 74 | 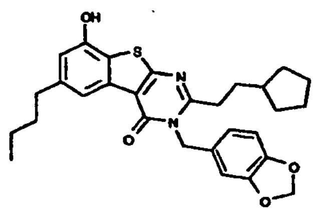 | 3,4-METHYLENE-DIOXY-BENZYLAMINE | 3-CYCLOPENTYL-PROPIONYL CHLORIDE | 504.21 | 2.39 |

(continued)

| No | Formula | R1-NH$_2$ (name) | R2-CO-Cl (name) | MH+ | RT [min] |
|---|---|---|---|---|---|
| 75 | 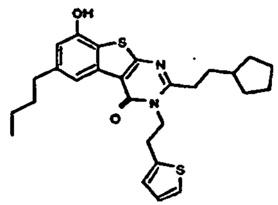 | THIOPHENE-2-ETHYLAMINE | 3-CYCLOPENTYL-PROPIONYL CHLORIDE | 480.19 | 2.44 |
| 76 | 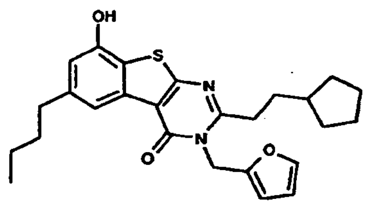 | FURFURYLAMINE | 3-CYCLOPENTYL-PROPIONYL CHLORIDE | 450.20 | 2.31 |
| 77 | 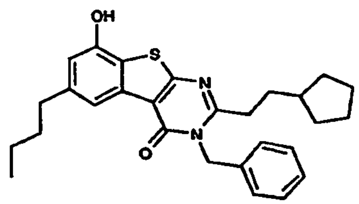 | BENZYLAMINE | 3-CYCLOPENTYL-PROPIONYL CHLORIDE | 460.22 | 2.44 |
| 78 | 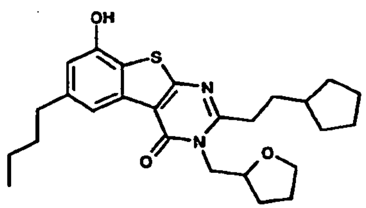 | TETRAHYDRO-FURFURYLAMINE | 3-CYCLOPENTYL-PROPIONYL CHLORIDE | 454.23 | 2.33 |

(continued)

| No | Formula | R1-$NH_2$ (name) | R2-CO-Cl (name) | MH+ | RT [min] |
|---|---|---|---|---|---|
| 79 | 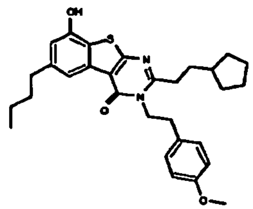 | 2-(4-METHOXY-PHENYL)-ETHYLAMINE | 3-CYCLOPENTYL-PROPIONYL CHLORIDE | 504.24 | 2.45 |
| 80 | 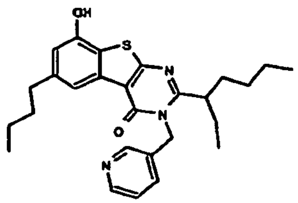 | 3-(AMINOMETHYL)-PYRIDINE | 2-ETHYL-HEXANOYL CHLORIDE | 463.23 | 2.18 |
| 81 | 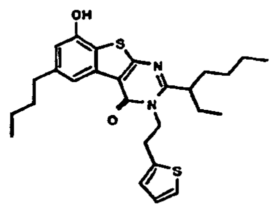 | THIOPHENE-2-ETHYLAMINE | 2-ETHYL-HEXANOYL CHLORIDE | 482.21 | 2.46 |
| 82 | 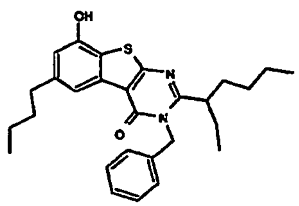 | BENZYLAMINE | 2-ETHYL-HEXANOYL CHLORIDE | 462.23 | 2.45 |

(continued)

| No | Formula | $R1-NH_2$ (name) | R2-CO-Cl (name) | MH+ | RT [min] |
|---|---|---|---|---|---|
| 83 | 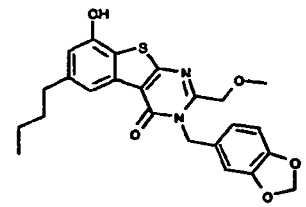 | 3,4-METHYLENE-DIOXYBENZYLAMINE | METHOXY-ACETYL CHLORIDE | 45.24 | 2.11 |
| 84 | 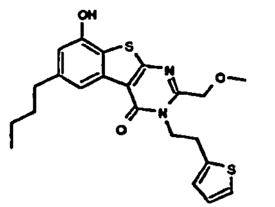 | THIOPHENE-2-ETHYLAMINE | METHOXY-ACETYL CHLORIDE | 428.12 | 2.12 |
| 85 | 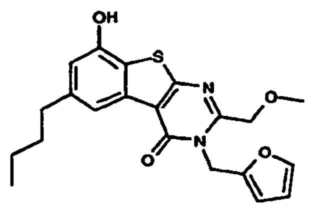 | FURFURYLAMINE | METHOXY-ACETYL CHLORIDE | 398.13 | 2.07 |
| 86 | 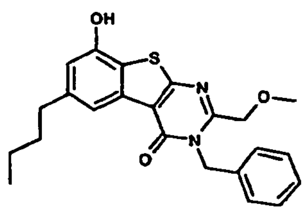 | BENZYLAMINE | METHOXY-ACETYL CHLORIDE | 408.15 | 2.11 |

(continued)

| No | Formula | R1-$NH_2$ (name) | R2-CO-Cl (name) | MH+ | RT [min] |
|---|---|---|---|---|---|
| 87 | 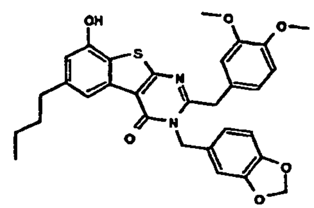 | 3,4-METHYLENE-DIOXY-BENZYLAMINE | (3,4-DIMETHOXY-PHENYL)-ACETYL CHLORIDE | 558.18 | 2.14 |
| 88 | 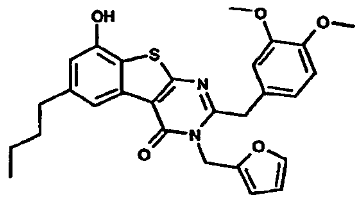 | FURFURYLAMINE | (3,4-DIMETHOXY-PHENYL)-ACETYL CHLORIDE | 504.17 | 2.11 |
| 89 | 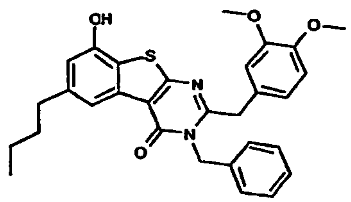 | BENZYLAMINE | (3,4-DIMETHOXY-PHENYL)-ACETYL CHLORIDE | 514.19 | 2.15 |
| 90 | 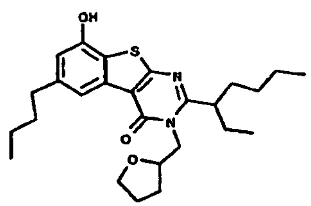 | TETRAHYDRO-FURFURYLAMINE | 2-ETHYL-HEXANOYL CHLORIDE | 456.24 | 2.36 |

(continued)

| No | Formula | R1-$NH_2$ (name) | R2-CO-Cl (name) | MH+ | RT [min] |
|---|---|---|---|---|---|
| 91 | 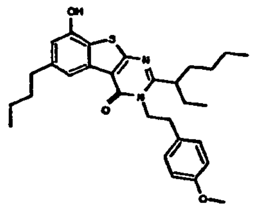 | 2-(4-METNOXY-PHENYL)-ETHYLAMINE | 2-ETHYL-HEXANOYL CHLORIDE | 506.26 | 2.50 |
| 92 | 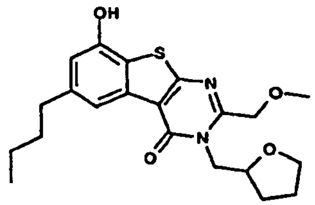 | TETRAHYDRO-FURFURYLAMINE | METHOXY-ACETYL CHLORIDE | 402.16 | 2.05 |
| 93 | 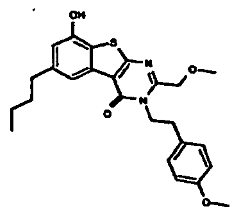 | 2-(4-METHOXY-PHENYL)-ETHYLAMINE | METHOXY-ACETYL CHLORIDE | 452.18 | 2.15 |
| 94 | 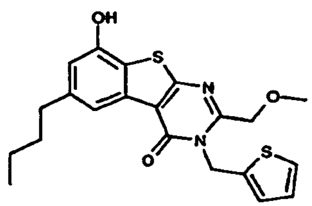 | THIOPHENE-2-MPTHYLAMINE | METHOXY-ACETYL CHLORIDE | 414.11 | 2.10 |

(continued)

| No | Formula | R1-$NH_2$ (name) | R2-CO-Cl (name) | MH+ | RT [min] |
|---|---|---|---|---|---|
| 95 | 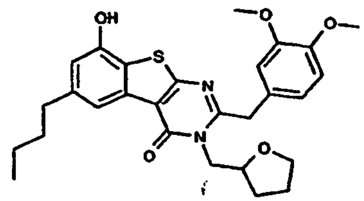 | TETRAHYDRO-FURFURYLAMINE | (3,4-DIMETHOXY-PHENYL)-ACETYL CHLORIDE | 508.20 | 2.09 |
| 96 | 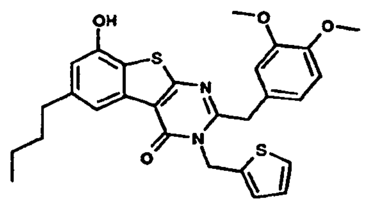 | THIOPHENE-2-METHYLAMINE | (3,4-DIMETHOXY-PHENYL)-ACETYL CHLORIDE | 520.15 | 2.14 |

**Table 4: Compounds No. 97 to 123 of the general formula q-4:**

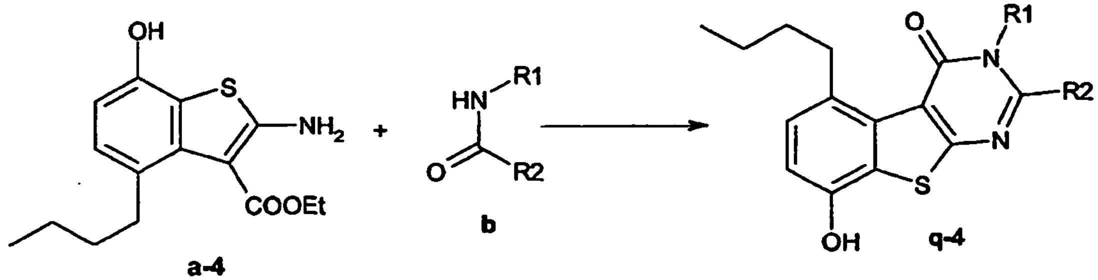

a-4 + b → q-4

| No | Formula | R1-NH$_2$ (name) | R2-CO-Cl (name) | MH+ | RT [min] |
|---|---|---|---|---|---|
| 97 | 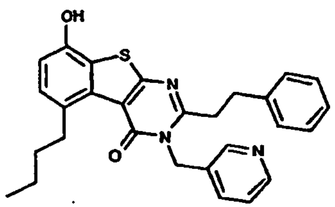 | 3-(AMINOMETHYL)-PYRIDINE | 3-PHENYL-3-PHENYL-PROPIONYL CHLORIDE | 469.18 | 2.13 |
| 98 | 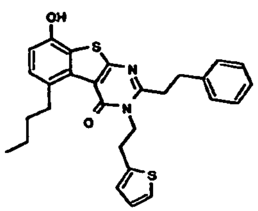 | THIOPHENE-2-ETHYLAMINE | 3-PHENYL-PROPIONYL CHLORIDE | 488.16 | 2.28 |
| 99 | 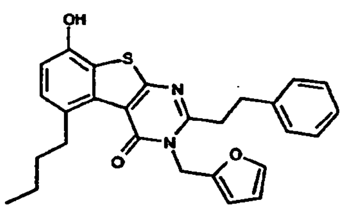 | FURFURYLAMINE | 3-PHENYL-PROPIONYL CHLORIDE | 458.17 | 2.23 |

(continued)

| No | Formula | R1-$NH_2$ (name) | R2-CO-Cl (name) | MH+ | RT [min] |
|---|---|---|---|---|---|
| 100 | 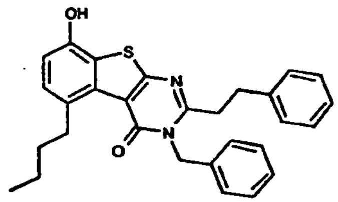 | BENZYLAMINE | 3-PHENYL-PROPIONYL CHLORIDE | 468.19 | 2.29 |
| 101 | 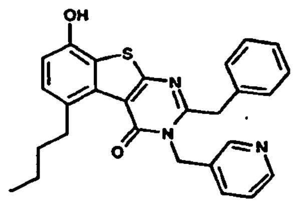 | 3-(AMINO-METHYL)-PYRIDINE | PHENYL-ACETYL CHLORIDE | 455.17 | 2.07 |
| 102 | 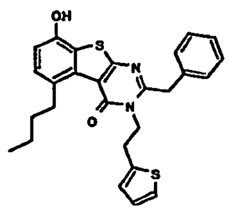 | THIOPHENE-2-ETHYLAMINE | PHENYLACETYL CHLORIDE | 474.14 | 2.26 |
| 103 | 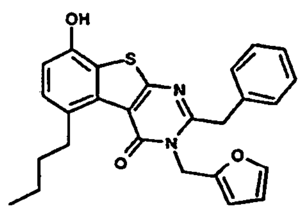 | FURFURYLAMINE | PHENYLACETYL CHLORIDE | 444.15 | 2.21 |

(continued)

| No | Formula | R1-$NH_2$ (name) | R2-CO-Cl (name) | MH+ | RT [min] |
|---|---|---|---|---|---|
| 104 | 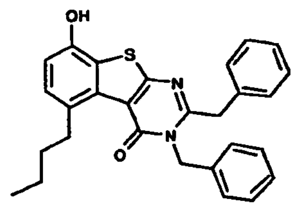 | BENZYLAMINE | PHENYLACETYL CHLORIDE | 454.17 | 2.26 |
| 105 | 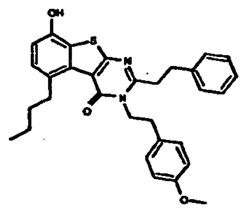 | 2-(4-METHOXY- PHENYL)-ETHYLAMINE | 3-PHENYL-PROPIONYL CHLORIDE | 512.21 | 2.32 |
| 106 | 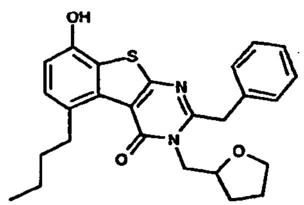 | TETRAHYDRO-FURFURYLAMINE | PHENYLACETYL CHLORIDE | 448.18 | 2.21 |
| 107 | 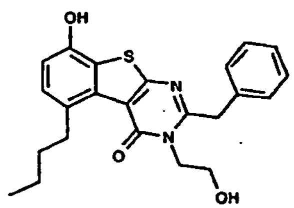 | ETHANOLAMINE | PHENYLACETYL CHLORIDE | 408.15 | 2.03 |
| 108 | 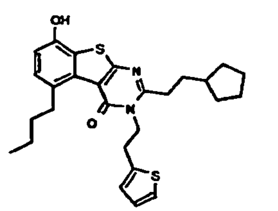 | THIOPHENE-2-ETHYLAMINE | 3-CYCLOPENTYL-PROPIONYL CHLORIDE | 480.19 | 2.47 |

(continued)

| No | Formula | R1-NH$_2$ (name) | R2-CO-Cl (name) | MH+ | RT [min] |
|---|---|---|---|---|---|
| 109 | 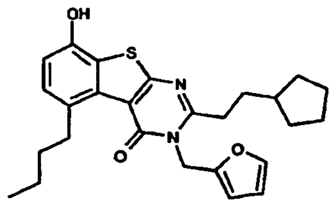 | FURFURYLAMINE | 3- CYCLOPENTYL-PROPIONYL CHLORIDE | 450.20 | 2.38 |
| 110 | 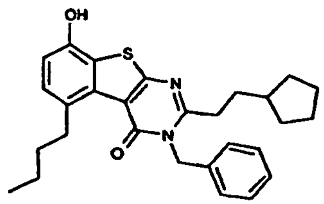 | BENZYLAMINE | 3-CYCLOPENTYL-PROPIONYL CHLORIDE | 460.22 | 2.45 |
| 111 | 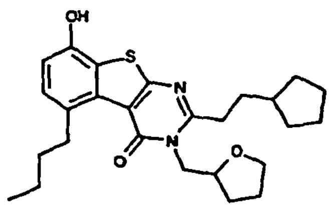 | TETRAHYDRO-FURFURYLAMINE | 3-CYCLOPENTYL-PROPIONYL CHLORIDE | 454.23 | 2.36 |
| 112 | 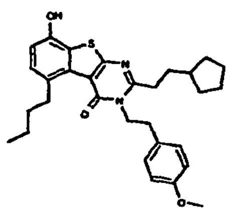 | 2-(4-METHOXY-PHENYL)-ETHYLAMINE | 3-CYCLOPENTYL-PROPIONYL CHLORIDE | 504.24 | 2.49 |

(continued)

| No | Formula | R1-$NH_2$ (name) | R2-CO-Cl (name) | MH+ | RT [min] |
|---|---|---|---|---|---|
| 113 | 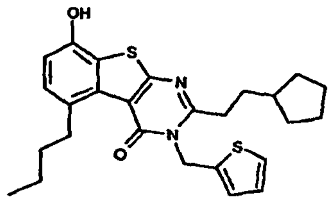 | THIOPHENE-2-METHYLAMINE | 3-CYCLOPENTYL-PROPIONYL CHLORIDE | 466.17 | 2.43 |
| 114 | 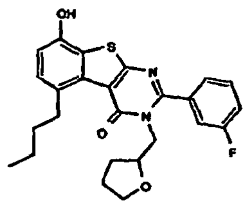 | TETRAHYDRO-FURFURYLAMINE | 3-FLUORO-BENZOYL CHLORIDE | 452.55 | |
| 115 | 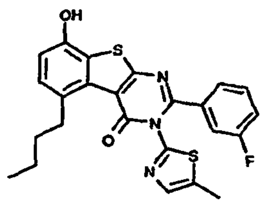 | 2-AMINO-5-METHYL-THIAZOLE | 3-FLUORO-BENZOYL CHLORIDE | 465.10 | 2.27 |
| 116 | 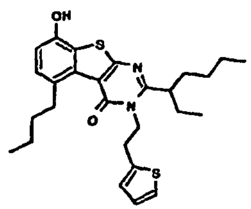 | THIOPHENE-2-ETHYLAMINE | 2-ETHYL-HEXANOYL CHLORIDE | 482.21 | 2.50 |
| 117 | 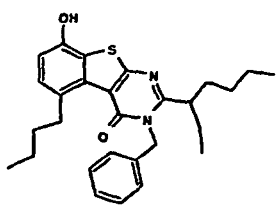 | BENZYLAMINE | 2-ETHYL-HEXANOYL CHLORIDE | 462.23 | 2.44 |

(continued)

| No | Formula | R1-$NH_2$ (name) | R2-CO-Cl (name) | MH+ | RT [min] |
|---|---|---|---|---|---|
| 118 | 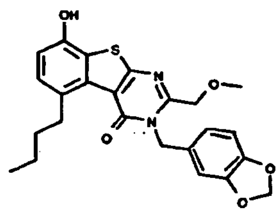 | 3,4-METHYLENE-DIOXYBENZYL-AMINE | METHOXY-ACETYL CHLORIDE | 452.14 | 2.12 |
| 119 | 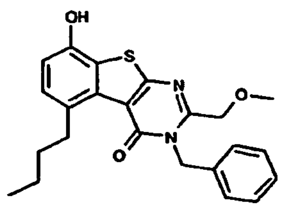 | BENZYLAMINE | METHOXY-ACETYL CHLORIDE | 408.15 | 2.13 |
| 120 | 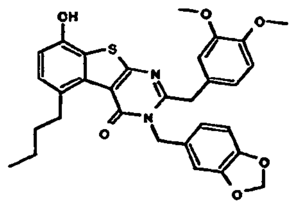 | 3,4-METHYLENE-DIOXYBENZYL-AMINE | (3,4-BIMETHOXY-PHENYL) ACETYL CHLORIDE | 558.18 | 2.15 |
| 121 | 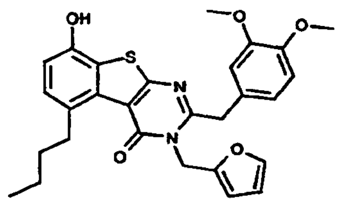 | FURFURYLAMINE | (3,4-DIMETHOXY-PHENYL) ACETYL CHLORIDE | 504.17 | 2.13 |
| 122 | 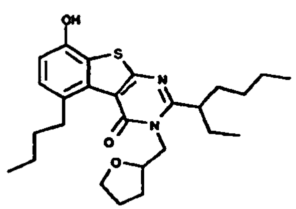 | TETRAHYDRO-FURFURYLAMINE | 2-ETHYL-HEXANOYL CHLORIDE | 456.24 | 2.36 |

(continued)

| No | Formula | R1-NH$_2$ (name) | R2-CO-Cl (name) | MH+ | RT [min] |
|---|---|---|---|---|---|
| 123 | 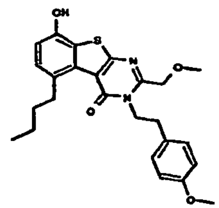 | 2-(4-METHOXY-PHENYL)-ETHYLAMINE | METHOXY-ACETYL CHLORIDE | 452.18 | 2.15 |

Further compounds falling under the scope of general formula (I) can be prepared by a reaction as shown in Scheme 6 using a primary amine R1-$NH_2$ selected from the following table 5 and an acid chloride R2-CO-Cl selected from the following table 6 as starting materials. This mixture is reacted with a particular 2-amino-benzo[b]thiophene-3-carboxylic acid ethyl ester of general formula a-3-OH (e.g. selected from the compounds as displayed in Scheme 7).

**Table 5: Different primary amines R1-$NH_2$ which can be used as starting materials**

| R1-$NH_2$ (name) |
|---|
| (S)-(+)-(2,2-DIMETHYL-[1,3]-DIOXOLAN-4-YL)-METHYLAMINE |
| 1-(3-AMINOPROPYL)-2-PYRROLIDINONE |
| 1-AMINOINDAN |
| 2-(2-AMINOETHYL)PYRIDINE |
| 2-AMINO-1-PHENYLETHANOL |
| 2-AMINO-5-METHYLTHIAZOLE |
| 2-AMINOACETOPHENONE HYDROCHLORIDE |
| 2-PHENOXYETHYLAMINE |
| 2-THIOPHENEETHYLAMINE |
| 3-(AMINOMETHYL)PYRIDINE |
| 3,4-DICHLOROBENZYLAMINE |
| 3,4-DIHYDROXYBENZYLAMINE |
| 3-AMINOPYRAZINE-2-CARBOXYLIC ACID METHYL ESTER |
| 3-AMINOQUINOLINE |
| 4-(2-AMINOETHYL)BENZENESULFONAMIDE MONOHYDROCHLORIDE |
| 4-(2-AMINOETHYL)MORPHOLINE |
| 4-(AMINOMETHYL)PYRIDINE |
| 4-AMINO-1-BENZYLPIPERIDINE |
| 4-METHYLSULFONYLBENZYLAMINE HYDROCHLORIDE |
| 5-(AMINOMETHYL)-2,3-DIHYDROBENZO[B]FURAN |
| 6-AMINOPHTHALIDE |
| 9-AMINOFLUORENE HYDROCHLORIDE |
| ANILINE |
| BENZYLAMINE |
| CYCLOHEXYLAMINE |
| CYCLOPROPYLAMINE |
| ETHANOLAMINE |
| FURFURYLAMINE |
| METHYL 4-AMINOBUTYRATE HYDROCHLORIDE |
| N-BUTYLAMINE |
| NN-DIMETHYLETHYLENEDIAMINE |
| PHENETHYLAMINE |
| PIPERONYLAMINE |
| TETRAHYDROFURFURYLAMINE |
| THIOPHENE-2-METHYLAMINE |

**Table 6: Different acid chloride R2-CO-Cl which can be used as starting materials**

| **R2-CO-Cl (name)** |
|---|
| 1-PHENYL-5-(TRIFLUOROMETHYL)PYRAZOLE-4-CARBONYL CHLORIDE |
| 2-(4-CHLOROPHENOXY)-2-METHYLPROPANOYL CHLORIDE |
| 2-(4-CHLOROPHENOXY)PYRIDINE-3-CARBONYL CHLORIDE |
| 2,4-DICHLOROBENZOYL CHLORIDE |
| **R2-CO-Cl (name)** |
| 2,4-DIFLOUROBENZOYL CHLORIDE |
| 2,6-DIFLUOROBENZOYL CHLORIDE |
| 2-ETHYLHEXANOYL CHLORIDE |
| 2-FUROYL CHLORIDE |
| 2-METHOXYBENZOYL CHLORIDE |
| 2-NAPHTHOYL CHLORIDE |
| 3,3-DIMETHYLACRYLOYL CHLORIDE |
| 3,4-DICHLOROBENZOYL CHLORIDE |
| 3,4-DIMETHOXYPHENYLACETYL CHLORIDE |
| 3,5-BIS(TRIFLUOROMETHYL)BENZOYL CHLORIDE |
| 3-CYANOBENZOYL CHLORIDE |
| 3-CYCLOPENTYLPROPIONYL CHLORIDE |
| 3-FLUOROBENZOYL CHLORIDE |
| 4-[(DIPROPYLAMINO)SULFONYL]BENZENE-1-CARBONYL CHLORIDE |
| 4-CYANOBENZOYL CHLORIDE |
| BENZO[B]THIOPHENE-2-CARBONYL Chloride |
| CYCLOPROPANECARBONYL CHLORIDE |
| DIPHENYLACETYL CHLORIDE |
| ETHYL SUCCINYL CHLORIDE |
| HYDROCINNAMOYL CHLORIDE |
| ISONICOTINOYL CHLORIDE HYDROCHLORIDE |
| METHOXYACETYL CHLORIDE |
| METHYL MALONYL CHLORIDE |
| METHYL OXALYL CHLORIDE |
| PHENYLACETYL CHLORIDE |

BIOLOGICAL TESTING MATERIALS AND METHODS

1. Inhibtion of the 17β-hydroxysteroid dehydrogenase type 1, type 2 and type 3 enzyme

The compounds were screened in respect of 17β-HSD enzyme activity in vitro on established MCF-7 cell lines, each stably expressing one of the respective 17β-HSD isoenzymes. The interconversion of substrate by each isoenzyme and the 17β-HSD inhibiting activity of chemical compounds in these cell lines were detected by HPLC system.

Varying amounts of the test compounds were incubated in the growth medium of the 17β-HSD expressing cells together tritium labeled substrate (2nM estrone for 17β-HSD type 1; 2 nM estradiol for 17β-HSD type 2; and 2 nM

androstenedione for 17β-HSD type 3). The medium samples were removed after exact incubation time and the reaction is stopped by trichloroacetic acid (TCA). The samples were analyzed by HPLC-coupled flow scintillation analysis.

For each enzyme type, the HSD-inhibiting activity of an individual test compound was calculated by comparing the conversion of a control sample without any test compound (referred to as "Negative Control") to the (reduced) conversion of the test sample containing the particular compound to be tested (referred to as "Test Sample").

$$\underline{\%\ \text{inhibition}} = 100 \times \frac{\text{Conversion in Negative Control} - \text{Conversion in Test Sample}}{\text{Conversion Negative Control}}$$

The obtained results are shown in Table 7 below. Two concentrations of each compound were used. The number of the compound refers to the numbers indicated in the Experimental Section.

**Table 7: % Inhibition of the 17β-HSD enzymes type 1, type 2 and type 3**

| Compound | HSD1 | | HSD2 | | HSD3 | |
|---|---|---|---|---|---|---|
| | 1 μM | 10 μM | 1 μM | 10 μM | 1 μM | 10 μM |
| No. 1 | 16.8 | 32.9 | 9.2 | 18.8 | 8.1 | 37.7 |
| No. 2 | 24.6 | 63.3 | 9.2 | 11.0 | 24.7 | 10.5 |
| No. 3 | 16.6 | 31.2 | 22.7 | 19.9 | 5.1 | 13.8 |
| No. 4 | 2.1 | 15.0 | 2.1 | 4.3 | -1.1 | 7.1 |
| No. 5 | -3.3 | 5.5 | 8.3 | 5.6 | 1.7 | 7.3 |
| No. 6 | 21.5 | 21.6 | 25.4 | 14.2 | 14.4 | 22.3 |
| No. 7 | 7.7 | 5.7 | -3.0 | 5.7 | -2.4 | 17.4 |
| No. 8 | 17.3 | 19.2 | 14.2 | 23.5 | -2.7 | 6.4 |
| No. 9 | 7.0 | 22.6 | 2.1 | -6.3 | -5.3 | 11.9 |
| No. 10 | 18.0 | 28.6 | 12.7 | 21.0 | 9.8 | 35.0 |
| No. 11 | 15.0 | 5.7 | 18.1 | 18.2 | 5.2 | 16.8 |
| No. 12 | 12.1 | 10.9 | 6.2 | 15.8 | 5.8 | 21.1 |
| No. 17 | 5.9 | 7.3 | 16.1 | 19.6 | 7.4 | 8.5 |

2. Estrogen Receptor Binding Assay

The binding affinity of the compounds of the invention to the estrogen receptor a and to the estrogen receptor β may be determined according to the in vitro ER binding assays described by Koffmann et al. [Koffmann B et al. (1991) J. Steroid. Biochem. Mol. Biol. 38:135]. Alternatively, an estrogen receptor binding assay may be performed according to international patent application PCT/US/17799 (published as WO 00/07996).

3. Estrogen Receptor Transactivation Assays

Compounds of the invention showing binding affinity towards the estrogen receptor may be further tested with regard to their individual estrogenic or anti-estrogenic potential (agonistic binding or antagonistic binding to the ERα or ERβ). The determination of the estrogen receptor agonist activity may be performed according to an in vitro assay system using the MMTV-ERE-LUC reporter system which is for example described within US patent application No. 10/289079 (published as US 2003/0170292):

To assay estrogen receptor agonist activity, Hela cells are grown in 24-well microtiter plates and then transiently co-transfected with two plasmids using lipofectamine. The first plasmid comprises DNA encoding human estrogen receptor (either ER-alpha or ER-beta), and the second plasmid comprises an estrogen-driven reporter system comprising: a luciferase reporter gene (LUC) whose transcription is under the control of upstream regulatory elements comprising

4 copies of the vitellogenin estrogen response element (ERE) cloned into the mouse mammary tumor virus (MMTV) promoter (the full name for the reporter system being "MMTV-ERE-LUC"). Cells are exposed to the compounds of the invention in RPMI 1640 medium, supplemented with 10% charcoal-treated fetal calf serum, 2 mM L-glutamine, 0.1 mM non-essential amino acids and 1 mM sodium pyruvate for 42-48 hours at 37˚C in a 5% carbon dioxide incubator. Concurrently, cells exposed to estradiol (1 nM) serve as positive controls. Replicate wells exposed to the solvent in which the compounds of the invention are dissolved (i.e. ethanol or methanol) are used as negative controls. After the 42-48 hr incubation period, cells are rinsed with phosphate buffered saline (PBS), lysis buffer (Promega Corp) is added, and cell lysates are collected for measurement of luciferase activity with a luminometer. Estrogenic activity of the compounds of the invention is expressed as fold-increase in luciferase activity as compared to that observed in negative control cells.

Alternatively, the determination of the estrogen receptor transactivation activity (estrogenicity assay or agonist assay) and of the inhibitory potency of transactivation activity (anti-estrogenicity assay or antagonist assay) may be performed according to international patent application PCT/US/17799 (published as WO 00/07996). CITED LITERATURE


- Andersson S. (1995) "Molecular genetics of androgenic 17β-Hydroxyteroid Dehydrogenases. J. Steroid Biochem. Molec. Biol., 55:533-534].
- Dong Y et al. (1998) "17β-hydroxysteroid dehydrogenases in human bone cells" J. Bone Min. Res., 13:1539-1546
- Gadad AK, Kapsi SG, Anegundi RI, Pattan SR, Mahajanshetti CS, Shishoo CJ. (1996) "Synthesis and antihyperlipaemic activity of some 2-aminomethyl-3-aryl-5,6,7,8-tetrahydrobenzo(b) / 5,6-dimethylthieno (2,3-d)-pyrimidin-4-ones." Arzneimittelforschung. 46(10):981-5.
- Geissler WM et al. (1994) "Male pseudohermaphroditism caused by mutations of testicular 17beta-hydroxysteroid dehydrogenase 3." Nat Genet, 7:34-9.
- JP48042271 B
- JP62132884
- Kapustina MV, Kharizomenova A, Shvedov VI, Fomina AN, Nikolaeva IS, Golovanova EM, Bogdanova GA, Alekseeva LM (1990) "Synthesis and antiviral activity of 7-oxo- and 7-hydroxy-4,5,6,7-tetrahydrobenzo[b]-thiophene derivatives" Chem. Heterocycl. Compd. (Engl. Transl.) 24:1269-271.
- Kapustina, M.V, Nikolaeva, I.S., Kharizomenova, I.A., Shvedov, V.I., Pushkina, T.V., Fomina, A. N. (1992) Pharm. Chem. J. 789.
- Kapustina, M.V., Kharizomenova, I.A., Shvedov, V.I., (1991) Chem. Heterocycl. Compd. (Engl. Trans.) 425.
- Kharizomenova A, Kapustina MV, Grinev AN, Sheinker YN, Alekseeva LM, Kuleshova EF (1984) "Synthesis and structure of derivatives of 7-oxo-4,5,6,7-tetrahydrobenzo [b]-thiophene and 7-hydroxy [b]thiophene" Chem. Heterocycl. Compd. (Engl. Transl.) 20:1339-1342.
- Koeller S & Lellouche JP (1999) "Preparation of Formate Esters from O-TBDMS/O-TES Protected Alcohols. A One-Step Conversion Using the Vilsmeier-Haack Complex POCI3/DMF" Tetrahedron Letters 40(38):7043-7046.
- Koffman B, Modarress KJ, Beckerman T, Bashirelahi N. (1991) "Evidence for involvement of tyrosine in estradiol binding by rat uterus estrogen receptor." J Steroid Biochem Mol Biol. 38(2):135-9.
- Labrie et al. (2000) "Role of 17 beta-hydroxysteroid dehydrogenases in sex steroid formation in peripheral intracrine tissues" Trends Endocrinol Metab., 11:421-7
- Labrie F et al. (1997) "The key role of 17 beta-hydroxysteroid dehydrogenases in sex steroid biology." Steroids, 62: 148-58
- Lidström P, Tierney J, Wathey B, Westman J (2001) "Microwave assisted organic synthesis - a review" Tetrahedron 57(45):9225-9283.
- Lipshutz BH, Wilhelm RS, Kozlowski JA (1984) "Conjugate addition reactions of α,β-unsaturated ketones with higher order, mixed organocuprate reagents, R2Cu(CN)Li2" J. Org. Chem. 49:3938-3942.
- Manhas MS, Sharma SD, Amin SG. (1972) "Heterocyclic compounds. 4. Synthesis and antiinflammatory activity of some substituted thienopyrimidinones." J Med Chem. 15(1):106-7.
- Manjunath KS, Mohan S, Naragund LV, Shishoo CJ. (1997) "Synthesis and evaluation of 2-chloromethyl-3-N-substituted arylthieno(2,3-d)pyrimidin-4-ones and derivatives for central nervous system depressant activity." Arzneimittelforschung. 47(9):1005-8.
- Mitra RB & Joshi VS (1988) "A novel synthesis of ketoprofen, important non-steroidal antiinflammatory agent" Synth. Comm.:18:2259.
- Oefelein MG & Comum R (2000) "Failure to achieve castrate levels of testosterone during luteinizing hormone releasing hormone agonist therapy: the case for monitoring serum testosterone and a treatment decision algorithm." J Urol.; 164:726-9.
- Perrissin M, Duc CL, Narcisse G, Bakri-Logeais F, Huguet F (1980) "Tétrahydro-4,5,6,7 benzo-[b] et tétrahydro-5,6,7,8 (4H)-cyclohepta[b]thiophene; Eur. J. Med. Chem. Chim. Ther. 15:413-418.

- Poirier D. (2003) "Inhibitors of 17 beta-hydroxysteroid dehydrogenases" Curr Med Chem. 10:453-77
- Tamaya et al. (1985) "Comparison of cellular levels of steroid receptors in uterine leiomyoma and myometrium." Acta Obstet Gynecol Scand., 64:307-9
- Tani M, Ariyasu T, Ohtsuka M, Koga T, Ogawa Y, Yokoyama Y, Murakami Y (1996) "New Strategy for indole synthesis from ethyl pyrrole-2-carboxylate (Synthetic Studies on Indoles and Related Compounds. XXXIX)" Chem. Pharm. Bull. 44:55-61.
- US 2003/0170292
- US 5,597,823
- WO 00/07996
- WO 01/42181
- WO 02/26706
- WO 2004/085457
- WO 98/30556
- WO 98/32724
- WO 99/12540


## Claims

**1.** A compound of formula (I)

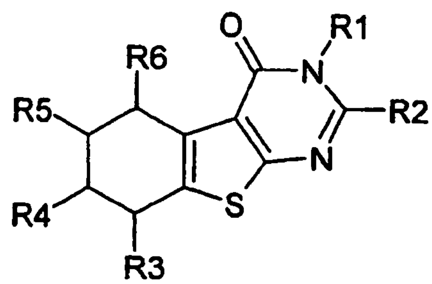

(I)

wherein

R1 and R2 are individually selected from the group consisting of

(i) -$C_1$-$C_{12}$-alkyl, which alkyl is linear, cyclic, branched or partially unsaturated, which is optionally substituted with one, two or three substituents individually selected from the group consisting of hydroxyl, $C_1$-$C_{12}$-alkoxy, thiol, $C_1$-$C_{12}$-alkylthio, aryloxy, arylacyl, -CO-OR, -O-CO-R, heteroaryl-acyloxy, and -$N(R)_2$;
wherein the aryl moiety is phenyl or naphthyl, and is optionally substituted with one, two or three halogen;
wherein the heteroaryl moiety is thienyl, furyl or pyridinyl
(ii) aryl and aryl-$C_1$-$C_{12}$-alkyl,
whereby the alkyl moiety is optionally substituted with one or two hydroxyl groups, and
whereby the aryl moiety is optionally substituted with one to five substituents individually selected from the group consisting of halogen, hydroxyl, $C_1$-$C_{12}$-alkoxy, nitro, nitrile, $C_1$-$C_{12}$-alkyl, halogenated $C_1$-$C_{12}$-alkyl, -$SO_2$-$N(R)_2$, $C_1$-$C_{12}$-alkylsulphonyl;
or which aryl may be optionally substituted by two groups which are attached to adjacent carbon atoms and are combined into a saturated cyclic 5, 6 or 7-membered ring system, optionally containing one, two or three heteroatoms, such as N or O, the number of N atoms being 0-3 and the number of O atoms each being 0-2,
whereby the cyclic ring system may optionally be further substituted by an oxo group;
(iii) heteroaryl and heteroaryl-$C_1$-$C_{12}$-alkyl,
whereby the heteroaryl group is optionally substituted with one, two or three substituents individually selected from the group consisting of halogen, $C_1$-$C_{12}$-alkyl, halogenated $C_1$-$C_8$-alkyl, -CO-OR, aryl or aryloxy,
whereby the aryl group is selected from phenyl or naphthyl and is optionally substituted with one, two or three halogen atoms;
(iv) cycloheteroalkyl and cycloheteroalkyl-$C_1$-$C_8$-alkyl,

whereby the cycloheteroalkyl moiety is selected from the group consisting of pyrrolidinyl, tetrahydrofuryl, tetrahydrothiophenyl, tetrahydropyridinyl, dioxolyl, azetidinyl, thiazolidinyl, oxazolidinyl, piperidinyl, morpholinyl, thiomorpholinyl, piperazinyl, azepanyl, diazepanyl, oxazepanyl, thiazepanyl, dihydro-1H-pyrrolyl, and 1,3-dihydro-benzoimidazolyl;
whereby the cycloheteroalkyl moiety is optionally substituted with up to two substituents individually selected from the group consisting of oxo, $C_1$-$C_{12}$-alkyl, hydroxyl, $C_1$-$C_{12}$-alkoxy and aryl-$C_1$-$C_{12}$-alkyl;

or R2 itself is independently selected from -CO-R, -CO-O-R, or -CO-N(R)$_2$;
whereby R1 and R2 cannot be simultaneously unsubstituted alkyl;
R3 and R4 are individually selected from the group consisting of hydrogen, oxo, thio, halogen or dihalogen, -CO-R, preferably CHO, -CO-O-R, nitrile, -CO-N(R)$_2$, -O-CO-R, -O-R, -S-R, -N(R)$_2$, -$C_1$-$C_{12}$-alkyl, which alkyl is linear, cyclic, branched or partially unsaturated, and which alkyl is optionally substituted with one, two or three substituents individually selected from the group consisting of hydroxyl, $C_1$-$C_{12}$-alkoxy, thiol, and -N(R)$_2$;
R5 and R6 are individually selected from the group consisting of
hydrogen, halogen, -O-R, -CO-O-R, -CO-R,
$C_1$-$C_{12}$-alkyl, which alkyl is linear, cyclic, branched or partially unsaturated, and which alkyl is optionally substituted with one, two or three substituents individually selected from the group consisting of hydroxyl, $C_1$-$C_{12}$-alkoxy, thiol, $C_1$-$C_{12}$-alkylthio, and - CO-OR; and
aryl and aryl-$C_1$-$C_{12}$-alkyl, whereby the aryl moiety is optionally substituted with one to five substituents individually selected from the group consisting of halogen, hydroxyl, $C_1$-$C_{12}$-alkoxy, nitro, nitrile, $C_1$-$C_{12}$-alkyl, halogenated $C_1$-$C_{12}$-alkyl,
whereby R5 and R6 cannot be simultaneously hydrogen, and
whereby R6 is different from halogen, if R5 represents hydrogen;

wherein R represents hydrogen, $C_1$-$C_{12}$-alkyl or phenyl, the phenyl group being optionally substituted with one, two or three substituents selected from the group consisting of halogen, hydroxyl, and $C_1$-$C_4$-alkoxy; and
the hydrocarbon chain -C(R3)-C(R4)-C(R5)-C(R6)- of the six-membered ring is saturated or contains one or two double bonds between the carbon atoms,
or a pharmaceutically acceptable salt thereof.

**2.** A compound of formula (I) according to claim 1, wherein the six-membered ring comprising the hydrocarbon chain -C(R3)-C(R4)-C(R5)-C(R6)- is an aromatic ring.

**3.** A compound of formula (I) according to claim 1, wherein the six-membered ring comprising the hydrocarbon chain -C(R3)-C(R4)-C(R5)-C(R6)- is other than an aromatic ring.

**4.** A compound of formula (I) according to any of the preceding claims 1 to 3, wherein R2 is selected from the group consisting of

(i) -$C_1$-$C_8$-alkyl, which alkyl is linear, cyclic, branched or partially unsaturated,
which is optionally substituted with one, two or three substituents individually selected from the group consisting of hydroxyl, $C_1$-$C_8$-alkoxy, thiol, $C_1$-$C_8$-alkylthio, aryloxy, - CO-O-$C_1$-$C_8$-alkyl, and -O-CO-R';
whereby said aryl group is phenyl or naphthyl, and is optionally substituted with one, two or three halogen atoms;
(ii) aryl and aryl-$C_1$-$C_8$-alkyl,
whereby the aryl moiety is optionally substituted with one to five substituents individually selected from the group consisting of halogen, hydroxyl, $C_1$-$C_8$-alkoxy, nitro, nitrile, halogenated $C_1$-$C_8$-alkyl, -$SO_2$-N(R')$_2$,
(iii) heteroaryl and heteroaryl-$C_1$-$C_8$-alkyl,
whereby the heteroaryl group is optionally substituted with one, two or three substituents individually selected from the group consisting of halogen, $C_1$-$C_8$-alkyl, halogenated $C_1$-$C_8$-alkyl, aryl or aryloxy,
whereby the aryl group is selected from phenyl or naphthyl and is optionally substituted with one, two or three halogen atoms;
(iv) -CO-R',
(v) -CO-N(R')$_2$, and
(vi) -CO-O-R';

wherein R' represents hydrogen or $C_1$-$C_8$-alkyl.

**5.** A compound of formula (I) according to claim 4, wherein R2 is

(a) a residue of formula (II)

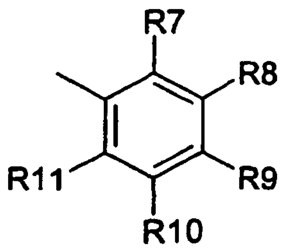

(II)

wherein

R7 is hydrogen, halogen, hydroxyl or $C_1$-$C_4$-alkoxy,
R8 is hydrogen, $C_1$-$C_4$-alkoxy, hydroxyl, nitrile, halogen, or halogenated $C_1$-$C_4$-alkyl,
R9 is hydrogen, $C_1$-$C_4$-alkoxy, hydroxyl, nitrile, halogen, or N,N-di-$C_1$-$C_4$-alkyl-sulphonamide
R10 is hydrogen, $C_1$-$C_4$-alkoxy, hydroxyl, nitrile, halogen, or halogenated $C_1$-$C_4$-alkyl, and
R11 is hydrogen, halogen, hydroxyl or $C_1$-$C_4$-alkoxy;

or (b)

(i) -$C_1$-$C_8$-alkyl, which alkyl is linear, cyclic, branched or partially unsaturated;
(ii) -$C_1$-$C_4$-alkyl, substituted with one or two substituents selected from the group consisting of
-CO-O-R";
-O-R";
-O-Ar, whereby Ar is phenyl optionally substituted with halogen;
-O-CO-R",
phenyl or biphenyl, optionally substituted in the phenyl moiety with one, two or three $C_1$-$C_4$-alkoxy groups;
(iii) -CO-O-R",
(iv) -CO-R",
(v) naphthyl,
(vi) heteroaryl,
whereby the heteroaryl group is optionally substituted by one or two substituents individually selected from the group consisting of halogen, $C_1$-$C_4$-alkyl, halogenated $C_1$-$C_4$-alkyl, phenyl and phenoxy,
whereby the phenyl group is optionally substituted with one, two or three halogen;

wherein R" represents hydrogen or $C_1$-$C_4$-alkyl.

**6.** A compound of formula (I) according to claim 5, wherein R2 is

(i) -$C_3$-$C_8$-alkyl, which alkyl is linear, cyclic or branched,
(ii) -$C_1$-$C_4$-alkyl, optionally substituted with one or two substituents independently selected from the group consisting of $C_1$-$C_4$-alkoxy and hydroxyl,
(iii) phenyl-$C_1$-$C_4$-alkyl,
wherein the phenyl group is optionally substituted with one or two $C_1$-$C_4$-alkoxy groups,
(iv) heteroaryl,
which heteroaryl moiety is selected from the group consisting of furyl, pyridinyl, thienyl, thiazolyl and pyrimidinyl,
(v) a residue of formula (II)

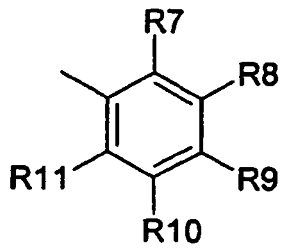

(II)

wherein

R7 is hydrogen, $C_1$-$C_4$-alkoxy or halogen,
R8 is hydrogen, halogen, $C_1$-$C_4$-alkoxy, or hydroxyl,
R9 is hydrogen, hydroxyl or $C_1$-$C_4$-alkoxy,
R10 is hydrogen, halogen, $C_1$-$C_4$-alkoxy, or hydroxyl, and
R11 is hydrogen, $C_1$-$C_4$-alkoxy or halogen.

**7.** A compound of formula (I) according to any of the preceding claims 1 to 6, wherein R1 is selected from the group consisting of

(i) -$C_1$-$C_8$-alkyl, which alkyl is linear, cyclic, branched or partially unsaturated,
which is optionally substituted with one, two or three substituents individually selected from the group consisting of hydroxyl, $C_1$-$C_8$-alkoxy, thiol, -$NH_2$, $C_1$-$C_8$-alkylthio, aryloxy, arylacyl, -CO-O-$C_1$-$C_8$-alkyl, $C_1$-$C_8$-alkylacyloxy, heteroaryl-acyloxy, and $C_1$-$C_8$-alkylamino;
whereby said aryl group is phenyl or naphthyl, and is optionally substituted with one, two or three halogen;
whereby said heteroaryl group is thienyl, furyl or pyridinyl,
(ii) aryl and aryl-$C_1$-$C_8$-alkyl,
wherein the alkyl moiety is optionally substituted with one or two hydroxyl groups, and
wherein the aryl moiety is optionally substituted with one to five substituents individually selected from the group consisting of halogen, hydroxyl, $C_1$-$C_8$-alkoxy, $C_1$-$C_8$-alkylsulphonyl, -$SO_2$-N($C_1$-$C_8$-alkyl)$_2$, $C_1$-$C_8$-alkyl, halogenated $C_1$-$C_8$-alkyl;
or which aryl may be optionally substituted by two groups which are attached to adjacent carbon atoms and are combined into a saturated cyclic 5 or 6-membered ring system, optionally containing one, two or three heteroatoms, such as N or O, the number of N atoms being 0-3 and the number of O atoms each being 0-2, whereby the cyclic ring system may optionally be further substituted by an oxo group;
(iii) heteroaryl and heteroaryl-$C_1$-$C_8$-alkyl,
whereby the heteroaryl group is optionally substituted with one, two or three substituents individually selected from the group consisting of halogen, $C_1$-$C_8$-alkyl, and -CO-O-$C_1$-$C_8$-alkyl;
(iv) cycloheteroalkyl and cycloheteroalkyl-$C_1$-$C_8$-alkyl,
whereby the cycloheteroalkyl moiety is optionally substituted with up to two substituents individually selected from the group consisting of oxo, $C_1$-$C_8$-alkyl, hydroxyl, $C_1$-$C_8$-alkoxy and aryl-$C_1$-$C_8$-alkyl.

**8.** A compound of formula (I) according to claim 7, wherein R1 is selected from the group consisting of

(i) -$C_1$-$C_8$-alkyl, which alkyl is linear, cyclic or branched,
(ii) -$C_1$-$C_4$-alkyl, substituted with one or two substituents independently selected from the group consisting of -O-R"; -O-Ar, -O-CO-HetAr, -CO-Ar, -CO-O-R", and -N(R")$_2$,
(iii) aryl and aryl-$C_1$-$C_4$-alkyl,
wherein the alkyl moiety is optionally substituted with a hydroxyl group; and
wherein the aryl moiety is optionally substituted with one, two or three substituents individually selected from the group consisting of halogen, -O-R"; -$SO_2$-R", -$SO_2$-N(R")$_2$,
or which aryl may be optionally substituted by two groups which are attached to adjacent carbon atoms and are combined into a saturated cyclic 5 or 6 membered ring system, optionally containing up to two O atoms, whereby the cyclic ring system may optionally be further substituted by an oxo group;
(iv) heteroaryl and heteroaryl-$C_1$-$C_4$-alkyl,
whereby the heteroaryl group is optionally substituted with one or two substituents individually selected from the group consisting of halogen, -$C_1$-$C_4$-alkyl, and -CO-O-R";

(v) cycloheteroalkyl and cycloheteroalkyl-$C_1$-$C_4$-alkyl,
whereby the cycloheteroalkyl moiety is optionally substituted with up to two substituents individually selected from the group consisting of oxo, $C_1$-$C_4$-alkyl, preferably methyl, and -$C_1$-$C_4$-alkyl-Ar,

wherein

Ar represent phenyl, optionally substituted with halogen or $C_1$-$C_4$-alkoxy,
HetAr represents thienyl, furyl, or pyridinyl, and
R" represents hydrogen or $C_1$-$C_4$-alkyl.

**9.** A compound of formula (I) according to claim 8, wherein R1 is selected from the group consisting of

(i) -$C_1$-$C_4$-alkyl, optionally substituted with one or two hydroxy groups,
(ii) phenyl-$C_1$-$C_4$-alkyl,
wherein the phenyl group is optionally substituted with one or two $C_1$-$C_4$-alkoxy groups, or wherein the phenyl group is substituted by two groups which are attached to adjacent carbon atoms and are combined into a saturated cyclic 5 or 6-membered ring system, optionally containing one or two O-atoms;
(iii) heteroaryl or heteroaryl-$C_1$-$C_4$-alkyl,
which heteroaryl moiety is selected from the group consisting of furyl, pyridinyl, thienyl, thiazolyl and pyrimidinyl, wherein the heteroaryl group is optionally substituted with $C_1$-$C_4$-alkyl, and
(iv) cycloheteroalkyl-$C_1$-$C_4$-alkyl;
which cycloheteroalkyl moiety is selected from the group consisting of tetrahydrofuryl, piperidinyl, morpholinyl, and pyrrolidinyl.

**10.** A compound of formula (I) according to any of the preceding claims 1 to 3, wherein R1 and R2 are independently selected from the group consisting of:

(i) $C_3$-$C_8$-alkyl, which alkyl is linear, cyclic or branched,
(ii) -$C_1$-$C_4$-alkyl, optionally substituted with one or two substituents independently selected from the group consisting of $C_1$-$C_4$-alkoxy and hydroxyl,
(iii) phenyl or phenyl-$C_1$-$C_4$-alkyl,
wherein the phenyl group is optionally substituted with one to five substituents individually selected from the group consisting of halogen, $C_1$-$C_4$-alkoxy, and hydroxyl,
or wherein the phenyl group is substituted by two groups which are attached to adjacent carbon atoms and are combined into a saturated cyclic 5 or 6-membered ring system, optionally containing one or two heteroatoms, such as N or O;
(iv) heteroaryl or heteroaryl-$C_1$-$C_4$-alkyl,
wherein the heteroaryl group is optionally substituted with $C_1$-$C_4$-alkyl, and
(v) cycloheteroalkyl-$C_1$-$C_4$-alkyl.

**11.** A compound of formula (I) according to any of the preceding claims 1 to 10, wherein R3 is selected from the group consisting of
hydrogen, oxo, -O-R', -O-Ar, -O-CO-R', halogen, thio, -S-R', and -S-Ar, wherein
R' represents hydrogen or $C_1$-$C_4$-alkyl, and
Ar represents phenyl, optionally substituted with one or more substituents selected from the group consisting of halogen, hydroxyl or $C_1$-$C_4$-alkoxy.

**12.** A compound of formula (I) according to claim 11, wherein R3 is selected from the group consisting of hydrogen, oxo, and hydroxyl.

**13.** A compound of formula (I) according to any of the preceding claims 1 to 12, wherein R4 is selected from the group consisting of
hydrogen, $C_1$-$C_4$-alkyl, optionally substituted with hydroxyl; -CO-R', -CO-O-R', halogen and dihalogen,
wherein R' represents hydrogen or $C_1$-$C_4$-alkyl.

**14.** A compound of formula (I) according to claim 13, wherein R4 is selected from the group consisting of hydrogen and halogen.

**15.** A compound of formula (I) according to any of the preceding claims 1 to 14, wherein R5 is selected from the group consisting of hydrogen, -COOR', phenyl-$C_1$-$C_4$-alkyl, $C_1$-$C_4$-alkyl, and $C_1$-$C_4$-alkyl substituted with -COOR', wherein R' represents H or $C_1$-$C_4$-alkyl.

**16.** A compound of formula (I) according to claim 15, wherein R5 is selected from the group consisting of hydrogen, benzyl and $C_1$-$C_4$-alkyl.

**17.** A compound of formula (I) according to any of the preceding claims 1 to 16, wherein R6 is selected from the group consisting of hydrogen, halogen, -O-R', phenyl-$C_1$-$C_4$-alkyl, $C_1$-$C_4$-alkyl, and $C_1$-$C_4$-alkyl substituted with -COOR', wherein R' represents H or $C_1$-$C_4$-alkyl.

**18.** A compound of formula (I) according to claim 17, wherein R6 is selected from the group consisting of hydrogen, halogen, benzyl, and $C_1$-$C_4$-alkyl.

**19.** A compound of formula (I) according to claim 1,

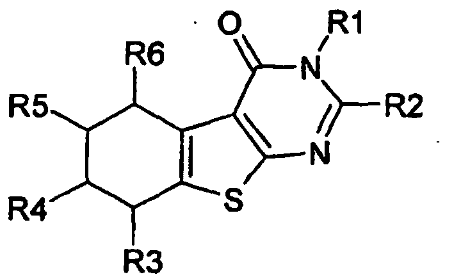

**(I)**

wherein

R1 and R2 are independently selected from the group consisting of:

(i) -$C_3$-$C_8$-alkyl, which alkyl is linear, cyclic or branched,
(ii) -$C_1$-$C_4$-alkyl, optionally substituted with one or two substituents independently selected from the group consisting of $C_1$-$C_4$-alkoxy and hydroxyl,
(iii) phenyl or phenyl-$C_1$-$C_4$-alkyl,
wherein the phenyl group is optionally substituted with one to five substituents individually selected from the group consisting of halogen, $C_1$-$C_4$-alkoxy, and hydroxyl,
or wherein the phenyl group is substituted by two groups which are attached to adjacent carbon atoms and are combined into a saturated cyclic 5 or 6-membered ring system, optionally containing one or two heteroatoms, such as N or O;
(iv) heteroaryl or heteroaryl-$C_1$-$C_4$-alkyl,
wherein the heteroaryl group is optionally substituted with $C_1$-$C_4$-alkyl, and
(v) cycloheteroalkyl-$C_1$-$C_4$-alkyl;

R3 is selected from the group consisting of hydrogen, oxo, and hydroxyl;
R4 is selected from the group consisting of hydrogen and halogen,
R5 is selected from the group consisting of hydrogen, phenyl-$C_1$-$C_2$-alkyl, $C_1$-$C_4$-alkyl and -$C_1$-$C_4$-alkyl-CO-O-$C_1$-$C_4$-alkyl; and
R6 is selected from the group consisting of hydrogen, halogen, phenyl-$C_1$-$C_2$-alkyl, $C_1$-$C_4$-alkyl and -$C_1$-$C_4$-alkyl-CO-O-$C_1$-$C_4$-alkyl.

**20.** A compound of formula (I) according to claim 19, wherein

R1 is selected from the group consisting of

(i) -$C_1$-$C_4$-alkyl, optionally substituted with one or two hydroxy-groups,
(ii) phenyl-$C_1$-$C_4$-alkyl,
wherein the phenyl group is optionally substituted with one or two $C_1$-$C_4$-alkoxy groups,

or wherein the phenyl group is substituted by two groups which are attached to adjacent carbon atoms and are combined into a saturated cyclic 5 or 6-membered ring system, optionally containing one or two O-atoms;
(iii) heteroaryl or heteroaryl-$C_1$-$C_4$-alkyl,
which heteroaryl moiety is selected from the group consisting of furyl, pyridinyl, thienyl, thiazolyl and pyrimidinyl,
wherein the heteroaryl group is optionally substituted with $C_1$-$C_4$-alkyl, and
(iv) cycloheteroalkyl-$C_1$-$C_4$-alkyl;
which cycloheteroalkyl moiety is selected from the group consisting of tetrahydrofuryl, piperidinyl, morpholinyl, and pyrrolidinyl; and

R2 is selected from the group consisting of

(i) $C_3$-$C_8$-alkyl, which alkyl is linear, cyclic or branched,
(ii) -$C_1$-$C_4$-alkyl, optionally substituted with one or two substituents independently selected from the group consisting of $C_1$-$C_4$-alkoxy and hydroxyl,
(iii) phenyl-$C_1$-$C_4$-alkyl,
wherein the phenyl group is optionally substituted with one or two $C_1$-$C_4$-alkoxy groups,
(iv) heteroaryl,
which heteroaryl moiety is selected from the group consisting of furyl, pyridinyl, thienyl, thiazolyl and pyrimidinyl,
(v) a residue of formula (II)

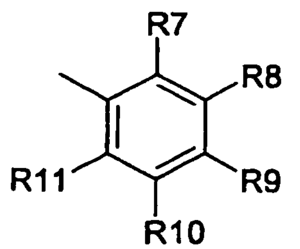

(II)

wherein

R7 is hydrogen, halogen or $C_1$-$C_4$-alkoxy,
R8 is hydrogen, halogen, $C_1$-$C_4$-alkoxy, or hydroxyl,
R9 is hydrogen, hydroxyl or $C_1$-$C_4$-alkoxy,
R10 is hydrogen, halogen, $C_1$-$C_4$-alkoxy, or hydroxyl, and
R11 is hydrogen, halogen or $C_1$-$C_4$-alkoxy.

**21.** A compound of formula (I) according to claim 1 selected from the group consisting of:

3-Benzyl-5-methyl-2-(3,4,5-trimethoxyphenyl)-5,6,7,8-tetrahydro-3H-benzo[4,5]thieno[2,3-d]pyrimidin-4-one,
3-Benzyl-5-methyl-2-(3,4,5-trimethoxyphenyl)-6,7-dihydro-3H,5H-benzo[4,5]thieno[2,3-d]pyrimidin-4,8-dione,
3-Benzyl-7-bromo-2-(2-bromo-3,4,5-trimethoxyphenyl)-5-methyl-6,7-dihydro-3H,5H-benzo[4,5]thieno[2,3-d]pyrimidine-4,8-dione,
3-Benzyl-2-(2-bromo-3,4,5-trimethoxyphenyl)-8-hydroxy-5-methyl-3H-benzo[4,5]thieno[2,3-d]pyrimidin-4-one,
3-Benzyl-6-methyl-2-(3,4,5-trimethoxyphenyl)-5,6,7,8-tetrahydro-3H-benzo[4,5]thieno[2,3-d]pyrimidin-4-one,
3-Benzyl-6-methyl-2-(3,4,5-trimethoxyphenyl)-6,7-dihydro-3H,5H-benzo[4,5]thieno[2,3-d]pyrimidin4,8-dione,
3-Benzyl-7-bromo-6-methyl-2-(2-bromo-3,4,5-trimethoxyphenyl)-6,7-dihydro-3H-benzo[4,5]thieno[2,3-d]pyrimidin-4,8-dione,
3-Benzyl-2-(2-bromo-3,4,5-trimethoxyphenyl)-8-hydroxy-6-methyl-3H-benzo[4,5]thieno[2,3-d]pyrimidin-4-one,
3-Butyl-6-methyl-2-(3,4,5-trimethoxyphenyl)-5,6,7,8-tetrahydro-3H-benzo[4,5]thieno[2,3-d]pyrimidin-4-one,
3-Butyl-6-methyl-2-(3,4,5-trimethoxyphenyl)-6,7-dihydro-3H,5H-benzo[4,5]thierio[2,3-d]pyrimidin-4,8-dione,
3-Butyl-7-bromo-6-methyl-2-(2-bromo-3,4,5-trimethoxyphenyl)-6,7-dihydro-3H-benzo[4,5]thieno[2,3-d]pyrimidin-4,8-dione,

3-Butyl-2-(2-bromo-3,4,5-trimethoxyphenyl)-8-hydroxy-6-methyl-3H-benzo[4,5]thieno[2,3-d]pyrimidin-4-one,
2-(2-Bromo-3-hydroxy-4,5-dimethoxyphenyl)-3-butyl-8-hydroxy-6-methyl-3H-benzo[4,5]thieno[2,3-d]pyrimidin-4-one,
2-(2-Bromo-3,4-dihydroxy-5-methoxyphenyl)-3-butyl-8-hydroxy-6-methyl-3H-benzo[4,5]thieno[2,3-d]pyrimidin-4-one,
7-bromo-3-butyl-2-(2-bromo-3,4,5-trimethoxyphenyl)-8-hydroxy-6-methyl-3H-benzo[4,5]thieno[2,3-d]pyrimidin-4-one, and
7-Bromo-2-(2-bromo-3-hydroxy-4,5-dimethoxyphenyl)-3-butyl-8-hydroxy-6-methyl-3H-benzo[4,5]thieno[2,3-d]pyrimidin-4-one,

or a pharmaceutically acceptable salt thereof.

**22.** A compound of formula (I) according to any of the preceding claims 1 to 21 for use as a medicament.

**23.** Use of a compound of formula (I) as defined in any of the preceding claims 1 to 21 for the manufacture of a medicament for the treatment and/or prevention of a steroid hormone dependent disease or disorder.

**24.** Use according to claim 23, wherein the steroid hormone dependent disease or disorder is a disease or disorder requiring the inhibition of a 17β-hydroxysteroid dehydrogenase enzyme, preferably of the 17β-HSD type 1, 17β-HSD type 2 or 17β-HSD type 3.

**25.** Use according to claim 23, wherein the steroid hormone dependent disease or disorder is selected from the group consisting of breast cancer, prostate carcinoma, ovarian cancer, uterine cancer, endometrial cancer and endometrial hyperplasia, endometriosis, uterine fibroids, uterine leiomyoma, adenomyosis, dysmenorrhea, menorrhagia, metrorrhagia, prostadynia, benign prostatic hyperplasia, prostatitis, acne, seborrhea, hirsutism, androgenic alopecia, precocious puberty, adrenal hyperplasia, polycystic ovarian syndrome, urinary dysfunction, osteoporosis, multiple sclerosis, rheumatoid arthritis, Alzheimer's disease, colon cancer, tissue wounds, skin wrinkles and cataracts.

**26.** A pharmaceutical composition comprising as active agent at least one of the compounds of formula (I) as defined in any of the preceding claims 1 to 21, and at least a pharmaceutically acceptable carrier.

## Patentansprüche

**1.** Verbindung der Formel (I)

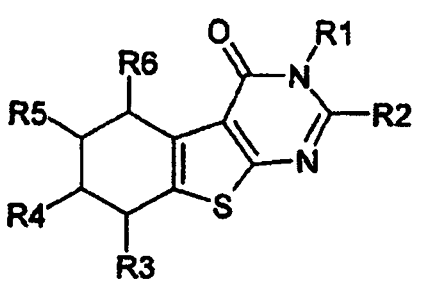

(I)

worin

R1 und R2 individuell aus der Gruppe bestehend aus

(i) $-C_1-C_{12}$-Alkyl, das linear, cyclisch, verzweigt oder teilweise ungesättigt ist und gegebenenfalls durch einen, zwei oder drei individuell aus der Gruppe bestehend aus Hydroxyl, $C_1-C_{12}$-Alkoxy, Thiol, $C_1-C_{12}$-Alkylthio, Aryloxy, Arylacyl, -CO-OR, -O-CO-R, Heteroaryl-acyloxy und $-N(R)_2$ ausgewählte Substituenten substituiert ist;

wobei die Arylgruppierung Phenyl oder Naphthyl ist und gegebenenfalls durch ein, zwei oder drei Halogene substituiert ist;
wobei die Heteroarylgruppierung Thienyl, Furyl oder Pyridinyl ist;
(ii) Aryl und Aryl-$C_1$-$C_{12}$-alkyl,
wobei die Alkylgruppierung gegebenenfalls durch eine oder zwei Hydroxylgruppen substituiert ist und wobei die Arylgruppierung gegebenenfalls durch einen bis fünf individuell aus der Gruppe bestehend aus Halogen, Hydroxyl, $C_1$-$C_{12}$-Alkoxy, Nitro, Nitril, $C_1$-$C_{12}$-Alkyl, halogeniertem $C_1$-$C_{12}$-Alkyl, -$SO_2$-$N(R)_2$ und $C_1$-$C_{12}$-Alkylsulfonyl ausgewählte Substituenten substituiert ist;
oder das Aryl gegebenenfalls durch zwei Gruppen substituiert sein kann, die an benachbarte Kohlenstoffatome gebunden sind und zusammengenommen ein gesättigtes cyclisches 5-, 6- oder 7-gliedriges Ringsystem bilden, das gegebenenfalls ein, zwei oder drei Heteroatome, wie N oder O, enthält, wobei die Zahl der N-Atome 0-3 beträgt und die Zahl der O-Atome jeweils 0-2 beträgt,
wobei das cyclische Ringsystem gegebenenfalls ferner durch eine Oxogruppe substituiert sein kann;
(iii) Heteroaryl und Heteroaryl-$C_1$-$C_{12}$-alkyl,
wobei die Heteroarylgruppe gegebenenfalls durch einen, zwei oder drei individuell aus der Gruppe bestehend aus Halogen, $C_1$-$C_{12}$-Alkyl, halogeniertem $C_1$-$C_8$-Alkyl, -CO-OR, Aryl oder Aryloxy ausgewählte Substituenten substituiert ist,
wobei die Arylgruppe unter Phenyl oder Naphthyl ausgewählt ist und gegebenenfalls durch ein, zwei oder drei Halogenatome substituiert ist;
(iv) Cycloheteroalkyl und Cycloheteroalkyl-$C_1$-$C_8$-alkyl,
wobei die Cycloheteroalkylgruppierung aus der Gruppe bestehend aus Pyrrolidinyl, Tetrahydrofuryl, Tetrahydrothiophenyl, Tetrahydropyridinyl, Dioxolyl, Azetidinyl, Thiazolidinyl, Oxazolidinyl, Piperidinyl, Morpholinyl, Thiomorpholinyl, Piperazinyl, Azepanyl, Diazepanyl, Oxazepanyl, Thiazepanyl, Dihydro-1H-pyrrolyl und 1,3-Dihydrobenzoimidazolyl ausgewählt ist;
wobei die Cycloheteroalkylgruppierung gegebenenfalls durch bis zu zwei individuell aus der Gruppe bestehend aus Oxo, $C_1$-$C_{12}$-Alkyl, Hydroxyl, $C_1$-$C_{12}$-Alkoxy und Aryl-$C_1$-$C_{12}$-alkyl ausgewählte Substituenten substituiert ist;

ausgewählt sind
oder R2 selbst unabhängig unter -CO-R, -CO-O-R oder -CO-$N(R)_2$ ausgewählt ist;
wobei R1 und R2 nicht gleichzeitig für unsubstituiertes Alkyl stehen können;
R3 und R4 individuell aus der Gruppe bestehend aus Wasserstoff, Oxo, Thio, Halogen oder Dihalogen, -CO-R, vorzugsweise CHO, -CO-O-R, Nitril, -CO-$N(R)_2$, -O-CO-R, -O-R, -S-R, -$N(R)_2$, $C_1$-$C_{12}$-Alkyl, das linear, cyclisch, verzweigt oder teilweise ungesättigt ist und gegebenenfalls durch einen, zwei oder drei individuell aus der Gruppe bestehend aus Hydroxyl, $C_1$-$C_{12}$-Alkoxy, Thiol und -$N(R)_2$ ausgewählte Substituenten substituiert ist, ausgewählt sind;
R5 und R6 individuell aus der Gruppe bestehend aus Wasserstoff, Halogen, -O-R, -CO-O-R, -CO-R, $C_1$-$C_{12}$-Alkyl, das linear, cyclisch, verzweigt oder teilweise ungesättigt ist und gegebenenfalls durch einen, zwei oder drei individuell aus der Gruppe bestehend aus Hydroxyl, $C_1$-$C_{12}$-Alkoxy, Thiol, $C_1$-$C_{12}$-Alkylthio und -CO-OR ausgewählte Substituenten substituiert ist; und
Aryl und Aryl-$C_1$-$C_{12}$-alkyl, wobei die Arylgruppierung gegebenenfalls durch einen bis fünf individuell aus der Gruppe bestehend aus Halogen, Hydroxyl, $C_1$-$C_{12}$-Alkoxy, Nitro, Nitril, $C_1$-$C_{12}$-Alkyl und halogeniertem $C_1$-$C_{12}$-Alkyl ausgewählte Substituenten substituiert ist,
ausgewählt sind,
wobei R5 und R6 nicht gleichzeitig für Wasserstoff stehen können und
wobei R6 von Halogen verschieden ist, wenn R5 für Wasserstoff steht;

wobei R für Wasserstoff, $C_1$-$C_{12}$-Alkyl oder Phenyl steht, wobei die Phenylgruppe gegebenenfalls durch einen, zwei oder drei aus der Gruppe bestehend aus Halogen, Hydroxyl und $C_1$-$C_4$-Alkoxy ausgewählte Substituenten substituiert ist und
die Kohlenwasserstoffkette -C(R3)-C(R4)-C(R5)-C(R6)- des sechsgliedrigen Rings gesättigt ist oder eine oder zwei Doppelbindungen zwischen Kohlenstoffatomen enthält,
oder ein pharmazeutisch verträgliches Salz davon.

**2.** Verbindung der Formel (I) nach Anspruch 1, worin es sich bei dem sechsgliedrigen Ring mit der Kohlenwasserstoffkette -C(R3)-C(R4)-C(R5)-C(R6)-um einen aromatischen Ring handelt.

**3.** Verbindung der Formel (I) nach Anspruch 1, worin es sich bei dem sechsgliedrigen Ring mit der Kohlenwasserstoffkette -C(R3)-C(R4)-C(R5)-C(R6)-nicht um einen aromatischen Ring handelt.

**4.** Verbindung der Formel (I) nach einem der vorhergehenden Ansprüche 1 bis 3, worin R2 aus der Gruppe bestehend aus

(i) -$C_1$-$C_8$-Alkyl, das linear, cyclisch, verzweigt oder teilweise ungesättigt ist und gegebenenfalls durch einen, zwei oder drei individuell aus der Gruppe bestehend aus Hydroxyl, $C_1$-$C_8$-Alkoxy, Thiol, $C_1$-$C_8$-Alkylthio, Aryloxy, -CO-O-$C_1$-$C_8$-Alkyl und -O-CO-R' ausgewählte Substituenten substituiert ist;
wobei die Arylgruppe Phenyl oder Naphthyl ist und gegebenenfalls durch ein, zwei oder drei Halogenatome substituiert ist;
(ii) Aryl und Aryl-$C_1$-$C_8$-alkyl,
wobei die Arylgruppierung gegebenenfalls durch einen bis fünf individuell aus der Gruppe bestehend aus Halogen, Hydroxyl, $C_1$-$C_8$-Alkoxy, Nitro, Nitril, halogeniertem $C_1$-$C_8$-Alkyl und -$SO_2$-N(R')$_2$ ausgewählte Substituenten substituiert ist;
(iii) Heteroaryl und Heteroaryl-$C_1$-$C_8$-alkyl,
wobei die Heteroarylgruppe gegebenenfalls durch einen, zwei oder drei individuell aus der Gruppe bestehend aus Halogen, $C_1$-$C_8$-Alkyl, halogeniertem $C_1$-$C_8$-Alkyl, Aryl oder Aryloxy ausgewählte Substituenten substituiert ist,
wobei die Arylgruppe unter Phenyl oder Naphthyl ausgewählt ist und gegebenenfalls durch ein, zwei oder drei Halogenatome substituiert ist;
(iv) -CO-R',
(v) -CO-N(R')$_2$ und
(vi) -CO-O-R'

ausgewählt ist;
wobei R' für Wasserstoff oder $C_1$-$C_8$-Alkyl steht.

**5.** Verbindung der Formel (I) nach Anspruch 4, worin R2 für

(a) einen Rest der Formel (II)

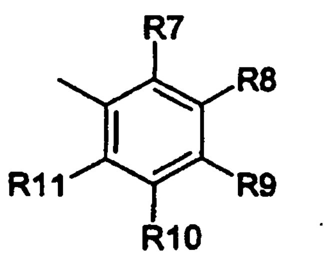

(II)

worin

R7 Wasserstoff, Halogen, Hydroxyl oder $C_1$-$C_4$-Alkoxy bedeutet,
R8 Wasserstoff, $C_1$-$C_4$-Alkoxy, Hydroxyl, Nitril, Halogen oder halogeniertes $C_1$-$C_4$-Alkyl bedeutet,
R9 Wasserstoff, $C_1$-$C_4$-Alkoxy, Hydroxyl, Nitril, Halogen oder N,N-Di-$C_1$-$C_4$-alkylsulfonamid bedeutet,
R10 Wasserstoff, $C_1$-$C_4$-Alkoxy, Hydroxyl, Nitril, Halogen oder halogeniertes $C_1$-$C_4$-Alkyl bedeutet und
R11 Wasserstoff, Halogen, Hydroxyl oder $C_1$-$C_4$-Alkoxy bedeutet;

oder (b)

(i) -$C_1$-$C_8$-Alkyl, das linear, cyclisch, verzweigt oder teilweise ungesättigt ist;
(ii) -$C_1$-$C_4$-Alkyl, das durch einen oder zwei aus der Gruppe bestehend aus
-CO-O-R'';
-O-R'';
-O-Ar, wobei Ar gegebenenfalls durch Halogen substituiertes Phenyl bedeutet;

-O-CO-R'' und
Phenyl oder Biphenyl, das gegebenenfalls in der Phenylgruppierung durch eine, zwei oder drei $C_1$-$C_4$-Alkoxygruppen substituiert ist; ausgewählte Substituenten substituiert ist;
(iii) -CO-O-R'',
(iv) -CO-R'',
(v) Naphthyl,
(vi) Heteroaryl,

wobei die Heteroarylgruppe gegebenenfalls durch einen oder zwei individuell aus der Gruppe bestehend aus Halogen, $C_1$-$C_4$-Alkyl, halogeniertem $C_1$-$C_4$-Alkyl, Phenyl und Phenoxy ausgewählte Subtituenten substituiert ist,
wobei die Phenylgruppe gegebenenfalls durch ein, zwei oder drei Halogene substituiert ist;

steht;
wobei R'' für Wasserstoff oder $C_1$-$C_4$-Alkyl steht.

**6.** Verbindung der Formel (I) nach Anspruch 5, worin R2 für

(i) -$C_3$-$C_8$-Alkyl, das linear, cyclisch oder verzweigt unabhängig ist;
(ii) -$C_1$-$C_4$-Alkyl, das gegebenenfalls durch einen oder zwei aus der Gruppe bestehend aus $C_1$-$C_4$-Alkoxy und Hydroxyl ausgewählte Substituenten substituiert ist;
(iii) Phenyl-$C_1$-$C_4$-alkyl,
wobei die Phenylgruppe gegebenenfalls durch eine oder zwei $C_1$-$C_4$-Alkoxygruppen substituiert ist;
(iv) Heteroaryl,
wobei die Heteroarylgruppierung aus der Gruppe bestehend aus Furyl, Pyridinyl, Thienyl, Thiazolyl und Pyrimidinyl ausgewählt ist,
(v) einen Rest der Formel (II)

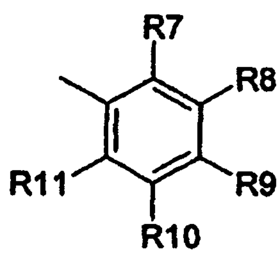

(II)

worin

R7 Wasserstoff, $C_1$-$C_4$-Alkoxy oder Halogen bedeutet,
R8 Wasserstoff, Halogen, $C_1$-$C_4$-Alkoxy oder Hydroxyl bedeutet,
R9 Wasserstoff, Hydroxyl oder $C_1$-$C_4$-Alkoxy bedeutet,
R10 Wasserstoff, Halogen, $C_1$-$C_4$-Alkoxy oder Hydroxyl bedeutet und
R11 Wasserstoff, $C_1$-$C_4$-Alkoxy oder Halogen, bedeutet;

steht.

**7.** Verbindung der Formel (I) nach einem der vorhergehenden Ansprüche 1 bis 6, worin R1 aus der Gruppe bestehend aus

(i) -$C_1$-$C_8$-Alkyl, das linear, cyclisch, verzweigt oder teilweise ungesättigt ist und gegebenenfalls durch einen, zwei oder drei individuell aus der Gruppe bestehend aus Hydroxyl, $C_1$-$C_8$-Alkoxy, Thiol, -$NH_2$, $C_1$-$C_8$-Alkylthio, Aryloxy, Arylacyl, -CO-O-$C_1$-$C_8$-Alkyl, $C_1$-$C_8$-Alkylacyloxy, Heteroaryl-acyloxy und $C_1$-$C_8$-Alkylamino ausgewählte Substituenten substituiert ist;
wobei die Arylgruppe Phenyl oder Naphthyl ist und gegebenenfalls durch ein, zwei oder drei Halogene substituiert ist;
wobei die Heteroarylgruppe Thienyl, Furyl oder Pyridinyl ist;
(ii) Aryl und Aryl-$C_1$-$C_8$-alkyl,
wobei die Alkylgruppierung gegebenenfalls durch eine oder zwei Hydroxylgruppen substituiert ist und
wobei die Arylgruppierung gegebenenfalls durch einen bis fünf individuell aus der Gruppe bestehend aus Halogen, Hydroxyl, $C_1$-$C_8$-Alkoxy, $C_1$-$C_8$-Alkylsulfonyl, -$SO_2$-N($C_1$-$C_8$-Alkyl)$_2$, $C_1$-$C_8$-Alkyl und halogeniertem $C_1$-$C_8$-Alkyl ausgewählte Substituenten substituiert ist;
oder das Aryl gegebenenfalls durch zwei Gruppen substituiert sein kann, die an benachbarte Kohlenstoffatome gebunden sind und zusammengenommen ein gesättigtes cyclisches 5- oder 6- gliedriges Ringsystem bilden, das gegebenenfalls ein, zwei oder drei Heteroatome, wie N oder O, enthält,
wobei die Zahl der N-Atome 0-3 beträgt und die Zahl der O-Atome jeweils 0-2 beträgt,
wobei das cyclische Ringsystem gegebenenfalls ferner durch eine Oxogruppe substituiert sein kann;
(iii) Heteroaryl und Heteroaryl-$C_1$-$C_8$-alkyl,
wobei die Heteroarylgruppe gegebenenfalls durch einen, zwei oder drei individuell aus der Gruppe bestehend aus Halogen, $C_1$-$C_8$-Alkyl und -CO-O-$C_1$-$C_8$-Alkyl ausgewählte Substituenten substituiert ist,
(iv) Cycloheteroalkyl und Cycloheteroalkyl-$C_1$-$C_8$-alkyl,
wobei die Cycloheteroalkylgruppierung gegebenenfalls durch bis zu zwei individuell aus der Gruppe bestehend aus Oxo, $C_1$-$C_8$-Alkyl, Hydroxyl, $C_1$-$C_8$-Alkoxy und Aryl-$C_1$-$C_8$-alkyl ausgewählte Substituenten substituiert ist;

ausgewählt ist.

**8.** Verbindung der Formel (I) nach Anspruch 7, worin R1 aus der Gruppe bestehend aus

(i) -$C_1$-$C_8$-Alkyl, das linear, cyclisch oder verzweigt ist,
(ii) -$C_1$-$C_4$-Alkyl, das durch einen oder zwei unabhängig aus der Gruppe bestehend aus -O-R", -O-Ar, -O-CO-HetAr, -CO-Ar, -CO-O-R'' und -N(R'')$_2$ ausgewählte Substituenten substituiert ist,
(iii) Aryl und Aryl-$C_1$-$C_4$-alkyl,
wobei die Alkylgruppierung gegebenenfalls durch eine Hydroxylgruppe substituiert ist und
wobei die Arylgruppierung gegebenenfalls durch einen, zwei oder drei individuell aus der Gruppe bestehend aus Halogen, -O-R'', -$SO_2$-R'' und -$SO_2$-N(R'')$_2$ ausgewählte Substituenten substituiert ist;
oder das Aryl gegebenenfalls durch zwei Gruppen substituiert sein kann, die an benachbarte Kohlenstoffatome gebunden sind und zusammengenommen ein gesättigtes cyclisches 5- oder 6-gliedriges Ringsystem bilden, das gegebenenfalls bis zu zwei O-Atome enthält,
wobei das cyclische Ringsystem gegebenenfalls ferner durch eine Oxogruppe substituiert sein kann;
(iv) Heteroaryl und Heteroaryl-$C_1$-$C_4$-alkyl,
wobei die Heteroarylgruppe gegebenenfalls durch einen oder zwei individuell aus der Gruppe bestehend aus Halogen, $C_1$-$C_4$-Alkyl und -CO-O-R'' ausgewählte Substituenten substituiert ist,
(v) Cycloheteroalkyl und Cycloheteroalkyl-$C_1$-$C_4$-alkyl, wobei die Cycloheteroalkylgruppierung gegebenenfalls durch bis zu zwei individuell aus der Gruppe bestehend aus Oxo, $C_1$-$C_4$-Alkyl, vorzugsweise Methyl, und $C_1$-$C_4$-Alkyl- Ar ausgewählte Substituenten substituiert ist,

ausgewählt ist,
wobei

Ar für gegebenenfalls durch Halogen oder $C_1$-$C_4$-Alkoxy substituiertes Phenyl steht,
HetAr für Thienyl, Furyl oder Pyridinyl steht und
R'' für Wasserstoff oder $C_{1-4}$-Alkyl steht.

**9.** Verbindung der Formel (I) nach Anspruch 8, worin R1 aus der Gruppe bestehend aus

(i) -$C_1$-$C_4$-Alkyl, gegebenenfalls durch eine oder zwei Hydroxylgruppen substituiert ist,
(ii) Phenyl-$C_1$-$C_4$-alkyl,
wobei die Phenylgruppe gegebenenfalls durch eine oder zwei $C_1$-$C_4$-Alkoxygruppen substituiert ist,
oder die Phenylgruppe durch zwei Gruppen substituiert ist, die an benachbarte Kohlenstoffatome gebunden sind und zusammengenommen ein gesättigtes cyclisches 5- oder 6-gliedriges Ringsystem bilden, das gegebenenfalls ein oder zwei O-Atome enthält,
(iii) Heteroaryl oder Heteroaryl-$C_1$-$C_4$-alkyl,
wobei die Heteroarylgruppierung aus der Gruppe bestehend aus Furyl, Pyridinyl, Thienyl, Thiazolyl und Pyrimidinyl ausgewählt ist,
wobei die Heteroarylgruppe gegebenenfalls durch $C_1$-$C_4$-Alkyl substituiert ist, und
(iv) Cycloheteroalkyl-$C_1$-$C_4$-alkyl,
wobei die Cycloheteroalkylgruppierung aus der Gruppe bestehend aus Tetrahydrofuryl, Piperidinyl, Morpholinyl und Pyrrolidinyl ausgewählt ist,

ausgewählt ist.

**10.** Verbindung der Formel (I) nach einem der vorhergehenden Ansprüche 1 bis 3, worin R1 und R2 unabhängig aus der Gruppe bestehend aus

(i) $C_3$-$C_8$-Alkyl, das linear, cyclisch oder verzweigt ist;
(ii) -$C_1$-$C_4$-Alkyl, das gegebenenfalls durch einen oder zwei unabhängig aus der Gruppe bestehend aus $C_1$-$C_4$-Alkoxy und Hydroxyl ausgewählte Substituenten substituiert ist;
(iii) Phenyl oder Phenyl-$C_1$-$C_4$-alkyl,
wobei die Phenylgruppe gegebenenfalls durch einen bis fünf individuell aus der Gruppe bestehend aus Halogen, $C_1$-$C_4$-Alkoxy und Hydroxyl ausgewählte Substituenten substituiert ist
oder die Phenylgruppe durch zwei Gruppen substituiert ist, die an benachbarte Kohlenstoffatome gebunden sind und zusammengenommen ein gesättigtes cyclisches 5- oder 6-gliedriges Ringsystem bilden, das gegebenenfalls ein oder zwei Heteroatome, wie N oder O, enthält;
(iv) Heteroaryl oder Heteroaryl-$C_1$-$C_4$-alkyl,
wobei die Heteroarylgruppe gegebenenfalls durch $C_1$-$C_4$-Alkyl substituiert ist, und
(v) Cycloheteroalkyl-$C_1$-$C_4$-alkyl

ausgewählt sind.

**11.** Verbindung der Formel (I) nach einem der vorhergehenden Ansprüche 1 bis 10, worin R3 aus der Gruppe bestehend aus
Wasserstoff, Oxo, -O-R', -O-Ar, -O-CO-R', Halogen, Thio, -S-R' und -S-Ar
ausgewählt ist, wobei
R' für Wasserstoff oder $C_1$-$C_4$-Alkyl steht und
Ar für gegebenenfalls durch einen oder mehrere Substituenten aus der Gruppe bestehend aus Halogen, Hydroxyl oder $C_1$-$C_4$-Alkoxy substituiertes Phenyl steht.

**12.** Verbindung der Formel (I) nach Anspruch 11, worin R3 aus der Gruppe bestehend aus Wasserstoff, Oxo und Hydroxyl ausgewählt ist.

**13.** Verbindung der Formel (I) nach einem der vorhergehenden Ansprüche 1 bis 12, worin R4 aus der Gruppe bestehend aus
Wasserstoff, gegebenenfalls durch Hydroxyl substituiertem $C_1$-$C_4$-Alkyl, -CO-R', -CO-O-R', Halogen und Dihalogen ausgewählt ist,
wobei R' für Wasserstoff oder $C_1$-$C_4$-Alkyl steht.

**14.** Verbindung der Formel (I) nach Anspruch 13, worin R4 aus der Gruppe bestehend aus Wasserstoff und Halogen ausgewählt ist.

**15.** Verbindung der Formel (I) nach einem der vorhergehenden Ansprüche 1 bis 14, worin R5 aus der Gruppe bestehend aus Wasserstoff, -COOR', Phenyl-$C_1$-$C_4$-alkyl, $C_1$-$C_4$-Alkyl und durch -COOR' substituiertem $C_1$-$C_4$-Alkyl ausgewählt ist, wobei R' für H oder $C_1$-$C_4$-Alkyl steht.

**16.** Verbindung der Formel (I) nach Anspruch 15, worin R5 aus der Gruppe bestehend aus Wasserstoff, Benzyl und $C_1$-$C_4$-Alkyl ausgewählt ist.

**17.** Verbindung der Formel (I) nach einem der vorhergehenden Ansprüche 1 bis 16, worin R6 aus der Gruppe bestehend aus Wasserstoff, Halogen, -O-R', Phenyl-$C_1$-$C_4$-alkyl, $C_1$-$C_4$-Alkyl und durch -COOR' substituiertem $C_1$-$C_4$-Alkyl ausgewählt ist, wobei R' für H oder $C_1$-$C_4$-Alkyl steht.

**18.** Verbindung der Formel (I) nach Anspruch 17, worin R6 aus der Gruppe bestehend aus Wasserstoff, Halogen,

Benzyl und $C_1$-$C_4$-Alkyl ausgewählt ist.

**19.** Verbindung der Formel (I) nach Anspruch 1

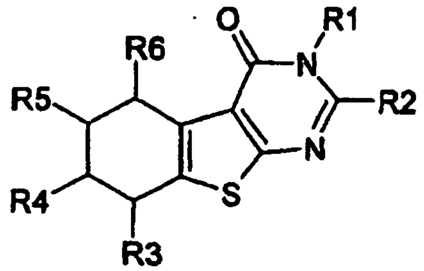

(I)

worin

R1 und R2 unabhängig aus der Gruppe bestehend aus

(i) -$C_3$-$C_8$-Alkyl, das linear, cyclisch oder verzweigt ist;
(ii) -$C_1$-$C_4$-Alkyl, das gegebenenfalls durch einen oder zwei unabhängig aus der Gruppe bestehend aus $C_1$-$C_4$-Alkoxy und Hydroxyl ausgewählte Substituenten substituiert ist;
(iii) Phenyl oder Phenyl-$C_1$-$C_4$-alkyl,
wobei die Phenylgruppe gegebenenfalls durch einen bis fünf individuell aus der Gruppe bestehend aus Halogen, $C_1$-$C_4$-Alkoxy und Hydroxyl ausgewählte Substituenten substituiert ist
oder die Phenylgruppe durch zwei Gruppen substituiert ist, die an benachbarte Kohlenstoffatome gebunden sind und zusammengenommen ein gesättigtes cyclisches 5- oder 6-gliedriges Ringsystem bilden, das gegebenenfalls ein oder zwei Heteroatome, wie N oder O, enthält;
(iv) Heteroaryl oder Heteroaryl-$C_1$-$C_4$-alkyl,
wobei die Heteroarylgruppe gegebenenfalls durch $C_1$-$C_4$-Alkyl substituiert ist, und
(v) Cycloheteroalkyl-$C_1$-$C_4$-alkyl ausgewählt sind;

R3 aus der Gruppe bestehend aus Wasserstoff, Oxo und Hydroxyl ausgewählt ist;
R4 aus der Gruppe bestehend aus Wasserstoff und Halogen ausgewählt ist;
R5 aus der Gruppe bestehend aus Wasserstoff, Phenyl-$C_1$-$C_2$-alkyl, $C_1$-$C_4$-Alkyl und -$C_1$-$C_4$-Alkyl-CO-O-$C_1$-$C_4$-alkyl ausgewählt ist und
R6 aus der Gruppe bestehend aus Wasserstoff, Halogen, Phenyl-$C_1$-$C_2$-alkyl, $C_1$-$C_4$-Alkyl und -$C_1$-$C_4$-Alkyl-CO-O-$C_1$-$C_4$-alkyl ausgewählt ist.

**20.** Verbindung der Formel (I) nach Anspruch 19, worin

R1 aus der Gruppe bestehend aus

(i) -$C_1$-$C_4$-Alkyl, gegebenenfalls durch eine oder zwei Hydroxylgruppen substituiert ist,
(ii) Phenyl-$C_1$-$C_4$-alkyl,
wobei die Phenylgruppe gegebenenfalls durch eine oder zwei $C_1$-$C_4$-Alkoxygruppen substituiert ist,
oder die Phenylgruppe durch zwei Gruppen substituiert ist, die an benachbarte Kohlenstoffatome gebunden sind und zusammengenommen ein gesättigtes cyclisches 5- oder 6-gliedriges Ringsystem bilden, das gegebenenfalls ein oder zwei O-Atome enthält,
(iii) Heteroaryl oder Heteroaryl-$C_1$-$C_4$-alkyl,
wobei die Heteroarylgruppierung aus der Gruppe bestehend aus Furyl, Pyridinyl, Thienyl, Thiazolyl und Pyrimidinyl ausgewählt ist,
wobei die Heteroarylgruppe gegebenenfalls durch $C_1$-$C_4$-Alkyl substituiert ist, und
(iv) Cycloheteroalkyl-$C_1$-$C_4$-alkyl,
wobei die Cycloheteroalkylgruppierung aus der Gruppe bestehend aus Tetrahydrofuryl, Piperidinyl, Morpholinyl und Pyrrolidinyl ausgewählt ist,

ausgewählt ist und
R2 aus der Gruppe bestehend aus

(i) $C_3$-$C_8$-Alkyl, das linear, cyclisch oder verzweigt ist;
(ii) -$C_1$-$C_4$-Alkyl, das gegebenenfalls durch einen oder zwei aus der Gruppe bestehend aus $C_1$-$C_4$-Alkoxy und Hydroxyl ausgewählte Substituenten substituiert ist;
(iii) Phenyl-$C_1$-$C_4$-alkyl,
wobei die Phenylgruppe gegebenenfalls durch eine oder zwei $C_1$-$C_4$-Alkoxygruppen substituiert ist;
(iv) Heteroaryl,
wobei die Heteroarylgruppierung aus der Gruppe bestehend aus Furyl, Pyridinyl, Thienyl, Thiazolyl und Pyrimidinyl ausgewählt ist,
(v) einem Rest der Formel (II)

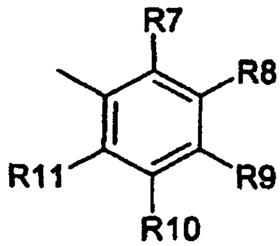

(II)

worin

R7 Wasserstoff, Halogen oder $C_1$-$C_4$-Alkoxy bedeutet,
R8 Wasserstoff, Halogen, $C_1$-$C_4$-Alkoxy oder Hydroxyl bedeutet,
R9 Wasserstoff, Hydroxyl oder $C_1$-$C_4$-Alkoxy bedeutet,
R10 Wasserstoff, Halogen, $C_1$-$C_4$-Alkoxy oder Hydroxyl bedeutet und
R11 Wasserstoff, Halogen oder $C_1$-$C_4$-Alkoxy bedeutet;

ausgewählt ist.

**21.** Verbindung der Formel (I) nach Anspruch 1, ausgewählt aus der Gruppe bestehend aus:

3-Benzyl-5-methyl-2-(3,4,5-trimethoxyphenyl)-5,6,7,8-tetrahydro-3H-benzo[4,5]thieno[2,3-d]-pyrimidin-4-on,
3-Benzyl-5-methyl-2-(3,4,5-trimethoxyphenyl)-6,7-dihydro-3H,5H-benzo[4,5]thieno[2,3-d]pyrimidin-4,8-dion,
3-Benzyl-7-brom-2-(2-brom-3,4,5-trimethoxyphenyl)-5-methyl-6,7-dihydro-3H,5H-benzo[4,5]thieno[2,3-d]pyrimidin-4,8-dion,
3-Benzyl-2-(2-brom-3,4,5-trimethoxyphenyl)-8-hydroxy-5-methyl-3H-benzo[4,5]thieno[2,3-d]-pyrimidin-4-on,
3-Benzyl-6-methyl-2-(3,4,5-trimethoxyphenyl)-5,6,7,8-tetrahydro-3H-benzo[4,5]thieno[2,3-d]-pyrimidin-4-on,
3-Benzyl-6-methyl-2-(3,4,5-trimethoxyphenyl)-6,7-dihydro-3H,5H-benzo[4,5]thieno[2,3-d]pyrimidin-4,8-dion,
3-Benzyl-7-brom-6-methyl-2-(2-brom-3,4,5-trimethoxyphenyl)-6,7-dihydro-3H-benzo[4,5]thieno-[2,3-d]pyrimidin-4,8-dion,
3-Benzyl-2-(2-brom-3,4,5-trimethoxyphenyl)-8-hydroxy-6-methyl-3H-benzo[4,5]thieno[2,3-d]-pyrimidin-4-on,
3-Butyl-6-methyl-2-(3,4,5-trimethoxyphenyl)-5,6,7,8-tetrahydro-3H-benzo[4,5]thieno[2,3-d]-pyrimidin-4-on,
3-Butyl-6-methyl-2-(3,4,5-trimethoxyphenyl)-6,7-dihydro-3H,5H-benzo[4,5]thieno[2,3-d]pyrimidin-4,8-dion,
3-Butyl-7-brom-6-methyl-2-(2-brom-3,4,5-trimethoxyphenyl)-6,7-dihydro-3H-benzo[4,5]thieno-[2,3-d]pyrimidin-4,8-dion,
3-Butyl-2-(2-brom-3,4,5-trimethoxyphenyl)-8-hydroxy-6-methyl-3H-benzo[4,5]thieno[2,3-d]-pyrimidin-4-on,
2-(2-Brom-3-hydroxy-4,5-dimethoxyphenyl)-3-butyl-8-hydroxy-6-methyl-3H-benzo[4,5]thieno[2,3-d]-pyrimidin-4-on,
2-(2-Brom-3,4-dihydroxy-5-methoxyphenyl)-3-butyl-8-hydroxy-6-methyl-3H-benzo[4,5]thieno[2,3-d]-pyrimidin-4-on,
7-Brom-3-butyl-2-(2-brom-3,4,5-trimethoxyphenyl)-8-hydroxy-6-methyl-3H-benzo[4,5]thieno[2,3-d]-pyrimidin-4-on und

7-Brom-2-(2-brom-3-hydroxy-4,5-dimethoxyphenyl)-3-butyl-8-hydroxy-6-methyl-3H-benzo[4,5]thieno-[2,3-d] pyrimidin-4-on,

oder ein pharmazeutisch verträgliches Salz davon.

**22.** Verbindung der Formel (I) nach einem der vorhergehenden Ansprüche 1 bis 21 zur Verwendung als Arzneimittel.

**23.** Verwendung einer Verbindung der Formel (I) gemäß einem der vorhergehenden Ansprüche 1 bis 21 zur Herstellung eines Arzneimittels zur Behandlung und/oder Prävention einer Steroidhormon-abhängigen Erkrankung oder Störung.

**24.** Verwendung nach Anspruch 23, bei der es sich bei der Steroidhormon-abhängigen Erkrankung oder Störung um eine Erkrankung oder Störung handelt, die die Inhibierung eines 17β-Hydroxysteroiddehydrogenaseenzyms, vorzugsweise von 17β-HSD Typ 1, 17β-HSD Typ 2 oder 17β-HSD Typ 3, erfordert.

**25.** Verwendung nach Anspruch 23, bei der die Steroidhormon-abhängige Erkrankung oder Störung aus der Gruppe bestehend aus Brustkrebs, Prostatakarzinom, Eierstockkrebs, Gebärmutterkrebs, endometrialem Krebs und endometrialer Hyperplasie, Endometriose, Gebärmutterfibroleiomyom, Gebärmuttermyom, Adenomyose, Dysmenorrhoe, Menorrhagia, Metrorrhagia, Prostadynia, gutartiger Prostatahyperplasie, Prostatitis, Akne, Seborrhoe, Hirsutismus, androgener Alopecia, Pubertas praecox, Nebennierenhyperplasie, Syndrom der polyzystischen Ovarien, Harndysfunktion, Osteoporose, Multipler Sklerose, rheumatoider Arthritis, Alzheimer-Krankheit, Dickdarmkrebs, Gewebewunden, Hautfalten und Katarakten ausgewählt ist.

**26.** Pharmazeutische Zusammensetzung, die als Wirkstoff mindestens eine der Verbindungen der Formel (I) gemäß einem der vorhergehenden Ansprüche 1 bis 21 und mindestens einen pharmazeutisch verträglichen Träger enthält.

**Revendications**

**1.** Composé de la formule (I)

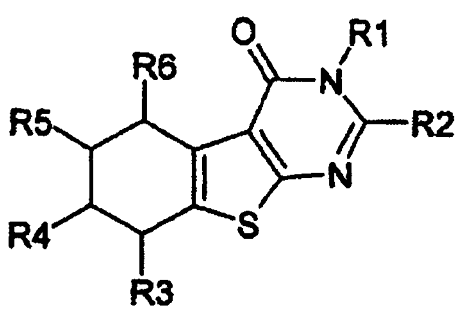

(I)

dans laquelle

R1 et R2 sont individuellement choisis parmi le groupe constitué

(i) d'un groupe alkyle de $C_1$ à $C_{12}$, lequel groupe alkyle est linéaire, cyclique, ramifié ou partiellement insaturé, lequel est éventuellement substitué par un, deux ou trois substituants choisis individuellement parmi le groupe constitué des groupes hydroxyle, alcoxy de $C_1$ à $C_{12}$, thiol, alkylthio de $C_1$ à $C_{12}$, aryloxy, arylacyle, -CO-OR, -O-CO-R, hétéroaryl-acyloxy et - $N(R)_2$ ;
dans lequel le groupement aryle est un groupe phényle ou un groupe naphtyle, et est éventuellement substitué par un, deux ou trois atomes d'halogène ;
dans lequel le groupement hétéroaryle est un groupe thiényle, un groupe furyle ou un groupe pyridinyle ;
(ii) d'un groupe aryle et d'un groupe aryl-alkyle de $C_1$ à $C_{12}$,
dans lequel le groupement alkyle est éventuellement substitué par un ou deux groupes hydroxyle, et par lequel le groupement aryle est éventuellement substitué par un à cinq substituants individuellement choisis

parmi le groupe constitué d'un atome d'halogène, d'un groupe hydroxyle, d'un groupe alcoxy de $C_1$ à $C_{12}$, d'un groupe nitro, d'un groupe nitrile, d'un groupe alkyle de $C_1$ à $C_{12}$, d'un groupe alkyle de $C_1$ à $C_{12}$ halogéné, d'un groupe $-SO_2-N(R)_2$, d'un groupe alkylsulfonyle de $C_1$ à $C_{12}$ ;
ou lequel groupe aryle peut être éventuellement substitué par deux groupes qui sont attachés à des atomes de carbone adjacents et qui sont combinés dans un système cyclique saturé à 5, 6 ou 7 chaînons, comprenant éventuellement un, deux ou trois hétéroatomes, comme un atome d'azote ou un atome d'oxygène, le nombre d'atomes d'azote variant de 0 à 3 et le nombre d'atomes d'oxygène variant chacun de 0 à 2, dans lequel le système cyclique peut éventuellement être en outre substitué par un groupe oxo ;
(iii) d'un groupe hétéroaryle et d'un groupe hétéroaryl-alkyle de $C_1$ à $C_{12}$,
dans lequel le groupe hétéroaryle est éventuellement substitué par un, deux ou trois substituants individuellement choisis parmi le groupe constitué d'un atome d'halogène, d'un groupe alkyle de $C_1$ à $C_{12}$, d'un groupe alkyle de $C_1$ à $C_{12}$ halogéné, d'un groupe -CO-OR, d'un groupe aryle ou d'un groupe aryloxy, dans lequel le groupe aryle est choisi parmi un groupe phényle ou un groupe naphtyle, et est éventuellement substitué par un, deux ou trois atomes d'halogène ;
(iv) d'un groupe cyclohétéroalkyle et d'un groupe cyclohétéroalkyl-alkyle de $C_1$ à $C_8$,

dans lequel le groupement cyclohétéroalkyle est choisi parmi le groupe constitué des groupes pyrrolidinyle, tétrahydrofuryle, tétrahydrothiophényle, tétrahydropyridinyle, dioxolyle, azétidinyle, thiazolidinyle, oxazolidinyle, pipéridinyle, morpholinyle, thiomorpholinyle, pipérazinyle, azépanyle, diazépanyle, oxazépanyle, thiazépanyle, dihydro-1H-pyrrolyle et 1,3-dihydrobenzoimidazolyle ;
dans lequel le groupement cyclohétéroalkyle est éventuellement substitué par jusqu'à deux substituants individuellement choisis parmi le groupe constitué d'un groupe oxo, d'un groupe alkyle de $C_1$ à $C_{12}$, d'un groupe hydroxyle, d'un groupe alcoxy de $C_1$ à $C_{12}$ et d'un groupe aryl-alkyle de $C_1$ à $C_{12}$ ;
ou R2 lui-même est indépendamment choisi parmi les groupes -CO-R, -CO-O-R ou $-CO-N(R)_2$ ;
dans lequel R1 et R2 ne peuvent être simultanément des groupes alkyle non substitués ;
R3 et R4 sont individuellement choisis parmi le groupe constitué d'un atome d'hydrogène, des groupes oxo, thio, d'un atome d'halogène ou d'un dihalogène, des groupes -CO-R, de préférence CHO, -CO-O-R, nitrile, $-CO-N(R)_2$, -O-CO-R, -O-R, -S-R, $-N(R)_2$, alkyle de $C_1$ à $C_{12}$, lequel groupe alkyle est linéaire, cyclique, ramifié ou partiellement insaturé, et lequel groupe alkyle est éventuellement substitué par un, deux ou trois substituants individuellement choisis parmi le groupe constitué des groupes hydroxyle, alcoxy de $C_1$ à $C_{12}$, thiol et $-N(R)_2$ ;
R5 et R6 sont individuellement choisis parmi le groupe constitué
d'un atome d'hydrogène, d'un atome d'halogène, des groupes -O-R, -CO-O-R, -CO-R,
d'un groupe alkyle de $C_1$ à $C_{12}$, lequel groupe alkyle est linéaire, cyclique, ramifié ou partiellement insaturé, et lequel groupe alkyle est éventuellement substitué par un, deux ou trois substituants choisis individuellement parmi le groupe constitué des groupes hydroxyle, alcoxy de $C_1$ à $C_{12}$, thiol, alkylthio de $C_1$ à $C_{12}$, et -CO-OR ; et
d'un groupe aryle et d'un groupe aryl-alkyle de $C_1$ à $C_{12}$, dans lesquels le groupement aryle est éventuellement substitué par un à cinq substituants individuellement choisis parmi le groupe constitué d'un atome d'halogène, des groupes hydroxyle, alcoxy de $C_1$ à $C_{12}$, nitro, nitrile, alkyle de $C_1$ à $C_{12}$, alkyle de $C_1$ à $C_{12}$ halogéné,

dans lequel R5 et R6 ne peuvent être simultanément un atome d'hydrogène, et
dans lequel R6 n'est pas un atome d'halogène, si R5 représente un atome d'hydrogène ;
dans lequel R représente un atome d'hydrogène, un groupe alkyle de $C_1$ à $C_{12}$ ou un groupe phényle, le groupe phényle étant éventuellement substitué par un, deux ou trois substituants choisis parmi le groupe constitué d'un atome d'halogène, d'un groupe hydroxyle et d'un groupe alcoxy de $C_1$ à $C_4$ ; et
la chaîne hydrocarbonée -C(R3)-C(R4)-C(R5)-C(R6)- du cycle à six chaînons est saturée ou contient une ou deux liaisons doubles entre les atomes de carbone,
ou un sel pharmaceutiquement acceptable de celui-ci.

**2.** Composé de la formule (I) selon la revendication 1, dans lequel le cycle à six chaînons comprenant la chaîne hydrocarbonée -C(R3)-C(R4)-C(R5)-C(R6)- est un cycle aromatique.

**3.** Composé de la formule (I) selon la revendication 1, dans lequel le cycle à six chaînons comprenant la chaîne hydrocarbonée -C(R3)-C(R4)-C(R5)-C(R6)- n'est pas un cycle aromatique.

**4.** Composé de la formule (I) selon l'une quelconque des revendications précédentes 1 à 3, dans lequel R2 est choisi parmi le groupe constitué

(i) d'un groupe alkyle de $C_1$ à $C_8$, lequel groupe alkyle est linéaire, cyclique, ramifié ou partiellement insaturé,

lequel est éventuellement substitué par un, deux ou trois substituants individuellement choisis parmi le groupe constitué d'un groupe hydroxyle, d'un groupe alcoxy de $C_1$ à $C_8$, d'un groupe thiol, d'un groupe alkylthio de $C_1$ à $C_8$, d'un groupe aryloxy, d'un groupe CO-O-alkyle de $C_1$ à $C_8$ et d'un groupe -O-CO-R' ;
dans lequel ledit groupe aryle est un groupe phényle ou un groupe naphtyle, et est éventuellement substitué par un, deux ou trois atomes d'halogène ;
(ii) d'un groupe aryle et d'un groupe aryl-alkyle de $C_1$ à $C_8$,
dans lequel le groupement aryle est éventuellement substitué par un à cinq substituants individuellement choisis parmi le groupe constitué d'un atome d'halogène, des groupes hydroxyle, alcoxy de $C_1$ à $C_8$, nitro, nitrile, alkyle de $C_1$ à $C_8$ halogéné, $-SO_2-N(R')_2$,
(iii) d'un groupe hétéroaryle et d'un groupe hétéroaryl-alkyle de $C_1$ à $C_8$,
dans lequel le groupe hétéroaryle est éventuellement substitué par un, deux ou trois substituants individuellement choisis parmi le groupe constitué d'un atome d'halogène, d'un groupe alkyle de $C_1$ à $C_8$, d'un groupe alkyle de $C_1$ à $C_8$ halogéné, d'un groupe aryle ou d'un groupe aryloxy,
dans lequel le groupe aryle est choisi parmi un groupe phényle ou un groupe naphtyle et est éventuellement substitué par un, deux ou trois atomes d'halogène ;
(iv) d'un groupe -CO-R',
(v) d'un groupe $-CO-N(R')_2$ et
(vi) d'un groupe -CO-O-R' ;

dans lequel R' représente un atome d'hydrogène ou un groupe alkyle de $C_1$ à $C_8$.

**5.** Composé de la formule (I) selon la revendication 4, dans lequel R2 est

(a) un résidu de la formule (II)

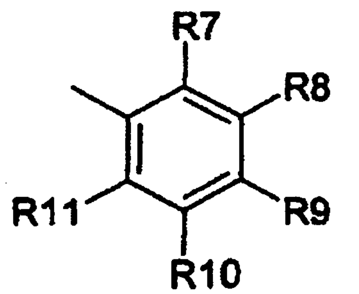

(II)

dans laquelle

R7 représente un atome d'hydrogène, un atome d'halogène, un groupe hydroxyle ou un groupe alcoxy de $C_1$ à $C_4$,
R8 représente un atome d'hydrogène, un groupe alcoxy de $C_1$ à $C_4$, un groupe hydroxyle, un groupe nitrile, un atome d'halogène ou un groupe alkyle de $C_1$ à $C_4$ halogéné,
R9 représente un atome d'hydrogène, un groupe alcoxy de $C_1$ à $C_4$, un groupe hydroxyle, un groupe nitrile, un atome d'halogène ou un groupe N,N-di-alkyle de $C_1$ à $C_4$-sulfonamide,
R10 représente un atome d'hydrogène, un groupe alcoxy de $C_1$ à $C_4$, un groupe hydroxyle, un groupe nitrile, un atome d'halogène ou un groupe alkyle de $C_1$ à $C_4$ halogéné, et R11 représente un atome d'hydrogène, un atome d'halogène, un groupe hydroxyle ou un groupe alcoxy de $C_1$ à $C_4$ ;

ou (b)

(i) un groupe alkyle de $C_1$ à $C_8$, lequel groupe alkyle est linéaire, cyclique, ramifié ou partiellement insaturé ;
(ii) un groupe alkyle de $C_1$ à $C_4$, substitué par un ou deux substituants choisis parmi le groupe constitué d'un groupe -CO-O-R" ;
d'un groupe -O-R" ;
d'un groupe -O-Ar, dans lequel Ar est un groupe phényle éventuellement substitué par un atome d'halogène ;

d'un groupe -O-CO-R",
d'un groupe phényle ou d'un groupe biphényle, éventuellement substitué dans le groupement phényle par un, deux ou trois groupes alcoxy de $C_1$ à $C_4$ ;
(iii) d'un groupe -CO-O-R" ;
(iv) d'un groupe -CO-R" ;
(v) d'un groupe naphtyle,
(vi) d'un groupe hétéroaryle,

dans lequel le groupe hétéroaryle est éventuellement substitué par un ou deux substituants individuellement choisis parmi le groupe constitué d'un atome d'halogène, d'un groupe alkyle de $C_1$ à $C_4$, d'un groupe alkyle de $C_1$ à $C_4$ halogéné, d'un groupe phényle et d'un groupe phénoxy,
dans lequel le groupe phényle est éventuellement substitué par un, deux ou trois atomes d'halogène ;
dans lequel R" représente un atome d'hydrogène ou un groupe alkyle de $C_1$ à $C_4$.

**6.** Composé de la formule (I) selon la revendication 5, dans lequel R2 est

(i) un groupe alkyle de $C_3$ à $C_8$, lequel groupe alkyle est linéaire, cyclique ou ramifié,
(ii) un groupe alkyle de $C_1$ à $C_4$, éventuellement substitué par un ou deux substituants indépendamment choisis parmi le groupe constitué d'un groupe alcoxy de $C_1$ à $C_4$ et d'un groupe hydroxyle,
(iii) un groupe phényl-alkyle de $C_1$ à $C_4$,
dans lequel le groupe phényle est éventuellement substitué par un ou deux groupes alcoxy de $C_1$ à $C_4$,
(iv) un groupe hétéroaryle,
lequel groupement hétéroaryle est choisi parmi le groupe constitué d'un groupe furyle, d'un groupe pyridinyle, d'un groupe thiényle, d'un groupe thiazolyle et d'un groupe pyrimidinyle,
(v) un résidu de la formule (II)

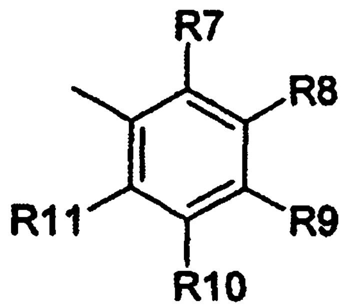

(II)

dans laquelle

R7 représente un atome d'hydrogène, un groupe alcoxy de $C_1$ à $C_4$ ou un atome d'halogène,
R8 représente un atome d'hydrogène, un atome d'halogène, un groupe alcoxy de $C_1$ à $C_4$ ou un groupe hydroxyle,
R9 représente un atome d'hydrogène, un groupe hydroxyle ou un groupe alcoxy de $C_1$ à $C_4$,
R10 représente un atome d'hydrogène, un atome d'halogène, un groupe alcoxy de $C_1$ à $C_4$ ou un groupe hydroxyle, et
R11 représente un atome d'hydrogène, un groupe alcoxy de $C_1$ à $C_4$ ou un atome d'halogène.

**7.** Composé de la formule (I) selon l'une quelconque des revendications précédentes 1 à 6, dans lequel R1 est choisi parmi le groupe constitué

(i) d'un groupe alkyle de $C_1$ à $C_8$, lequel groupe alkyle est linéaire, cyclique, ramifié ou partiellement insaturé, lequel est éventuellement substitué par un, deux ou trois substituants individuellement choisis parmi le groupe constitué des groupes hydroxyle, alcoxy de $C_1$ à $C_8$, thiol, $-NH_2$, alkylthio de $C_1$ à $C_8$, aryloxy, arylacyle, -CO-O-alkyle de $C_1$ à $C_8$, alkyle de $C_1$ à $C_8$-acyloxy, hétéroaryl-acyloxy et alkyle de $C_1$ à $C_8$-amino ;
dans lequel ledit groupe aryle est un groupe phényle ou un groupe naphtyle, et est éventuellement substitué

par un, deux ou trois atomes d'halogène ;
dans lequel ledit groupe hétéroaryle est un groupe thiényle, un groupe furyle ou un groupe pyridinyle,
(ii) d'un groupe aryle et d'un groupe aryl-alkyle de $C_1$ à $C_8$,
dans lequel groupement alkyle est éventuellement substitué par un ou deux groupes hydroxyle, et
dans lequel le groupement aryle est éventuellement substitué par un à cinq substituants individuellement choisis parmi le groupe constitué d'un atome d'halogène, des groupes hydroxyle, alcoxy de $C_1$ à $C_8$, alkyle de $C_1$ à $C_8$-sulfonyle, $-SO_2$-N(alkyle de $C_1$ à $C_8)_2$, alkyle de $C_1$ à $C_8$, alkyle de $C_1$ à $C_8$ halogéné ;
ou lequel groupe aryle peut être éventuellement substitué par deux groupes qui sont attachés à des atomes de carbone adjacents et qui sont combinés dans un système cyclique saturé à 5 ou 6 chaînons, comprenant éventuellement un, deux ou trois hétéroatomes, comme un atome d'azote ou d'oxygène, le nombre d'atomes d'azote variant de 0 à 3 et le nombre d'atomes d'oxygène variant chacun de 0 à 2,
dans lequel le système cyclique saturé peut éventuellement être en outre substitué par un groupe oxo ;
(iii) d'un groupe hétéroaryle et d'un groupe hétéroaryl-alkyle de $C_1$ à $C_8$,
dans lequel le groupe hétéroaryle est éventuellement substitué par un, deux ou trois substituants individuellement choisis parmi le groupe constitué d'un atome d'halogène, d'un groupe alkyle de $C_1$ à $C_8$ et d'un groupe -CO-O-alkyle de $C_1$ à $C_8$ ;
(iv) d'un groupe cyclohétéroalkyle et d'un groupe cyclohétéroalkyl-alkyle de $C_1$ à $C_8$,

dans lequel le groupement cyclohétéroalkyle est éventuellement substitué par jusqu'à deux substituants individuellement choisis parmi le groupe constitué d'un groupe oxo, d'un groupe alkyle de $C_1$ à $C_8$, d'un groupe hydroxyle, d'un groupe alcoxy de $C_1$ à $C_8$ et d'un groupe aryl-alkyle de $C_1$ à $C_8$.

**8.** Composé de la formule (I) selon la revendication 7, dans lequel R1 est choisi parmi le groupe constitué

(i) d'un groupe alkyle de $C_1$ à $C_8$, lequel groupe alkyle est linéaire, cyclique ou ramifié,
(ii) d'un groupe alkyle de $C_1$ à $C_4$, substitué par un ou deux substituants indépendamment choisis parmi le groupe constitué des groupes -O-R", -O-Ar, -O-CO-HetAr, -CO-Ar, -CO-O-R" et -N(R")$_2$,
(iii) d'un groupe aryle et d'un groupe aryl-alkyle de $C_1$ à $C_4$,
dans lequel le groupement alkyle est éventuellement substitué par un groupe hydroxyle ; et
dans lequel le groupement aryle est éventuellement substitué par un, deux ou trois substituants individuellement choisis parmi le groupe constitué d'un atome d'halogène et des groupes -O-R", $-SO_2$-R", $-SO_2$-N(R")$_2$,
ou lequel groupe aryle peut éventuellement être substitué par deux groupes qui sont attachés à des atomes de carbone adjacents et qui sont combinés dans un système cyclique saturé à 5 ou 6 chaînons, comprenant éventuellement jusqu'à deux atomes d'oxygène,
dans lequel le système cyclique peut éventuellement être en outre substitué par un groupe oxo ;
(iv) d'un groupe hétéroaryle et d'un groupe hétéroaryl-alkyle de $C_1$ à $C_4$,
dans lequel le groupe hétéroaryle est éventuellement substitué par un ou deux substituants individuellement choisis parmi le groupe constitué d'un atome d'halogène, d'un groupe alkyle de $C_1$ à $C_4$ et d'un groupe -CO-O-R" ;
(v) d'un groupe cyclohétéroalkyle et d'un groupe cyclohétéroalkyl-alkyle de $C_1$ à $C_4$,

dans lequel le groupement cyclohétéroalkyle est éventuellement substitué par jusqu'à deux substituants individuellement choisis parmi le groupe constitué d'un groupe oxo, d'un groupe alkyle de $C_1$ à $C_4$, de préférence un groupe méthyle, et d'un groupe alkyle de $C_1$ à $C_4$-Ar ;
dans lequel
Ar représente un groupe phényle, éventuellement substitué par un atome d'halogène ou un groupe alcoxy de $C_1$ à $C_4$,
HetAr représente un groupe thiényle, un groupe furyle ou un groupe pyridinyle, et
R" représente un atome d'hydrogène ou un groupe alkyle de $C_1$ à $C_4$.

**9.** Composé de la formule (I) selon la revendication 8, dans lequel R1 est choisi parmi le groupe constitué

(i) d'un groupe alkyle de $C_1$ à $C_4$, éventuellement substitué par un ou deux groupes hydroxy,
(ii) d'un groupe phényl-alkyle de $C_1$ à $C_4$,
dans lequel le groupe phényle est éventuellement substitué par un ou deux groupes alcoxy de $C_1$ à $C_4$,
ou dans lequel le groupe phényle est substitué par deux groupes qui sont attachés à des atomes de carbone adjacents et qui sont combinés dans un système cyclique saturé à 5 ou 6 chaînons, comprenant éventuellement un ou deux atomes d'oxygène ;
(iii) d'un groupe hétéroaryle ou d'un groupe hétéroaryl-alkyle de $C_1$ à $C_4$,
lequel groupement hétéroaryle est choisi parmi le groupe constitué d'un groupe furyle, d'un groupe pyridinyle,

d'un groupe thiényle, d'un groupe thiazolyle et d'un groupe pyrimidinyle,
dans lequel le groupement hétéroaryle est éventuellement substitué par un groupe alkyle de $C_1$ à $C_4$, et
(iv) d'un groupe cyclohétéroalkyl-alkyle de $C_1$ à $C_4$ ;
lequel groupement cyclohétéroalkyle est choisi parmi le groupe constitué d'un groupe tétrahydrofuryle, d'un groupe pipéridinyle, d'un groupe morpholinyle et d'un groupe pyrrolidinyle.

**10.** Composé de la formule (I) selon l'une quelconque des revendications précédentes 1 à 3, dans lequel R1 et R2 sont indépendamment choisis parmi le groupe constitué

(i) d'un groupe alkyle de $C_3$ à $C_8$, lequel groupe alkyle est linéaire, cyclique ou ramifié,
(ii) d'un groupe alkyle de $C_1$ à $C_4$, éventuellement substitué par un ou deux substituants indépendamment choisis parmi le groupe constitué d'un groupe alcoxy de $C_1$ à $C_4$ et d'un groupe hydroxyle,
(iii) d'un groupe phényle ou d'un groupe phényl-alkyle de $C_1$ à $C_4$,
dans lequel le groupe phényle est éventuellement substitué par un à cinq substituants individuellement choisis parmi le groupe constitué d'un atome d'halogène, d'un groupe alcoxy de $C_1$ à $C_4$ et d'un groupe hydroxyle,
ou dans lequel le groupe phényle est substitué par deux groupes qui sont attachés à des atomes de carbone adjacents et qui sont combinés dans un système cyclique saturé à 5 ou 6 chaînons, comprenant éventuellement un ou deux hétéroatomes, comme des atomes d'azote ou d'oxygène ;
(iv) d'un groupe hétéroaryle ou d'un groupe hétéroaryl-alkyle de $C_1$ à $C_4$,
dans lequel le groupe hétéroaryle est éventuellement substitué par un groupe alkyle de $C_1$ à $C_4$, et
(v) d'un groupe cyclohétéroalkyl-alkyle de $C_1$ à $C_4$.

**11.** Composé de la formule (I) selon l'une quelconque des revendications précédentes 1 à 10, dans lequel R3 est choisi parmi le groupe constitué d'un atome d'hydrogène, des groupes oxo, -O-R', -O-Ar, -O-CO-R', d'un atome d'halogène, des groupes thio, -S-R' et -S-Ar,
dans lequel
R' représente un atome d'hydrogène ou un groupe alkyle de $C_1$ à $C_4$, et
Ar représente un groupe phényle, éventuellement substitué par un ou plusieurs substituants choisis parmi le groupe constitué d'un atome d'halogène, d'un groupe hydroxyle ou d'un groupe alcoxy de $C_1$ à $C_4$.

**12.** Composé de la formule (I) selon la revendication 11, dans lequel R3 est choisi parmi le groupe constitué d'un atome d'hydrogène, d'un groupe oxo et d'un groupe hydroxyle.

**13.** Composé de la formule (I) selon l'une quelconque des revendications précédentes 1 à 12, dans lequel R4 est choisi parmi le groupe constitué
d'un atome d'hydrogène, d'un groupe alkyle de $C_1$ à $C_4$, éventuellement substitué par un groupe hydroxyle, un groupe -CO-R', un groupe -CO-O-R', un atome d'halogène et un dihalogène,
dans lequel R' représente un atome d'hydrogène ou un groupe alkyle de $C_1$ à $C_4$.

**14.** Composé de la formule (I) selon la revendication 13, dans lequel R4 est choisi parmi le groupe constitué d'un atome d'hydrogène et d'un atome d'halogène.

**15.** Composé de la formule (I) selon l'une quelconque des revendications précédentes 1 à 14, dans lequel R5 est choisi parmi le groupe constitué d'un atome d'hydrogène, des groupes -COOR', phényl-alkyle de $C_1$ à $C_4$, alkyle de $C_1$ à $C_4$ et alkyle de $C_1$ à $C_4$ substitué par un groupe -COOR', dans lequel R' représente un atome d'hydrogène ou un groupe alkyle de $C_1$ à $C_4$.

**16.** Composé de la formule (I) selon la revendication 15, dans lequel R5 est choisi parmi le groupe constitué d'un atome d'hydrogène, d'un groupe benzyle et d'un groupe alkyle de $C_1$ à $C_4$.

**17.** Composé de la formule (I) selon l'une quelconque des revendications précédentes 1 à 16, dans lequel R6 est choisi parmi le groupe constitué d'un atome d'hydrogène, d'un atome d'halogène, des groupes - O-R', phényl-alkyle de $C_1$ à $C_4$, alkyle de $C_1$ à $C_4$ et alkyle de $C_1$ à $C_4$ substitué par un groupe -COOR', dans lequel R' représente un atome d'hydrogène ou un groupe alkyle de $C_1$ à $C_4$.

**18.** Composé de la formule (I) selon la revendication 17, dans lequel R6 est choisi parmi le groupe constitué d'un atome d'hydrogène, d'un atome d'halogène, d'un groupe benzyle et d'un groupe alkyle de $C_1$ à $C_4$.

**19.** Composé de la formule (I) selon la revendication 1,

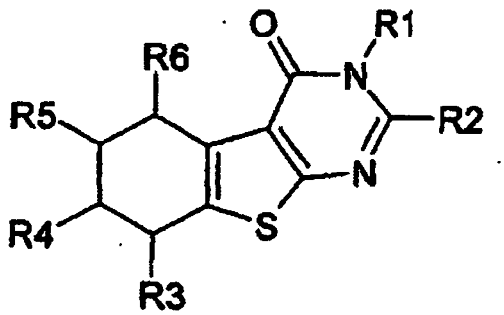

(I)

dans lequel

R1 et R2 sont indépendamment choisis parmi le groupe constitué

(i) d'un groupe alkyle de $C_3$ à $C_8$, lequel groupe alkyle est linéaire, cyclique ou ramifié,
(ii) d'un groupe alkyle de $C_1$ à $C_4$, éventuellement substitué par un ou deux substituants choisis indépendamment parmi le groupe constitué d'un groupe alcoxy de $C_1$ à $C_4$ et d'un groupe hydroxyle,
(iii) d'un groupe phényle ou d'un groupe phényl-alkyle de $C_1$ à $C_4$,
dans lequel le groupe phényle est éventuellement substitué par un à cinq substituants individuellement choisis parmi le groupe constitué d'un atome d'halogène, d'un groupe alcoxy de $C_1$ à $C_4$ et d'un groupe hydroxyle,
ou dans lequel le groupe phényle est substitué par deux groupes qui sont attachés à des atomes de carbone adjacents et qui sont combinés dans un système cyclique saturé à 5 ou 6 chaînons, comprenant éventuellement un ou deux hétéroatomes, comme des atomes d'azote ou d'oxygène ;
(iv) d'un groupe hétéroaryle ou d'un groupe hétéroaryl-alkyle de $C_1$ à $C_4$,
dans lequel le groupe hétéroaryle est éventuellement substitué par un groupe alkyle de $C_1$ à $C_4$, et
(v) d'un groupe cyclohétéroalkyl-alkyle de $C_1$ à $C_4$ ;

R3 est choisi parmi le groupe constitué d'un atome d'hydrogène, d'un groupe oxo et d'un groupe hydroxyle ;
R4 est choisi parmi le groupe constitué d'un atome d'hydrogène et d'un atome d'halogène,
R5 est choisi parmi le groupe constitué d'un atome d'hydrogène, d'un groupe phényl-alkyle de $C_1$ à $C_2$, d'un groupe alkyle de $C_1$ à $C_4$ et d'un groupe alkyle de $C_1$ à $C_4$-CO-O-alkyle de $C_1$ à $C_4$, et
R6 est choisi parmi le groupe constitué d'un atome d'hydrogène, d'un atome d'halogène, d'un groupe phénylalkyle de $C_1$ à $C_2$, d'un groupe alkyle de $C_1$ à $C_4$ et d'un groupe alkyle de $C_1$ à $C_4$-CO-O-alkyle de $C_1$ à $C_4$.

**20.** Composé de la formule (I) selon la revendication 19, dans lequel

R1 est choisi parmi le groupe constitué

(i) d'un groupe alkyle de $C_1$ à $C_4$, éventuellement substitué par un ou deux groupes hydroxy,
(ii) d'un groupe phényl-alkyle de $C_1$ à $C_4$,
dans lequel le groupe phényle est éventuellement substitué par un ou deux groupes alcoxy de $C_1$ à $C_4$,
ou dans lequel le groupe phényle est substitué par deux groupes qui sont attachés à des atomes de carbone adjacents et qui sont combinés dans un système cyclique saturé à 5 ou 6 chaînons, comprenant éventuellement un ou deux atomes d'oxygène ;
(iii) d'un groupe hétéroaryle ou d'un groupe hétéroaryl-alkyle de $C_1$ à $C_4$,
lequel groupement hétéroaryle est choisi parmi le groupe constitué d'un groupe furyle, d'un groupe pyridinyle, d'un groupe thiényle, d'un groupe thiazolyle et d'un groupe pyrimidinyle,
dans lequel le groupe hétéroaryle est éventuellement substitué par un groupe alkyle de $C_1$ à $C_4$, et
(iv) d'un groupe cyclohétéroalkyl-alkyle de $C_1$ à $C_4$ ;
lequel groupement cyclohétéroalkyle est choisi parmi le groupe constitué d'un groupe tétrahydrofuryle, d'un groupe pipéridinyle, d'un groupe morpholinyle et d'un groupe pyrrolidinyle ; et

R2 est choisi parmi le groupe constitué

(i) d'un groupe alkyle de $C_3$ à $C_8$, lequel groupe alkyle est linéaire, cyclique ou ramifié,
(ii) d'un groupe alkyle de $C_1$ à $C_4$, éventuellement substitué par un ou deux substituants indépendamment choisis parmi le groupe constitué d'un groupe alcoxy de $C_1$ à $C_4$ et d'un groupe hydroxyle,
(iii) d'un groupe phényl-alkyle de $C_1$ à $C_4$,
dans lequel le groupe phényle est éventuellement substitué par un ou deux groupes alcoxy de $C_1$ à $C_4$,
(iv) d'un groupe hétéroaryle,
lequel groupement hétéroaryle est choisi parmi le groupe constitué d'un groupe furyle, d'un groupe pyridinyle, d'un groupe thiényle, d'un groupe thiazolyle et d'un groupe pyrimidinyle,
(v) d'un résidu de la formule (II)

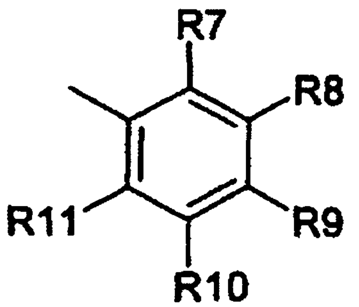

(II)

dans laquelle

R7 représente un atome d'hydrogène, un atome d'halogène ou un groupe alcoxy de $C_1$ à $C_4$,
R8 représente un atome d'hydrogène, un atome d'halogène, un groupe alcoxy de $C_1$ à $C_4$ ou un groupe hydroxyle,
R9 représente un atome d'hydrogène, un groupe hydroxyle ou un groupe alcoxy de $C_1$ à $C_4$,
R10 représente un atome d'hydrogène, un atome d'halogène, un groupe alcoxy de $C_1$ à $C_4$ ou un groupe hydroxyle, et
R11 représente un atome d'hydrogène, un atome d'halogène ou un groupe alcoxy de $C_1$ à $C_4$.

**21.** Composé de la formule (I) selon la revendication 1, choisi parmi le groupe constitué des composés suivants :

3-benzyl-5-méthyl-2-(3,4,5-triméthoxyphényl)-5,6,7,8-tétrahydro-3H-benzo[4,5]thiéno[2,3-d]pyrimidin-4-one,
3-benzyl-5-méthyl-2-(3,4,5-triméthoxyphényl)-6,7-dihydro-3H,5H-benzo[4,5]thiéno[2,3-d]pyrimidine-4,8-dione,
3-benzyl-7-bromo-2-(2-bromo-3,4,5-triméthoxyphényl)-5-méthyl-6,7-dihydro-3H,5H-benzo[4,5]thiéno[2,3-d]pyrimidine-4,8-dione,
3-benzyl-2-(2-bromo-3,4,5-triméthoxyphényl)-8-hydroxy-5-méthyl-3H-benzo[4,5]thiéno[2,3-d]pyrimidin-4-one,
3-benzyl-6-méthyl-2-(3,4,5-triméthoxyphényl)-5,6,7,8-tétrahydro-3H-benzo[4,5]thiéno[2,3-d]pyrimidin-4-one,
3-benzyl-6-méthyl-2-(3,4,5-triméthoxyphényl)-6,7-dihydro-3H,5H-benzo[4,5]thiéno[2,3-d]pyrimidine-4,8-dione,
3-benzyl-7-bromo-6-méthyl-2-(2-bromo-3,4,5-triméthoxyphényl)-6,7-dihydro-3H-benzo[4,5]thiéno[2,3-d]pyrimidine-4,8-dione,
3-benzyl-2-(2-bromo-3,4,5-triméthoxyphényl)-8-hydroxy-6-méthyl-3H-benzo[4,5]thiéno[2,3-d]pyrimidin-4-one,
3-butyl-6-méthyl-2-(3,4,5-triméthoxyphényl)-5,6,7,8-tétrahydro-3H-benzo[4,5]thiéno[2,3-d]pyrimidin-4-one,
3-butyl-6-méthyl-2-(3,4,5-triméthoxyphényl)-6,7-dihydro-3H,5H-benzo[4,5]thiéno[2,3-d]pyrimidine-4,8-dione,
3-butyl-7-bromo-6-méthyl-2-(2-bromo-3,4,5-triméthoxyphényl)-6,7-dihydro-3H-benzo[4,5]thiéno[2,3-d]pyrimidine-4,8-dione,
3-butyl-2-(2-bromo-3,4,5-triméthoxyphényl)-8-hydroxy-6-méthyl-3H-benzo[4,5]thiéno[2,3-d]pyrimidin-4-one,
2-(2-bromo-3-hydroxy-4,5-diméthoxyphényl)-3-butyl-8-hydroxy-6-méthyl-3H-benzo[4,5]thiéno[2,3-d]pyrimidin-4-one,

2-(2-bromo-3,4-dihydroxy-5-méthoxyphényl)-3-butyl-8-hydroxy-6-méthyl-3H-benzo[4,5]thiéno[2,3-d]pyrimidin-4-one,
7-bromo-3-butyl-2-(2-bromo-3,4,5-triméthoxyphényl)-8-hydroxy-6-méthyl-3H-benzo[4,5]thiéno[2,3-d]pyrimidin-4-one, et
7-bromo-2-(2-bromo-3-hydroxy-4,5-diméthoxyphényl)-3-butyl-8-hydroxy-6-méthyl-3H-benzo[4,5]thiéno[2,3-d]pyrimidin-4-one,

ou un sel pharmaceutiquement acceptable de ceux-ci.

**22.** Composé de la formule (I) selon l'une quelconque des revendications précédentes 1 à 21 destiné à être utilisé comme médicament.

**23.** Utilisation d'un composé de la formule (I) tel que défini dans l'une quelconque des revendications précedentes 1 à 21 dans la fabrication d'un médicament pour le traitement et/ou la prévention d'une maladie ou d'un trouble liés à une hormonodépendance aux stéroïdes.

**24.** Utilisation selon la revendication 23, dans laquelle la maladie ou le trouble lié à une hormonodépendance aux stéroïdes est une maladie ou un trouble qui requiert l'inhibition d'une enzyme 17β-hydroxystéroïde déshydrogénase, de préférence la 17β-HSD de type 1, la 17β-HSD de type 2 ou la 17β-HSD de type 3.

**25.** Utilisation selon la revendication 23, dans laquelle la maladie ou le trouble lié à une hormonodépendance aux stéroïdes est choisi(e) parmi le groupe constitué du cancer du sein, du cancer de la prostate, du cancer des ovaires, du cancer de l'utérus, du cancer de l'endomètre et de l'hyperplasie de l'endomètre, de l'endométriose, de fibromes utérins, du fibromyome, de l'adénomyose, de l'algoménorrhée, de la ménorragie, de la métrorragie, de la prostadynie, de l'hypertrophie bénigne de la prostate, de la prostatite, de l'acné, de la séborrhée, de l'hirsutisme, de l'alopécie androgène, de la puberté précoce, de l'hyperplasie surrénale, du syndrome des ovaires polykystiques, d'une dysfonction urinaire, de l'ostéoporose, de la sclérose en plaques, de la polyarthrite rhumatoïde, de la maladie d'Alzheimer, du cancer du côlon, de blessures cutanées, de rides et de cataractes.

**26.** Composition pharmaceutique comprenant comme agent actif au moins un des composés de la formule (I) tels que définis dans l'une quelconque des revendications précédentes 1 à 21 et au moins un vecteur pharmaceutiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004085457 A
- US 6541463 B
- WO 0142181 A
- WO 9832724 A
- WO 9830556 A
- WO 9912540 A
- WO 0226706 A
- JP 48042271 B
- US 5597823 A
- JP 62132884 B
- EP 2004006230 W
- EP 2004006231 W
- US 17799 W
- WO 0007996 A
- US 10289079 B
- US 20030170292 A


### Non-patent literature cited in the description


- **LABRIE et al.** *Trends Endocrinol Metab.,* 2000, vol. 11, 421-7
- **LABRIE F et al.** *Steroids,* 1997, vol. 62, 148-58
- **TAMAYA et al.** *Acta Obstet Gynecol Scand.,* 1985, vol. 64, 307-9
- **POIRIER D.** *Curr Med Chem.,* 2003, vol. 10, 453-77
- **GEISSLER WM et al.** *Nat Genet.,* 1994, vol. 7, 34-9
- **OEFELEIN MG ; CORNUM R.** *J Urol.,* 2000, vol. 164, 726-9
- **ANDERSSON S.** *J. Steroid Biochem. Molec. Biol.,* 1995, vol. 55, 533-534
- **DONG Y et al.** *J. Bone Min. Res.,* 1998, vol. 13, 1539-1546
- **MANHAS MS et al.** *J Med Chem.,* 1972, vol. 15 (1), 106-7
- **GADAD AK et al.** *Arzeimittelforschung,* 1996, vol. 46 (10), 981-5
- **MANJUNATH KS et al.** *Arzneimittelforschung,* 1997, vol. 47 (9), 1005-8
- **KAPUSTINA, M.V. ; KHARIZOMENOVA, I.A ; SHVEDOV, V.I.** Chem. Heterocycl. Compd. *Engl. Trans.,* 1991, 425
- **KOELLER, S. ; LELLOUCHE, J.-P.** *Tetrahedron Lett,* 1999, vol. 40, 7043
- **KAPUSTINA, M.V ; NIKOLAEVA, I.S. ; KHARIZOMENOVA, I.A ; SHVEDOV, V.I. ; PUSHKINA, T.V. ; FOMINA, A. N.** *Pharm. Chem. J.,* 1992, 789
- **LIDSTROEM, P. et al.** *Tetrahedron,* 2001, vol. 57, 9225
- **KAPUSTINA, M.V. ; KHARIZOMENOVA, I.A. ; SHVEDOV, V.I.** Chem. Heterocycl. Compd. *Engl. Trans.,* 1991, 425
- **GÜTSCHOW M. et al.** *J. Med. Chem.,* 1999, vol. 42, 5437
- **MITRA ; JOSHI.** *Synth. Comm.,* 1988, vol. 18, 2259
- **PERRISSIN et al.** *Eur. J. Med. Chem. Chim. Ther.,* 1980, vol. 15, 413-418
- **TANI et al.** *Chem. Pharm. Bull.,* 1996, vol. 44, 55-61
- **LIPSHUTZ et al.** *J. Org. Chem.,* 1984, vol. 49, 3938-3942
- **KOFFMANN B et al.** *J. Steroid. Biochem. Mol. Biol.,* 1991, vol. 38, 135
- **ANDERSSON S.** Molecular genetics of androgenic 17β-Hydroxyteroid Dehydrogenases. *J. Steroid Biochem. Molec. Biol.,* 1995, vol. 55, 533-534
- **DONG Y et al.** 17β-hydroxysteroid dehydrogenases in human bone cells. *J. Bone Min. Res.,* 1998, vol. 13, 1539-1546
- **GADAD AK ; KAPSI SG ; ANEGUNDI RI ; PATTAN SR ; MAHAJANSHETTI CS ; SHISHOO CJ.** Synthesis and antihyperlipaemic activity of some 2-aminomethyl-3-aryl-5,6,7,8-tetrahydrobenzo(b) / 5,6-dimethylthieno (2,3-d)-pyrimidin-4-ones. *Arzneimittelforschung,* 1996, vol. 46 (10), 981-5
- **GEISSLER WM et al.** Male pseudohermaphroditism caused by mutations of testicular 17beta-hydroxysteroid dehydrogenase 3. *Nat Genet,* 1994, vol. 7, 34-9
- **KAPUSTINA MV ; KHARIZOMENOVA A ; SHVEDOV VI ; FOMINA AN ; NIKOLAEVA IS ; GOLOVANOVA EM ; BOGDANOVA GA ; ALEKSEEVA LM.** Synthesis and antiviral activity of 7-oxo-and7-hydroxy-4,5,6,7-tetrahydrobenzo[b]-thiophene derivatives. *Chem. Heterocycl. Compd. (Engl. Transl.),* 1990, vol. 24, 1269-271

- **KAPUSTINA, M.V ; NIKOLAEVA, I.S. ; KHARIZOMENOVA, I.A. ; SHVEDOV, V.I. ; PUSHKINA, T.V. ; FOMINA, A. N.** *Pharm. Chem. J.,* 1992, 789
- **KAPUSTINA, M.V. ; KHARIZOMENOVA, I.A. ; SHVEDOV, V.I.** *Chem. Heterocycl. Compd. (Engl. Trans.),* 1991, 425
- **KHARIZOMENOVA A ; KAPUSTINA MV ; GRINEV AN ; SHEINKER YN ; ALEKSEEVA LM ; KULESHOVA EF.** Synthesis and structure of derivatives of 7-oxo-4,5,6,7-tetrahydrobenzo [b]-thiophene and 7-hydroxy [b]thiophene. *Chem. Heterocycl. Compd. (Engl. Transl.),* 1984, vol. 20, 1339-1342
- **KOELLER S ; LELLOUCHE JP.** Preparation of Formate Esters from O-TBDMS/O-TES Protected Alcohols. A One-Step Conversion Using the Vilsmeier-Haack Complex POCl3/DMF. *Tetrahedron Letters,* 1999, vol. 40 (38), 7043-7046
- **KOFFMAN B ; MODARRESS KJ ; BECKERMAN T ; BASHIRELAHI N.** Evidence for involvement of tyrosine in estradiol binding by rat uterus estrogen receptor. *J Steroid Biochem Mol Biol.,* 1991, vol. 38 (2), 135-9
- **LABRIE et al.** Role of 17 beta-hydroxysteroid dehydrogenases in sex steroid formation in peripheral intracrine tissues. *Trends Endocrinol Metab.,* 2000, vol. 11, 421-7
- **LABRIE F et al.** The key role of 17 beta-hydroxysteroid dehydrogenases in sex steroid biology. *Steroids,* 1997, vol. 62, 148-58
- **LIDSTRÖM P ; TIERNEY J ; WATHEY B ; WESTMAN J.** Microwave assisted organic synthesis - a review. *Tetrahedron,* 2001, vol. 57 (45), 9225-9283
- **LIPSHUTZ BH ; WILHELM RS ; KOZLOWSKI JA.** Conjugate addition reactions of $\alpha,\beta$-unsaturated ketones with higher order, mixed organocuprate reagents, R2Cu(CN)Li. *J. Org. Chem.,* 1984, vol. 49, 3938-3942
- **MANHAS MS ; SHARMA SD ; AMIN SG.** Heterocyclic compounds. 4. Synthesis and antiinflammatory activity of some substituted thienopyrimidinones. *J Med Chem.,* 1972, vol. 15 (1), 106-7
- **MANJUNATH KS ; MOHAN S ; NARAGUND LV ; SHISHOO CJ.** Synthesis and evaluation of 2-chloromethyl-3-N-substituted arylthieno(2,3-d)pyrimidin-4-ones and derivatives for central nervous system depressant activity. *Arzneimittelforschung,* 1997, vol. 47 (9), 1005-8
- **MITRA RB ; JOSHI VS.** A novel synthesis of ketoprofen, important non-steroidal antiinflammatory agent. *Synth. Comm.,* 1988, vol. 18, 2259
- **OEFELEIN MG ; COMUM R.** Failure to achieve castrate levels of testosterone during luteinizing hormone releasing hormone agonist therapy: the case for monitoring serum testosterone and a treatment decision algorithm. *J Urol.,* 2000, vol. 164, 726-9
- **PERRISSIN M ; DUC CL ; NARCISSE G ; BAKRI-LOGEAIS F ; HUGUET F.** Tétrahydro-4,5,6,7 benzo-[b] et tétrahydro-5,6,7,8 (4H)-cyclohepta[b]thiophene. *Eur. J. Med. Chem. Chim. Ther.,* 1980, vol. 15, 413-418
- **POIRIER D.** Inhibitors of 17 beta-hydroxysteroid dehydrogenases. *Curr Med Chem.,* 2003, vol. 10, 453-77
- **TAMAYA et al.** Comparison of cellular levels of steroid receptors in uterine leiomyoma and myometrium. *Acta Obstet Gynecol Scand.,* 1985, vol. 64, 307-9
- **TANI M ; ARIYASU T ; OHTSUKA M ; KOGA T ; OGAWA Y ; YOKOYAMA Y ; MURAKAMI Y.** New Strategy for indole synthesis from ethyl pyrrole-2-carboxylate (Synthetic Studies on Indoles and Related Compounds. XXXIX). *Chem. Pharm. Bull.,* 1996, vol. 44, 55-61